# EUROPEAN PATENT APPLICATION

(11) **EP 4 410 309 A1**
(43) Date of publication of application: **07.08.2024**
(21) Application number: 22876588.9
(22) Date of filing: 30.09.2022
(51) Int. Cl.: A61K 39/395, A23K 20/147, A23L 33/18, A61P 1/16, A61P 29/00

(54) **COMPOSITION CONTAINING MONOCLONAL ANTIBODY**

(30) Priority: 30.09.2021 JP 2021162273
(71) Applicant: Igalphan corporation, Nagoya-shi, Aichi 451-0045 (JP)
(72) Inventor: SHINKURA Reiko, Tokyo 113-8654 (JP); IWAISAKO Keiko, Kyoto-shi, Kyoto 602-8580 (JP)
(74) Representative: Grünecker Patent- und Rechtsanwälte PartG mbB
(86) International application number: PCT/JP2022/036877
(87) International publication number: WO 2023/054726

(57) **Abstract**

The present disclosure relates to a pharmaceutical composition, a food composition, or a feed composition containing a monoclonal antibody that binds to enteric bacteria and used for preventing or treating an impairment or disease attributed to liver inflammation, and a therapeutic method using these compositions.

## Description

### Technical Field

In the present specification, the contents of Japanese Patent Application No. 2021-110421 are incorporated herein by reference.

The present disclosure relates to a pharmaceutical composition, a food composition, or a feed composition containing an IgA antibody that binds to enteric bacteria and used for preventing or treating an impairment or disease attributed to liver inflammation, and a therapeutic method using these compositions.

### Background Art

Liver fibrosis is a state in which excessive connective tissue is accumulated in the liver due to healing of a liver tissue injury site accompanied by excessive extracellular matrix proliferation. In the liver fibrosis, excess connective tissue is accumulated in the liver, which reflects scarring that occurs in response to chronically recurring hepatocellular injury. In general, hepatocytes that have fallen due to repeated liver cell impairments are replaced by connective tissue, fibrosis progresses, the structure of liver tissue collapses, and liver function is ultimately impaired. When such collapse is widespread, liver cirrhosis is diagnosed. Various types of chronic liver impairments can cause fibrosis (Non Patent Literature 1).

Since fibrosis reflects a response to a liver impairment, a primary treatment of liver fibrosis focuses on eliminating the cause of the liver impairment. Such a treatment includes a nucleic acid analog treatment for hepatitis B or elimination of hepatitis C virus in chronic viral hepatitis, abstinence in alcoholic liver disease, elimination of heavy metals such as iron in hemochromatosis and copper in Wilson's disease, decompression of bile ducts in biliary obstruction, and the like. Such a treatment stops the progression of fibrosis and may result in partial healing of fibrotic changes in some patients (Non Patent Literature 1).

Therapies intended to cure fibrosis are usually too toxic or have no proven effect for long-term administration. Other anti-fibrotic treatments are under study. Silymarin is widely used as an alternative medicine to treat liver fibrosis. Silymarin is considered to be safe, but is ineffective except when used in combination with a specific drug for treating hepatitis C (Non Patent Literature 1).

Therefore, there remains a need to provide an additional therapeutic agent for inhibiting liver fibrosis and preventing and treating an impairment or disease associated with liver fibrosis such as liver cirrhosis.

It is known that an enteric bacteria-binding monoclonal antibody modifies intestinal flora by oral ingestion and improves the enteric environment (Non Patent Literature 2). However, it is not known that an enteric bacteria-binding monoclonal antibody is associated with liver fibrosis.

### Citation List

### Non Patent Literatures

Non Patent Literature 1: MSD Manual Professional Edition, access date: September 10, 2021: URL:https://www.msdmanuals.com/ja-jp/professional/02-hepatic-and-biliary-disorders/fibrosis-and-cirrhosis/hepatic-fibrosis
Non Patent Literature 2: Okai S et al. Nat Microbiol., 2016, 1(9); 16103.
Non Patent Literature 3: O'brien A, et al., Liver Int. 2016; 36: 837-846.

### Summary of Invention

### Technical Problem

As described above, there is a need to provide an effective therapeutic agent for inhibiting liver fibrosis and preventing and treating an impairment or disease associated with liver fibrosis such as liver cirrhosis. An object of the present disclosure is to provide a drug and method for inhibiting liver fibrosis by inhibiting inflammation of liver tissue, thereby providing a drug and method for preventing and treating an impairment or disease associated with liver fibrosis such as liver cirrhosis.

### Solution to Problem

As a result of intensive studies, the present inventors have unexpectedly found that inflammation of liver tissue causing liver fibrosis can be inhibited by orally administering an IgA antibody that specifically recognizes enteric bacteria. Based on this unexpected finding, the present disclosure provides a drug and method for inhibiting liver fibrosis by inhibiting inflammation of liver tissue, and/or a drug and method for preventing and treating an impairment or disease associated with liver fibrosis such as liver cirrhosis.

In one aspect, the present disclosure provides a pharmaceutical composition, a food composition, or a feed composition containing a monoclonal antibody that binds to enteric bacteria and used for preventing or treating an impairment or disease attributed to liver inflammation.

In one aspect, the present disclosure provides a method for preventing or treating an impairment or disease attributed to liver inflammation using a pharmaceutical composition, a food composition, or a feed composition containing a monoclonal antibody that binds to enteric bacteria.

In one aspect, the present disclosure provides a method for preventing or treating an impairment or disease attributed to liver inflammation, the method including administering a monoclonal antibody that binds to enteric bacteria to a subject in need of prevention or treatment of an impairment or disease attributed to liver inflammation.

More specifically, the present disclosure provides the following.

### [Item 1]

A pharmaceutical composition, a food composition, or a feed composition containing a monoclonal antibody that binds to enteric bacteria or an antigen-binding fragment thereof, in which the pharmaceutical composition, the food composition, or the feed composition is used for preventing or treating an impairment or disease attributed to liver inflammation.

### [Item 2]

The composition according to Item 1, in which the monoclonal antibody that binds to enteric bacteria or the antigen-binding fragment thereof is a monoclonal antibody that binds to a *Clostridium difficile* bacterial body or an antigen-binding fragment thereof.

### [Item 3]

The composition according to Item 1, in which the monoclonal antibody that binds to enteric bacteria or the antigen-binding fragment thereof is one or more antibodies or antigen-binding fragments thereof, the antibodies being selected from the group consisting of:
a) an antibody including:
   a heavy chain variable region containing an amino acid sequence of a heavy chain CDR1, an amino acid sequence of a heavy chain CDR2, and an amino acid sequence of a heavy chain CDR3, the heavy chain variable region containing an amino acid sequence set forth in SEQ ID NO: 7; and
   a light chain variable region containing an amino acid sequence of a light chain CDR1, an amino acid sequence of a light chain CDR2, and an amino acid sequence of a light chain CDR3, the light chain variable region containing an amino acid sequence set forth in SEQ ID NO: 8;
b) an antibody including:
   a heavy chain variable region containing an amino acid sequence of a heavy chain CDR1, an amino acid sequence of a heavy chain CDR2, and an amino acid sequence of a heavy chain CDR3, the heavy chain variable region containing an amino acid sequence set forth in SEQ ID NO: 17; and
   a light chain variable region containing an amino acid sequence of a light chain CDR1, an amino acid sequence of a light chain CDR2, and an amino acid sequence of a light chain CDR3, the light chain variable region containing an amino acid sequence set forth in SEQ ID NO: 18;
c) an antibody including:
   a heavy chain variable region containing an amino acid sequence of a heavy chain CDR1, an amino acid sequence of a heavy chain CDR2, and an amino acid sequence of a heavy chain CDR3, the heavy chain variable region containing an amino acid sequence set forth in SEQ ID NO: 27; and
   a light chain variable region containing an amino acid sequence of a light chain CDR1, an amino acid sequence of a light chain CDR2, and an amino acid sequence of a light chain CDR3, the light chain variable region containing an amino acid sequence set forth in SEQ ID NO: 28;
d) an antibody including:
   a heavy chain variable region containing an amino acid sequence of a heavy chain CDR1, an amino acid sequence of a heavy chain CDR2, and an amino acid sequence of a heavy chain CDR3, the heavy chain variable region containing an amino acid sequence set forth in SEQ ID NO: 37; and
   a light chain variable region containing an amino acid sequence of a light chain CDR1, an amino acid sequence of a light chain CDR2, and an amino acid sequence of a light chain CDR3, the light chain variable region containing an amino acid sequence set forth in SEQ ID NO: 38; and
e) an antibody containing,
   with respect to a reference antibody including a heavy chain variable region containing an amino acid sequence set forth in SEQ ID NO: 37 and a light chain variable region containing an amino acid sequence set forth in SEQ ID NO: 38,
   at least one amino acid mutation in at least one region selected from heavy chains CDR1 to 3, light chains CDR1 to 3, and a light chain FR1, and
   binding to the amino acid sequence RQEEHIELIAS in *E*. *coli* SHMT protein and the amino acid sequence VLDMMKLEKPE in C. *difficile* iPGM protein.

### [Item 4]

The composition according to Item 1, in which the monoclonal antibody that binds to enteric bacteria is one or more antibodies or antigen-binding fragments thereof, the antibodies being selected from the group consisting of:
a) an antibody including:
   a heavy chain variable region containing:
   a heavy chain CDR1 containing an amino acid sequence set forth in SEQ ID NO: 1;
   a heavy chain CDR2 containing an amino acid sequence set forth in SEQ ID NO: 2; and
   a heavy chain CDR3 containing an amino acid sequence set forth in SEQ ID NO: 3; and
   a light chain variable region containing:
      a light chain CDR1 containing an amino acid sequence set forth in SEQ ID NO: 4;
      a light chain CDR2 containing an amino acid sequence set forth in SEQ ID NO: 5; and
      a light chain CDR3 containing an amino acid sequence set forth in SEQ ID NO: 6;
b) an antibody including:
   a heavy chain variable region containing:
   a heavy chain CDR1 containing an amino acid sequence set forth in SEQ ID NO: 11;
   a heavy chain CDR2 containing an amino acid sequence set forth in SEQ ID NO: 12; and
   a heavy chain CDR3 containing an amino acid sequence set forth in SEQ ID NO: 13; and
   a light chain variable region containing:
      a light chain CDR1 containing an amino acid sequence set forth in SEQ ID NO: 14;
      a light chain CDR2 containing an amino acid sequence set forth in SEQ ID NO: 15; and
      a light chain CDR3 containing an amino acid sequence set forth in SEQ ID NO: 16;
c) an antibody including:
   a heavy chain variable region containing:
   a heavy chain CDR1 containing an amino acid sequence set forth in SEQ ID NO: 21;
   a heavy chain CDR2 containing an amino acid sequence set forth in SEQ ID NO: 22; and
   a heavy chain CDR3 containing an amino acid sequence set forth in SEQ ID NO: 23; and
   a light chain variable region containing:
      a light chain CDR1 containing an amino acid sequence set forth in SEQ ID NO: 24;
      a light chain CDR2 containing an amino acid sequence set forth in SEQ ID NO: 25; and
      a light chain CDR3 containing an amino acid sequence set forth in SEQ ID NO: 26;
d) an antibody including:
   a heavy chain variable region containing:
   a heavy chain CDR1 containing an amino acid sequence set forth in SEQ ID NO: 31;
   a heavy chain CDR2 containing an amino acid sequence set forth in SEQ ID NO: 32; and
   a heavy chain CDR3 containing an amino acid sequence set forth in SEQ ID NO: 33; and
   a light chain variable region containing:
      a light chain CDR1 containing an amino acid sequence set forth in SEQ ID NO: 34;
      a light chain CDR2 containing an amino acid sequence set forth in SEQ ID NO: 35; and
      a light chain CDR3 containing an amino acid sequence set forth in SEQ ID NO: 36; and
e) an antibody containing,
   with respect to a reference antibody including a heavy chain variable region containing an amino acid sequence set forth in SEQ ID NO: 37 and a light chain variable region containing an amino acid sequence set forth in SEQ ID NO: 38,
   at least one amino acid mutation in at least one region selected from heavy chains CDR1 to 3, light chains CDR1 to 3, and a light chain FR1, and
   binding to the amino acid sequence RQEEHIELIAS in *E*. *coli* SHMT protein and the amino acid sequence VLDMMKLEKPE in C. *difficile* iPGM protein.

### [Item 5]

The composition according to Item 1, in which the monoclonal antibody that binds to enteric bacteria is an antibody including:
a heavy chain variable region containing:
a heavy chain CDR1 containing an amino acid sequence of X₁YYIH;
a heavy chain CDR2 containing an amino acid sequence of RIDPENX₂X₃TTYAPKFQ; and
a heavy chain CDR3 containing an amino acid sequence of YCARSTVL; and
a light chain variable region containing:
   a light chain CDR1 containing an amino acid sequence of RX₄SQSIVHTNG;
   a light chain CDR2 containing an amino acid sequence of KLLIYKV; and
   a light chain CDR3 containing an amino acid sequence of GVYYFQGS,
   in which a light chain FR1 contains an amino acid sequence of TPLSLPVSLGDQA or an amino acid sequence of SPASX₅SVSLGDRX₆,
   X₁, X₂, and X₃ are each independently a neutral polar amino acid or an acidic polar amino acid,
   X₄ is a non-polar amino acid or a neutral polar amino acid, and
   X₅ and X₆ are each independently a non-polar amino acid.

### [Item 6]

The composition according to any one of claims 1 to 5, in which the monoclonal antibody that binds to enteric bacteria is an IgA antibody in a multimeric form or a monomeric form, an IgM antibody in a multimeric form or a monomeric form, an IgG antibody in a monomeric form or a combination thereof.

### [Item 7]

The composition according to any one of Items 1 to 6, in which the disease or impairment attributed to liver inflammation is chronic hepatitis, liver fibrosis, liver cirrhosis, liver inflammation following liver transplantation, autoimmune hepatitis, or intrahepatic lithiasis.

### [Item 8]

A method for preventing or treating an impairment or disease attributed to liver inflammation, the method including administering a monoclonal antibody that binds to enteric bacteria to a subject in need of prevention or treatment of an impairment or disease attributed to liver inflammation.

### Advantageous Effects of Invention

The present disclosure has an effect of providing a drug and method for inhibiting liver fibrosis by inhibiting inflammation of liver tissue, thereby providing a drug and method for preventing and treating liver cirrhosis.

### Brief Description of Drawings

Fig. 1 is a view illustrating results of measuring ALT and T-Bil for plasma of venous blood obtained from a mouse sacrificed 14 days after common bile duct ligation.
Fig. 2 is a view illustrating results of collagen stained with Sirius Red of a liver tissue section obtained from a mouse sacrificed 14 days after common bile duct ligation.
Fig. 3 is a view illustrating a result of calculating an area ratio of stained portions by ImageJ for the Sirius Red stained image illustrated in Fig. 2.
Fig. 4 is a view illustrating a heat map created by extracting differentially expressed genes in three groups of a control group, a BDL group, and an IgA group.
Fig. 5 is a view illustrating results of extracting differentially expressed genes between each two groups of control-BDL, BDL-IgA, and control-IgA.
Fig. 6A is a view illustrating results of pathway analysis, in which the results of control-BDL are illustrated.
Fig. 6B is a view illustrating results of pathway analysis, in which the results of control-IgA are illustrated.
Fig. 6C is a view illustrating results of pathway analysis, in which the results of BDL-IgA are illustrated.
Fig. 7 is a view illustrating a result of intestinal flora analysis.
Fig. 8A is a view illustrating a result of ANCOM in a subject treated with common bile duct ligation (BDL), in which the results of a W27IgA administration group (IgA14-21, IgA14-22, and IgA14-31) and a control group (non14-17, non14-18, and non14-30) are illustrated.
Fig. 8B is a view illustrating a result of ANCOM in a subject treated with no common bile duct ligation (BDL).
Fig. 9 is a view illustrating two-dimensional electrophoresis data when an antigen molecule of C. *difficile* is identified, in which the same spots in three SDS-PAGE images are indicated by red arrows, and a sample obtained from the spot is subjected to mass spectrometry.
Fig. 10 is a view illustrating results of Western blot and CBB stain of W27G2 antibodies for various synthetic peptides, in which the gray letters indicate an amino acid sequence common to each bacterial species, and the underline indicates the synthetic peptide recognized by the W27G2 antibodies.
Fig. 11 is a view illustrating results of Western blot and CBB stain of W27G2 antibodies for various synthetic peptides, in which the gray letters indicate an amino acid sequence common to each bacterial species, and the underline indicates the synthetic peptide recognized by the W27G2 antibodies.
Fig. 12 is a view illustrating a part of the results of database analysis of bacteria sharing an epitope of *E*. *coli* SHMT recognized by W27G2 antibodies.
Fig. 13 is a view illustrating crystals of RS_H000_L000GR Fab - *E. coli* SHMT (25-45).
Fig. 14 is a view illustrating crystals of an RS_H000_L000GR Fab-C. *difficile* iPGM (486-509) complex.
Fig. 15 is a three-dimensional reconfiguration image showing a binding mode between W27G2 antibodies and *E. coli* SHMT antigen (left) and a binding mode between W27G2 antibodies and C. *difficile* iPGM antigen.
Fig. 16 is a three-dimensional reconfiguration image showing a binding mode between W27G2 antibodies and *E. coli* SHMT antigen (left) and a binding mode between W27G2 antibodies and C. *difficile* iPGM antigen.
Fig. 17 illustrates that amino acid sequences of heavy chain variable regions of mutants of the W27G2 antibody which is an antibody binding to a C. *difficile* bacterial body of the present disclosure are aligned.
Fig. 18 illustrates that amino acid sequences of light chain variable regions of mutants of the W27G2 antibody which is an antibody binding to a C. *difficile* bacterial body of the present disclosure are aligned.

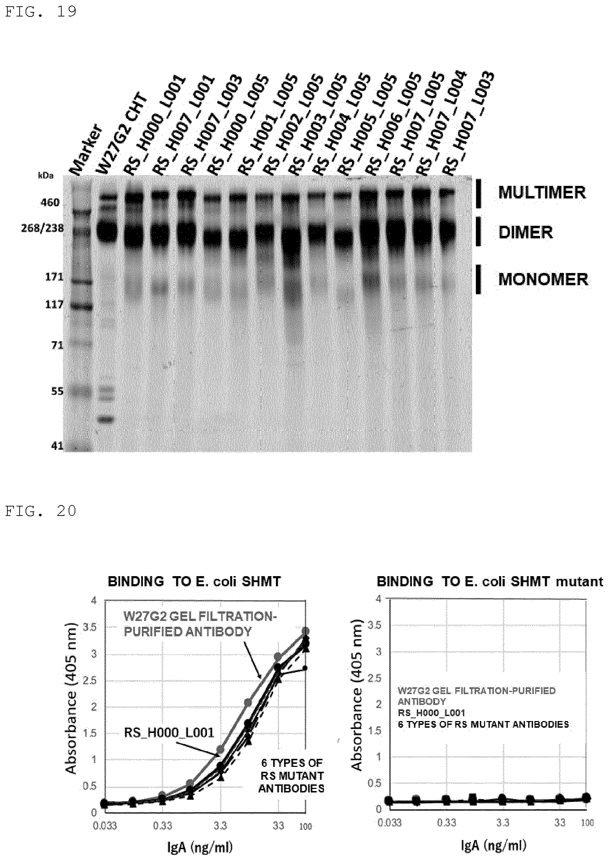

Fig. 19 is a view illustrating results of subjecting RS mutant recombinant purified antibodies to non-reduced SDS-PAGE, and performing Coomassie blue stain, in which Lane 1 shows a result of an antibody sample that is crudely purified from a W27G2 hybridoma culture solution by a hydroxyapatite column, and Lanes 2 to 14 show results of samples of various RS mutant recombinant purified antibodies produced by CHO cells (RS mutant recombinant purified antibodies are antibodies produced in CHO cells by introducing mutations into heavy chains or light chains of the W27G2 antibody so that antigen specificity is not substantially changed, and details are omitted in Fig. 19).
Fig. 20 is a view illustrating binding of a W27G2 antibody and various RS mutant recombinant purified antibodies obtained from a hybridoma to *E. coli* SHMT and SHMT mutants.
Fig. 21 is a view illustrating specific binding characteristics to total proteins of a plurality of bacteria for the W27G2 antibody and various RS mutant recombinant purified antibodies, in which W27G2-CHT (obtained by crudely purifying a W27 hybridoma culture supernatant with a hydroxyapatite column) and W27G2-GF (obtained by further purifying W27CHT into a multimer fraction with a gel filtration column) are used as the W27G2 antibodies, and RS_H000_L001, RS_H000_L005, and RS_H007_L005 are used as the RS mutant recombinant purified antibodies.
Fig. 22 is a view illustrating an *E. coli* growth inhibitory effect by various RS variant recombinant purified antibodies.

### Description of Embodiments

Decompensation of liver cirrhosis represents the final stage of liver cirrhosis, regardless of its underlying cause, and is a stage at which the morbidity and mortality of the disease are concentrated. It is considered that a patient exhibiting decompensated liver cirrhosis shows extremely high sensitivity to infection, and the sensitivity to the infection increases the risk of liver cirrhosis. As an excellent disease model of such decompensation of liver cirrhosis, it is known to use a common bile duct ligation (BDL) mouse (Non Patent Literature 3: O'brien A, et al., Liver Int. 2016; 36: 837-846). The common bile duct ligation (BDL) mouse provides an excellent disease model as a cholestasis liver impairment model.

The present inventors have found that, in a BDL mouse model, orally administering an enteric bacteria-binding monoclonal antibody for a certain period of time from immediately before BDL to after BDL surprisingly inhibits BDL-dependent liver tissue inflammation and reduces liver tissue impairment. Based on these test results, it has been found that the enteric bacteria-binding monoclonal antibody is useful for prevention and/or treatment of liver organization and an impairment or disease attributed to the liver organization such as liver cirrhosis.

### (Definition)

Unless defined otherwise, all technical terms and scientific terms used in the present specification have the same meanings as commonly understood by those skilled in the art to which the present disclosure pertains.

In the present specification, in a case where a plurality of ranges of numerical values are indicated, a range including any combination of a lower limit value and an upper limit value of the plurality of ranges is also meant in the same manner.

In the present disclosure, the term "substantially" has the same meaning as commonly understood by those skilled in the art to which the present disclosure pertains, but is used with the intention of encompassing, for example, a desired state and a state that is unavoidably unachieved due to biological or chemical properties, considering that biological or chemical phenomena may not completely achieve the desired state.

In the present disclosure, a case where the term "about" is used in connection with a numerical value x means that the value may vary, for example, within 10%, 9%, 8%, 7%, 6%, 5%, 4%, 3%, 2%, 1%, 0.5%, 0.1%, 0.05%, or 0.01%.

In the present disclosure, the term "containing" has the same meaning as commonly understood by those skilled in the art to which the present disclosure pertains, but includes, for example, "containing" and "consisting of", and specifically means that a composition "containing" A may also contain B as another component, in addition to containing only A.

In the present disclosure, the term "consisting of" or "composed of" used for a composition has the same meaning as commonly understood by those skilled in the art to which the present disclosure pertains, but is used to indicate components that solely constitute the composition. For example, a composition "consisting of" A contains only A. However, in one aspect, a composition "consisting of" A encompasses an aspect that includes contaminants other than A that are unavoidable in preparation based on biological and chemical properties.

In the present specification, the term "antibody" is used in the broadest sense, and includes, but is not limited to, a monoclonal antibody, a polyclonal antibody, and an antibody fragment that exhibit an intended antigen binding activity. A full-length antibody includes a heavy chain and a light chain mainly composed of a polypeptide. The heavy chain and the light chain each contain a site called a variable region that recognizes an antigen, and the sites are generally called a heavy chain variable region and a light chain variable region, respectively. The variable region has, in order from the amino terminus, sites referred to as CDR1 to 3, each of which is identified as a site that recognizes an antigen in more detail. Note that these CDR1 to 3 are also referred to in more detail as a heavy chain CDR1, a heavy chain CDR2, a heavy chain CDR3, a light chain CDR1, a light chain CDR2, a light chain CDR3, and the like. In addition, regions other than CDR1 to 3 of the heavy chains and the light chains are referred to as heavy chains FR1 to 4 and light chains FR1 to 4, respectively, in order from the amino terminus. The antibody may be in the form of an antibody composed of two heavy chains and two light chains, or in the form of an antibody composed of one heavy chain and one light chain (also referred to as a single-chain antibody).

The antibody may be any class such as IgG, IgE, IgM, IgD, IgA, or IgY, or may be a subclass such as IgG1, IgG2, IgG3, IgG4, IgA1, or IgA2.

The antibody may contain an amino acid sequence derived from the same species or may contain an amino acid derived from a different species. Examples of the amino acid sequence derived from the same species include amino acid sequences derived from a human, a mouse, a rat, a hamster, a rabbit, a goat, a donkey, a pig, a cow, a horse, a chicken, a monkey, a chimpanzee, a camel, and a llama.

The case of the amino acid sequence derived from a different species is not particularly limited, and examples thereof include amino acid sequences derived from two or more of a human, a mouse, a rat, a hamster, a rabbit, a goat, a sheep, a donkey, a pig, a cow, a horse, a chicken, a monkey, a chimpanzee, a camel, and a llama.

In the present specification, the "antigen-binding fragment" of the antibody refers to one or more fragments of the antibody that retain an ability to specifically bind to an antigen. It has been found that the ability of an antibody to specifically bind to an antigen can also be maintained by fragments consisting of a part thereof. As one aspect, an "antigen-binding fragment" of an antibody may be, but is not limited to, a Fab fragment consisting of a CH1 domain that is a part of a light chain variable region (VL), a heavy chain variable region (VH), a light chain constant region (CL), and a heavy chain constant region, a F(ab')2 fragment including two Fab fragments linked by a disulfide bridge at a hinge region, a Fd fragment consisting of the VH and CH1 domain, a Fv fragment consisting of the VL and VH domains of a single arm of an antibody, a dAb fragment including a single variable domain, and an isolated complementarity determining region (CDR).

The antibody of the present disclosure may be a CDR-grafted antibody. In one example, in a CDR-grafted antibody, some or all sequences of CDR regions of an antibody derived from one animal species are substituted with CDR sequences of another animal species. For example, CDRs of one or more mouse antibodies are substituted with CDR sequences of human antibodies.

Methods well known to those skilled in the art can be used in identifying heavy chains CDR1 to 3 in a heavy chain variable region and light chains CDR1 to 3 in a light chain variable region of an antibody. For example, "Kabat definition" (Kabat et al., Ann. NY Acad, Sci. 1971, Vol. 190, pp. 382-391 and Kabat, E.A. et al. Sequences of Proteins of Immunological Interest, 5th Ed. 1991, U.S. Department of Health and Human Services, NIH Publication, pp. 91-3242),"Chothia definition" (Chothia et al., Nature, 1989, Vol. 342, pp. 877-883), "Contact definition" (MacCallum et al., J. Mol. Biol., 1996, Vol. 262, pp. 732-745), and the like, which are well known to those skilled in the art, can be used. CDRs may be identified based on public database information to identify CDR sequences in the antibody.

In the present specification, the "identity" refers to a degree of the same amino acid sequence or base sequence of two or more comparable amino acid sequences or base sequences with respect to each other. Therefore, the higher the identity between two amino acid sequences or base sequences, the higher the identity or similarity between the sequences. A level of the identity between the amino acid sequences or base sequences is usually determined using FASTA, which is a tool for sequence analysis, and default parameters. Alternatively, it can be determined using the algorithm BLAST (for example, Karlin S, Altschul SF. Proc. Natl Acad Sci USA. 87:2264-2268 (1990), Karlin S, Altschul SF. Natl Acad Sci USA. 90:5873-7 (1993), and the like) by Karlin and Altschul. A program called BLASTN or BLASTX based on such a BLAST algorithm has been developed (for example, Altschul SF, GishW, Miller W, Myers EW, Lipman DJ. J Mol Biol. 215:403-10 (1990), and the like). Specific techniques for these analysis methods are known and can be referred to the NCBI website. For example, a case where a certain amino acid sequence A is a specific % identical to another amino acid sequence B means that the amino acid sequence A and the amino acid sequence B have the % identity.

In the present specification, the term "monoclonal" is a modifier indicating a characteristic of an antibody or the like obtained from a substantially homogeneous population of antibodies. The individual antibodies contained in such a population of antibodies are identical except for naturally occurring mutations that may be present in a small amount.

In the present specification, the term "conservative substitution technique" means a technique in which an amino acid residue is substituted with an amino acid residue having a similar side chain.

For example, substitution between amino acid residues having a basic side chain such as lysine, arginine, and histidine corresponds to a conservative substitution technique. Other examples of the conservative substitution technique include substitutions between amino acid residues having an acidic side chain such as aspartic acid and glutamic acid; substitutions between amino acid residues having an uncharged polar side chain such as glycine, asparagine, glutamine, serine, threonine, tyrosine, and cysteine; substitutions between amino acid residues having a non-polar side chain such as alanine, valine, leucine, isoleucine, proline, phenylalanine, methionine, and tryptophan; substitutions between amino acid residues having a β-branched side chain such as threonine, valine, and isoleucine; and substitutions between amino acid residues having an aromatic side chains such as tyrosine, phenylalanine, tryptophan, and histidine.

### 1. Antibody-Containing Composition

An antibody-containing composition of the present disclosure contains an IgA antibody that binds to enteric bacteria, and has an effect of inhibiting liver tissue inflammation and preventing or treating an impairment or disease associated with liver inflammation.

The antibody-containing composition of the present disclosure can be suitably used in the field of food and the field of feed with the expectation of exhibiting the above effect. Therefore, the composition of the present disclosure can be a food composition, a food material, or a feed composition.

### 2. Monoclonal Antibody That Binds to Enteric Bacteria

An antibody used in the antibody-containing composition of the present disclosure is a monoclonal antibody that binds to enteric bacteria. In one aspect, it is desirable to use monoclonal IgA, IgG, and IgM antibodies. The antibody can be used in either a multimeric form or a monomeric form. In one aspect, it is desirable to use a monomeric from or a multimeric form of the monoclonal IgA antibody, a monomeric from or a multimeric form of the IgM antibody, or a monomeric form of IgG. In one aspect, a plurality of antibodies may be used in combination.

As the monoclonal antibody that binds to enteric bacteria of the present disclosure, an antibody produced from an antibody-producing cell derived from B cells such as a hybridoma may be used, or an antibody produced by introducing a nucleic acid encoding the antibody into a cell other than the immune system using a genetic recombination technique may be used as a recombinant antibody.

In the present disclosure, the term "enteric bacteria" is not particularly limited as long as the enteric bacteria are bacteria that are resident in the intestine in a living body. Examples of such enteric bacteria include bacteria that form intestinal flora in a living body and are involved in maintenance of homeostasis of the intestinal immune system.

Examples of specific enteric bacteria include, but are not particularly limited to,
bacteria belonging to the genus *Prevotella,*
bacteria belonging to the genus *Bacteroides,*
bacteria belonging to the genus *Megamonas,*
bacteria belonging to the genus *Bifidobacterium,*
bacteria belonging to the genus *Faecalibacterium,*
bacteria belonging to the genus *Coprococcus,*
bacteria belonging to the genus *Ruminococcus,*
bacteria belonging to the genus *Blautia,*
bacteria belonging to the genus *Eubacterium,*
bacteria belonging to the genus *Roseburia,*
bacteria belonging to the genus *Lactobacillus,*
bacteria belonging to the genus *Clostridium,*
bacteria belonging to the genus *Escherichia,*
bacteria belonging to the genus *Staphylococcus,*
bacteria belonging to the genus *Enterococcus,*
bacteria belonging to the genus *Pseudomonas,*
bacteria belonging to the genus *Enterorhabdus,* and
bacteria belonging to the genus *Fusobacterium.*

In one aspect, the monoclonal antibody that binds to enteric bacteria of the present disclosure binds to C. *difficile* bacteria. Typically, the antibody of the present disclosure binds to undestroyed C. *difficile* present in a culture supernatant.

In one aspect, the monoclonal antibody that binds to enteric bacteria of the present disclosure binds to a plurality of enteric bacteria, including C. *difficile* bacteria. For example, the monoclonal antibody that binds to enteric bacteria of the present disclosure binds to two or more of the following bacteria:
bacteria belonging to the genus *Prevotella,*
bacteria belonging to the genus *Bacteroides,*
bacteria belonging to the genus *Megamonas,*
bacteria belonging to the genus *Bifidobacterium,*
bacteria belonging to the genus *Faecalibacterium,*
bacteria belonging to the genus *Coprococcus,*
bacteria belonging to the genus *Ruminococcus,*
bacteria belonging to the genus *Blautia,*
bacteria belonging to the genus *Eubacterium,*
bacteria belonging to the genus *Roseburia,*
bacteria belonging to the genus *Lactobacillus,*
bacteria belonging to the genus *Clostridium,*
bacteria belonging to the genus *Escherichia,*
bacteria belonging to the genus *Staphylococcus,*
bacteria belonging to the genus *Enterococcus,*
bacteria belonging to the genus *Pseudomonas,*
bacteria belonging to the genus *Enterorhabdus,* and
bacteria belonging to the genus *Fusobacterium.*

In one aspect, the monoclonal antibody that binds to enteric bacteria of the present disclosure is a W27G2 antibody or a variant thereof, and as shown in Examples, these antibodies have the property of binding to a plurality of enteric bacteria.

In one aspect, the monoclonal antibody that binds to enteric bacteria of the present disclosure includes:
a heavy chain variable region containing an amino acid sequence of a heavy chain CDR1, an amino acid sequence of a heavy chain CDR2, and an amino acid sequence of a heavy chain CDR3, the heavy chain variable region containing an amino acid sequence set forth in SEQ ID NO: 7; and
a light chain variable region containing an amino acid sequence of a light chain CDR1, an amino acid sequence of a light chain CDR2, and an amino acid sequence of a light chain CDR3, the light chain variable region containing an amino acid sequence set forth in SEQ ID NO: 8 .

| Sequence name (SEQ ID NO:) | Amino acid sequence |
|---|---|
| SNK0001_HV (SEQ ID NO: 7) | MEWIWIFLFILSGTAGVQSQVQLQQSGAELARPG ASVKLSCKASGYTFTSYGISWVKQRTGQGLEWIG EIYPRSGNTYYNEKFKGKATLTADKSSSTAYMEL RSLTSEDSAVYFCARLASSYYGSSYDWYFDVWGT GTTVTVSS |
| SNK0001_LV (SEQ ID NO: 8) | MRTPAQFLGILLLWFPGIKCDIKMTQSPSSMYAS LGERVTITCKASQDINSYLSWFQQKPGKSPKTLI YRANRLVDGVPSRFSGSGSGQDYSLTISSLEYED MGIYYCLQYDEFPLTFGAGTKLELK |

In one aspect, the monoclonal antibody that binds to enteric bacteria of the present disclosure includes:
a heavy chain variable region containing:
a heavy chain CDR1 containing an amino acid sequence set forth in SEQ ID NO: 1;
a heavy chain CDR2 containing an amino acid sequence set forth in SEQ ID NO: 2; and
a heavy chain CDR3 containing an amino acid sequence set forth in SEQ ID NO: 3; and
a light chain variable region containing:
a light chain CDR1 containing an amino acid sequence set forth in SEQ ID NO: 4;
a light chain CDR2 containing an amino acid sequence set forth in SEQ ID NO: 5; and
a light chain CDR3 containing an amino acid sequence set forth in SEQ ID NO: 6.

| Sequence name (SEQ ID NO:) | Amino acid sequence |
|---|---|
| SNK0001_HCDR1 (SEQ ID NO: 1) | SYGIS |
| SNK0001_HCDR2 (SEQ ID NO: 2) | EIYPRSGNTYYNEKFK |
| SNK0001_HCDR3 (SEQ ID NO: 3) | FCARLASS |
| SNK0001_LCDR1 (SEQ ID NO: 4) | KASQDINSYLS |
| SNK0001_LCDR2 (SEQ ID NO: 5) | RANRLVD |
| SNK0001_LCDR3 (SEQ ID NO: 6) | LQYDEFPLT |

In one aspect, the monoclonal antibody that binds to enteric bacteria of the present disclosure includes:
a heavy chain variable region containing an amino acid sequence set forth in SEQ ID NO: 7 or a sequence having at least 90%, at least 95%, at least 98%, or at least 99% sequence identity therewith; and
a light chain variable region containing an amino acid sequence set forth in SEQ ID NO: 8 or a sequence having at least 90%, at least 95%, at least 98%, or at least 99% sequence identity therewith.

In one aspect, the monoclonal antibody that binds to enteric bacteria of the present disclosure is an IgA recombinant purified antibody SNK0001AR. The IgA recombinant purified antibody is produced by applying a recombination technique based on a nucleic acid sequence encoding an IgM antibody SNK0001M produced by a hybridoma obtained from spleen-derived B cells. Similar to SNK0001AR, the IgM antibody SNK0001M or other classes of antibodies with similar variable regions can be used as the monoclonal antibodies of the present disclosure.

The antibody SNK0001AR has the following amino acid sequence configuration.

| Amino acid sequence of SNK0001AR | | |
|---|---|---|
| Heavy chain | Full-length sequence | MEWIWIFLFILSGTAGVQSQVQLQQSGAELARPGASVKLSCKASGYTFT SYGISWVKQRTGQGLEWIGEIYPRSGNTYYNEKFKGKATLTADKSSSTA YMELRSLTSEDSAVYFCARLASSYYGSSYDWYFDVWGTGTTVTVSSESA RNPTIYPLTLPRALSSDPVIIGCLIHDYFPSGTMNVTWGKSGKDITTVN FPPALASGGGYTMSSQLTLPAVECPEGESVKCSVQHDSNAVQELDVKCS GPPPPCPPCPPSCHPSLSLQRPALEDLLLGSDASLTCTLNGLRNPEGAV FTWEPSTGKDAVQKKAVQNSCGCYSVSSVLPGCAERWNSGASFKCTVTH PESDTLTGTIAKITVNTFPPQVHLLPPPSEELALNELVSLTCLVRAFNP KEVLVRWLHGNEELSPESYLVFEPLKEPGEGATTYLVTSVLRVSAELWK QGDQYSCMVGHEALPMNFTQKTIDRLSGKPTNVSVSVIMSEGDGICY |
| | Variable region | MEWIWIFLFILSGTAGVQSQVQLQQSGAELARPGASVKLSCKASGYTFT SYGISWVKQRTGQGLEWIGEIYPRSGNTYYNEKFKGKATLTADKSSSTA YMELRSLTSEDSAVYFCARLASSYYGSSYDWYFDVWGTGTTVTVSS |
| | CDR1 | SYGIS |
| | CDR2 | EIYPRSGNTYYNEKFK |
| | CDR3 | FCARLASS |
| Light chain | Full-length sequence | MRTPAQFLGILLLWFPGIKCDIKMTQSPSSMYASLGERVTITCKASQDI NSYLSWFQQKPGKSPKTLIYRANRLVDGVPSRFSGSGSGQDYSLTISSL EYEDMGIYYCLQYDEFPLTFGAGTKLELKRADAAPTVSIFPPSSEQLTS GGASVVCFLNNFYPKDINVKWKIDGSERQNGVLNSWTDQDSKDSTYSMS STLTLTKDEYERHNSYTCEATHKTSTSPIVKSFNRNEC |
| | Variable region | MRTPAQFLGILLLWFPGIKCDIKMTQSPSSMYASLGERVTITCKASQDI NSYLSWFQQKPGKSPKTLIYRANRLVDGVPSRFSGSGSGQDYSLTISSL EYEDMGIYYCLQYDEFPLTFGAGTKLELK |
| | CDR1 | KASQDINSYLS |
| | CDR2 | RANRLVD |
| | CDR3 | LQYDEFPLT |

In one aspect, the IgA antibody that binds to enteric bacteria of the present disclosure includes:
a heavy chain variable region containing an amino acid sequence of a heavy chain CDR1, an amino acid sequence of a heavy chain CDR2, and an amino acid sequence of a heavy chain CDR3, the heavy chain variable region containing an amino acid sequence set forth in SEQ ID NO: 17, and
a light chain variable region containing an amino acid sequence of a light chain CDR1, an amino acid sequence of a light chain CDR2, and an amino acid sequence of a light chain CDR3, the light chain variable region containing an amino acid sequence set forth in SEQ ID NO: 18.

| Sequence name (SEQ ID NO:) | Amino acid sequence |
|---|---|
| SNK0002_HV (SEQ ID NO: 17) | MGRLTSSFLLLIVPAYVLSQVTLKESGPGILQPSQ TLSLTCSFSGFSLSTFGMGVGWIRQPSGKGLEWLA HIWWDDDKYYNPALKSRLTISKDTSKNQVFLKIAN VDTADTATYYCARIAGFDYWGQGTTLTVSS |
| SNK0002_LV (SEQ ID NO: 18) | MHFQVQIFSFLLISASVIMSRGQIVLTQSPAIMSA SPGEKVTITCSASSSVSYMHWFQQKPGTSPKLWIY STSNLASGVPARFSGSGSGTSYSLTISRMEAEDAA TYYCQQRSSYPYTFGGGTKLEIK |

In one aspect, the monoclonal antibody that binds to enteric bacteria of the present disclosure includes:
a heavy chain variable region containing:
a heavy chain CDR1 containing an amino acid sequence set forth in SEQ ID NO: 11;
a heavy chain CDR2 containing an amino acid sequence set forth in SEQ ID NO: 12; and
a heavy chain CDR3 containing an amino acid sequence set forth in SEQ ID NO: 13; and
a light chain variable region containing:
a light chain CDR1 containing an amino acid sequence set forth in SEQ ID NO: 14;
a light chain CDR2 containing an amino acid sequence set forth in SEQ ID NO: 15; and
a light chain CDR3 containing an amino acid sequence set forth in SEQ ID NO: 16.

| Sequence name (SEQ ID NO:) | Amino acid sequence |
|---|---|
| SNK0002_HCDR1 (SEQ ID NO: 11) | TFGMG |
| SNK0002_HCDR2 (SEQ ID NO: 12) | LAHIWWDDDKYYNPAL |
| SNK0002_HCDR3 (SEQ ID NO: 13) | YYCARIAG |
| SNK0002_LCDR1 (SEQ ID NO: 14) | SASSSVSYMHW |
| SNK0002_LCDR2 (SEQ ID NO: 15) | STSNLASG |
| SNK0002_LCDR3 (SEQ ID NO: 16) | QRSSYPYTF |

In one aspect, the monoclonal antibody that binds to enteric bacteria of the present disclosure includes:
a heavy chain variable region containing an amino acid sequence set forth in SEQ ID NO: 17 or a sequence having at least 90%, at least 95%, at least 98%, or at least 99% sequence identity therewith; and
a light chain variable region containing an amino acid sequence set forth in SEQ ID NO: 18 or a sequence having at least 90%, at least 95%, at least 98%, or at least 99% sequence identity therewith.

In one aspect, the monoclonal antibody that binds to enteric bacteria of the present disclosure is an IgA recombinant purified antibody SNK0002AR. The IgA recombinant purified antibody is produced by applying a recombination technique using a nucleic acid sequence encoding an IgM antibody SNK0002M produced by a hybridoma obtained from spleen-derived B cells. Similar to SNK0002AR, the IgM antibody SNK0002M, or other classes of antibodies with similar variable regions, can be used as the monoclonal antibodies of the present disclosure.

The antibody SNK0002AR has the following amino acid sequence configuration.

| Amino acid sequence of SNK0002AR | | |
|---|---|---|
| Heavy chain | Full-length sequence | MGRLTSSFLLLIVPAYVLSQVTLKESGPGILQPSQTLSLTCSFSGFSLSTFGMGV GWIRQPSGKGLEWLAHIWWDDDKYYNPALKSRLTISKDTSKNQVFLKIANVDTAD TATYYCARIAGFDYWGQGTTLTVSSESARNPTIYPLTLPRALSSDPVIIGCLIHD YFPSGTMNVTWGKSGKDITTVNFPPALASGGGYTMSSQLTLPAVECPEGESVKCS VQHDSNAVQELDVKCSGPPPPCPPCPPSCHPSLSLQRPALEDLLLGSDASLTCTL NGLRNPEGAVFTWEPSTGKDAVQKKAVQNSCGCYSVSSVLPGCAERWNSGASFKC TVTHPESDTLTGTIAKITVNTFPPQVHLLPPPSEELALNELVSLTCLVRAFNPKE VLVRWLHGNEELSPESYLVFEPLKEPGEGATTYLVTSVLRVSAELWKQGDQYSCM VGHEALPMNFTQKTIDRLSGKPTNVSVSVIMSEGDGICY |
| | Variable region | MGRLTSSFLLLIVPAYVLSQVTLKESGPGILQPSQTLSLTCSFSGFSLSTFGMGV GWIRQPSGKGLEWLAHIWWDDDKYYNPALKSRLTISKDTSKNQVFLKIANVDTAD TATYYCARIAGFDYWGQGTTLTVSS |
| | CDR1 | TFGMG |
| | CDR2 | LAHIWWDDDKYYNPAL |
| | CDR3 | YYCARIAG |
| Light chain | Full-length sequence | MHFQVQIFSFLLISASVIMSRGQIVLTQSPAIMSASPGEKVTITCSASSSVSYMH WFQQKPGTSPKLWIYSTSNLASGVPARFSGSGSGTSYSLTISRMEAEDAATYYCQ QRSSYPYTFGGGTKLEIKRADAAPTVSIFPPSSEQLTSGGASVVCFLNNFYPKDI NVKWKIDGSERQNGVLNSWTDQDSKDSTYSMSSTLTLTKDEYERHNSYTCEATHK TSTSPIVKSFNRNEC |
| | Variable region | MHFQVQIFSFLLISASVIMSRGQIVLTQSPAIMSASPGEKVTITCSASSSVSYMH WFQQKPGTSPKLWIYSTSNLASGVPARFSGSGSGTSYSLTISRMEAEDAATYYCQ QRSSYPYTFGGGTKLEIK |
| | CDR1 | SASSSVSYMHW |
| | CDR2 | STSNLASG |
| | CDR3 | QRSSYPYTF |

In one aspect, the monoclonal antibody that binds to enteric bacteria of the present disclosure includes:
a heavy chain variable region containing an amino acid sequence of a heavy chain CDR1, an amino acid sequence of a heavy chain CDR2, and an amino acid sequence of a heavy chain CDR3, the heavy chain variable region containing an amino acid sequence set forth in SEQ ID NO: 27, and
a light chain variable region containing an amino acid sequence of a light chain CDR1, an amino acid sequence of a light chain CDR2, and an amino acid sequence of a light chain CDR3, the light chain variable region containing an amino acid sequence set forth in SEQ ID NO: 28.

| Sequence name (SEQ ID NO:) | Amino acid sequence |
|---|---|
| SNK0003_HV (SEQ ID NO: 27) | MGFSRIFLFLLSVTTGVHSQAYLQQSGAELVRPGAS VRMSCKASDYTFTSYNIHWVKQTPRQGLEWIGAIYS GNGATSHNQKFKGRATLTVDKSSSTAYMQLSSLTSE DSAVYFCTRVGLRSPFDFWGQGTTLTVSS |
| SNK0003_LV (SEQ ID NO: 28) | MESQTQVFVYMLLWLSGVDGDIVMTQSLKFMST SGGDRVSVTCKASQSVGTSVAWYQQKPGQSPKP LIYSASYRYSGVPDRFTGSGSGTDFSLTISNVQ SEDLAEYFCQQYNNYPYTFGGGTKLEIK |

In one aspect, the monoclonal antibody that binds to enteric bacteria of the present disclosure includes:
a heavy chain variable region containing:
a heavy chain CDR1 containing an amino acid sequence set forth in SEQ ID NO: 21;
a heavy chain CDR2 containing an amino acid sequence set forth in SEQ ID NO: 22; and
a heavy chain CDR3 containing an amino acid sequence set forth in SEQ ID NO: 23; and
a light chain variable region containing:
a light chain CDR1 containing an amino acid sequence set forth in SEQ ID NO: 24;
a light chain CDR2 containing an amino acid sequence set forth in SEQ ID NO: 25; and
a light chain CDR3 containing an amino acid sequence set forth in SEQ ID NO: 26.

| Sequence name (SEQ ID NO:) | Amino acid sequence |
|---|---|
| SNK0003_HCDR1 (SEQ ID NO: 21) | SYNIH |
| SNK0003_HCDR2 (SEQ ID NO: 22) | AIYSGNGATSHNQKFK |
| SNK0003_HCDR3 (SEQ ID NO: 23) | FCTRVGLR |
| SNK0003_LCDR1 (SEQ ID NO: 24) | KASQSVGTSVA |
| SNK0003_LCDR2 (SEQ ID NO: 25) | SASYRYS |
| SNK0003_LCDR3 (SEQ ID NO: 26) | QQYNNYPYT |

In one aspect, the monoclonal antibody that binds to enteric bacteria of the present disclosure includes:
a heavy chain variable region containing an amino acid sequence set forth in SEQ ID NO: 27 or a sequence having at least 90%, at least 95%, at least 98%, or at least 99% sequence identity therewith; and
a light chain variable region containing an amino acid sequence set forth in SEQ ID NO: 28 or a sequence having at least 90%, at least 95%, at least 98%, or at least 99% sequence identity therewith.

In one aspect, the monoclonal antibody that binds to enteric bacteria of the present disclosure is IgA antibody SNK0003A produced by a hybridoma obtained from B cells derived from intestinal mucosa lamina propria. IgA antibody recombinant purified antibody SNK0003AR produced by applying a recombination technique using a nucleic acid sequence encoding the antibody SNK0003A can also be used in the same manner. In addition, other classes of antibodies with similar variable regions can be used as the monoclonal antibodies of the present disclosure.

The antibody SNK0003A has the following amino acid sequence configuration.

| Amino acid sequence of SNK0003A | | |
|---|---|---|
| Heavy chain | Full-length sequence | MGFSRIFLFLLSVTTGVHSQAYLQQSGAELVRPGASVRMSCKASDYTFTSYNIHW VKQTPRQGLEWIGAIYSGNGATSHNQKFKGRATLTVDKSSSTAYMQLSSLTSEDS AVYFCTRVGLRSPFDFWGQGTTLTVSSESARNPTIYPLTLPRALSSDPVIIGCLI HDYFPSGTMNVTWGKSGKDITTVNFPPALASGGGYTMSSQLTLPAVECPEGESVK CSVQHDSNAVQELDVKCSGPPPPCPPCPPSCHPSLSLQRPALEDLLLGSDASLTC TLNGLRNPEGAVFTWEPSTGKDAVQKKAVQNSCGCYSVSSVLPGCAERWNSGASF KCTVTHPESDTLTGTIAKITVNTFPPQVHLLPPPSEELALNELVSLTCLVRAFNP KEVLVRWLHGNEELSPESYLVFEPLKEPGEGATTYLVTSVLRVSAELWKQGDQYS CMVGHEALPMNFTQKTIDRLSGKPTNVSVSVIMSEGDGICY |
| | Variable region | MGFSRIFLFLLSVTTGVHSQAYLQQSGAELVRPGASVRMSCKASDYTFTSYNIHW VKQTPRQGLEWIGAIYSGNGATSHNQKFKGRATLTVDKSSSTAYMQLSSLTSEDS AVYFCTRVGLRSPFDFWGQGTTLTVSS |
| | CDR1 | SYNIH |
| | CDR2 | AIYSGNGATSHNQKFK |
| | CDR3 | FCTRVGLR |
| Light chain | Full-length sequence | MESQTQVFVYMLLWLSGVDGDIVMTQSLKFMSTSGGDRVSVTCKASQSVGTSVAW YQQKPGQSPKPLIYSASYRYSGVPDRFTGSGSGTDFSLTISNVQSEDLAEYFCQQ YNNYPYTFGGGTKLEIKRADAAPTVSIFPPSSEQLTSGGASVVCFLNNFYPKDIN VKWKIDGSERQNGVLNSWTDQDSKDSTYSMSSTLTLTKDEYERHNSYTCEATHKT STSPIVKSFNRNEC |
| | Variable region | MESQTQVFVYMLLWLSGVDGDIVMTQSLKFMSTSGGDRVSVTCKASQSVGTSVAW YQQKPGQSPKPLIYSASYRYSGVPDRFTGSGSGTDFSLTISNVQSEDLAEYFCQQ YNNYPYTFGGGTKLEIK |
| | CDR1 | KASQSVGTSVA |
| | CDR2 | SASYRYS |
| | CDR3 | QQYNNYPYT |

In one aspect, the monoclonal antibody that binds to enteric bacteria of the present disclosure is an antibody containing,
with respect to a reference antibody including a heavy chain variable region containing an amino acid sequence set forth in SEQ ID NO: 37 and a light chain variable region containing an amino acid sequence set forth in SEQ ID NO: 38,
at least one amino acid mutation in at least one region selected from heavy chains CDR1 to 3, light chains CDR1 to 3, and a light chain FR1, and
binding to the amino acid sequence RQEEHIELIAS (SEQ ID NO: 72) in *E. coli* SHMT protein and the amino acid sequence VLDMMKLEKPE (SEQ ID NO: 73) in C. *difficile* iPGM protein.

| Sequence name (SEQ ID NO:) | Amino acid sequence |
|---|---|
| W27G2_HV (SEQ ID NO: 37) | MKCSWIIFFLMAVVTGVNSEVQLQQSGSEL VKSGASVKLSCTVSGFNFTDYYIHWVRQRT EQGLEWIGRIDPENDETTYAPKFQGKATMT ADTSSNTAYLQLTSLTSEDTAVYYCARSTV LDYWGHGTTLTVSS |
| W27G2_LV (SEQ ID NO: 38) | MKLPVRLLVLMFWIPGFSSDVLMTQTPLSL PVSLGDQASISCRASQSIVHTNGNTYLEWY LQKPGQSPKLLIYKVSNRFSGVPDRFSGSG SGTDFILKISRVEAEDLGVYYCFQGSHVPP TFGGGTKLEVK |

The at least one amino acid mutation of the antibody can be identified by applying a technique available to those skilled in the art by using results of three-dimensional structure analysis of conjugates of an antibody containing an amino acid sequence of the reference antibody and *E. coli* SHMT protein, and an antibody containing an amino acid sequence of the reference antibody and C. *difficile* iPGM protein. The number of amino acid mutations to the reference antibody may be 1, 2, 3, 4, 5, 6, 7, 8, 9, 10, 11, 12, 13, 14, 15, 16, 17, 18, 19, 20, or more.

Such a reference antibody is not particularly limited as long as it is an antibody including W27G2_HV (SEQ ID NO: 37) as a heavy chain variable region and W27G2_LV (SEQ ID NO: 38) as a light chain variable region. For example, an IgG antibody (W27RS_H000_L000GR) can be used. The antibody W27RS_H000_L000GR includes the same heavy chain variable region and light chain variable region as the antibody W27G2 produced by the hybridoma obtained from B cells derived from intestinal mucosa lamina propria.

| Amino acid sequence of W27RS_H000_L000GR | | |
|---|---|---|
| Heavy chain | Variable region | MKCSWIIFFLMAVVTGVNSEVQLQQSGSELVKSGASVKLSCTVSGFNFTDYYIHW VRQRTEQGLEWIGRIDPENDETTYAPKFQGKATMTADTSSNTAYLQLTSLTSEDT AVYYCARSTVLDYWGHGTTLTVSS |
| | CDR1 | DYYIH |
| | CDR2 | RIDPENDETTYAPKFQ |
| | CDR3 | YCARSTVL |
| Light chain | Variable region | MKLPVRLLVLMFWIPGFSSDVLMTQTPLSLPVSLGDQASISCRASQSIVHTNGNTYLE WYLQKPGQSPKLLIYKVSNRFSGVPDRFSGSGSGTDFILKISRVEAEDLGVYYCFQGS HVPPTFGGGTKLEVK |
| | CDR1 | RASQSIVHTNG |
| | CDR2 | KLLIYKV |
| | CDR3 | GVYYCFQGS |

It can be confirmed by a method well known to those skilled in the art, such as ELISA or Western blot, that an antibody containing an identified mutation actually binds to the amino acid sequence RQEEHIELIAS in *E. coli* SHMT protein and the amino acid sequence VLDMMKLEKPE in C. *difficile* iPGM protein.

In one aspect, the monoclonal antibody that binds to enteric bacteria of the present disclosure is an antibody including:
a heavy chain variable region containing:
a heavy chain CDR1 containing an amino acid sequence of X₁YYIH;
a heavy chain CDR2 containing an amino acid sequence of RIDPENX₂X₃TTYAPKFQ; and
a heavy chain CDR3 containing an amino acid sequence of YCARSTVL; and
a light chain variable region containing:
   a light chain CDR1 containing an amino acid sequence of RX₄SQSIVHTNG;
   a light chain CDR2 containing an amino acid sequence of KLLIYKV; and
   a light chain CDR3 containing an amino acid sequence of GVYYFQGS,
   in which a light chain FR1 contains an amino acid sequence of TPLSLPVSLGDQA or an amino acid sequence of SPASX₅SVSLGDRX₆,
   X₁, X₂, and X₃ are each independently a neutral polar amino acid or an acidic polar amino acid,
   X₄ is a non-polar amino acid or a neutral polar amino acid, and
   X₅ and X₆ are each independently a non-polar amino acid.

The antibody is designed to have a binding mode similar to that of the W27G2 antibody with respect to the amino acid sequence RQEEHIELIAS in *E. coli* SHMT protein and the amino acid sequence VLDMMKLEKPE in C. *difficile* iPGM protein by three-dimensional structure analysis, and actually exhibits a binding mode similar to that of the W27G2 antibody and a bacterial growth inhibitory effect on these bacteria.

In one aspect, the monoclonal antibody that binds to enteric bacteria of the present disclosure is an antibody including:
a heavy chain variable region containing:
a heavy chain CDR1 containing an amino acid sequence of X₁YYIH;
a heavy chain CDR2 containing an amino acid sequence of RIDPENX₂X₃TTYAPKFQ; and
a heavy chain CDR3 containing an amino acid sequence of YCARSTVL; and
a light chain variable region containing:
   a light chain CDR1 containing an amino acid sequence of RX₄SQSIVHTNG;
   a light chain CDR2 containing an amino acid sequence of KLLIYKV; and
   a light chain CDR3 containing an amino acid sequence of GVYYFQGS,
   in which a light chain FR1 contains an amino acid sequence of TPLSLPVSLGDQA or an amino acid sequence of SPASX₅SVSLGDRX₆,
   X₁ and X₂ are each independently asparagine or aspartic acid,
   X₃ is glutamine or glutamic acid,
   X₄ is alanine or serine,
   X₅ is leucine or methionine, and
   X₆ is alanine or valine.

In one aspect, the heavy chain of the monoclonal antibody that binds to enteric bacteria of the present disclosure includes:
a heavy chain CDR1 containing an amino acid sequence set forth in SEQ ID NO: 31 or 41;
a heavy chain CDR2 containing an amino acid sequence set forth in any one of SEQ ID NOs: 32 and 42 to 44; and
a heavy chain CDR3 containing an amino acid sequence set forth in SEQ ID NO: 33.

| Sequence name (SEQ ID NO:) | Amino acid sequence |
|---|---|
| W27G2_HCDR1 (SEQ ID NO: 31) | DYYIH |
| W27G2_HCDR2 (SEQ ID NO: 32) | RIDPENDETTYAPKFQ |
| W27G2_HCDR3 (SEQ ID NO: 33) | YCARSTVL |
| RS_HCDR1_D50N (SEQ ID NO: 41) | NYYIH |
| RS_HCDR2_D75N (SEQ ID NO: 42) | RIDPENNETTYAPKFQ |
| RS_HCDR2_E76Q (SEQ ID NO: 43) | RIDPENDQTTYAPKFQ |
| RS_HCDR2_D75N_E76Q (SEQ ID NO: 44) | RIDPENNQTTYAPKFQ |

In one aspect, the heavy chain of the monoclonal antibody that binds to enteric bacteria of the present disclosure includes:
a heavy chain CDR1 containing an amino acid sequence set forth in SEQ ID NO: 31;
a heavy chain CDR2 containing an amino acid sequence set forth in SEQ ID NO: 32; and
a heavy chain CDR3 containing an amino acid sequence set forth in SEQ ID NO: 33.

In one aspect, the heavy chain of the monoclonal antibody that binds to enteric bacteria of the present disclosure includes:
a heavy chain CDR1 containing an amino acid sequence set forth in SEQ ID NO: 31;
a heavy chain CDR2 containing an amino acid sequence set forth in SEQ ID NO: 42; and
a heavy chain CDR3 containing an amino acid sequence set forth in SEQ ID NO: 33.

In one aspect, the heavy chain of the monoclonal antibody that binds to enteric bacteria of the present disclosure includes:
a heavy chain CDR1 containing an amino acid sequence set forth in SEQ ID NO: 31;
a heavy chain CDR2 containing an amino acid sequence set forth in SEQ ID NO: 43; and
a heavy chain CDR3 containing an amino acid sequence set forth in SEQ ID NO: 33.

In one aspect, the heavy chain of the monoclonal antibody that binds to enteric bacteria of the present disclosure includes:
a heavy chain CDR1 containing an amino acid sequence set forth in SEQ ID NO: 31;
a heavy chain CDR2 containing an amino acid sequence set forth in SEQ ID NO: 44; and
a heavy chain CDR3 containing an amino acid sequence set forth in SEQ ID NO: 33.

In one aspect, the heavy chain of the monoclonal antibody that binds to enteric bacteria of the present disclosure includes:
a heavy chain CDR1 containing an amino acid sequence set forth in SEQ ID NO: 41;
a heavy chain CDR2 containing an amino acid sequence set forth in SEQ ID NO: 32; and
a heavy chain CDR3 containing an amino acid sequence set forth in SEQ ID NO: 33.

In one aspect, the heavy chain of the monoclonal antibody that binds to enteric bacteria of the present disclosure includes:
a heavy chain CDR1 containing an amino acid sequence set forth in SEQ ID NO: 41;
a heavy chain CDR2 containing an amino acid sequence set forth in SEQ ID NO: 42; and
a heavy chain CDR3 containing an amino acid sequence set forth in SEQ ID NO: 33.

In one aspect, the heavy chain of the monoclonal antibody that binds to enteric bacteria of the present disclosure includes:
a heavy chain CDR1 containing an amino acid sequence set forth in SEQ ID NO: 41;
a heavy chain CDR2 containing an amino acid sequence set forth in SEQ ID NO: 43; and
a heavy chain CDR3 containing an amino acid sequence set forth in SEQ ID NO: 33.

In one aspect, the heavy chain of the monoclonal antibody that binds to enteric bacteria of the present disclosure includes:
a heavy chain CDR1 containing an amino acid sequence set forth in SEQ ID NO: 41;
a heavy chain CDR2 containing an amino acid sequence set forth in SEQ ID NO: 44; and
a heavy chain CDR3 containing an amino acid sequence set forth in SEQ ID NO: 33.

In one aspect, the light chain of the monoclonal antibody that binds to enteric bacteria of the present disclosure includes:
a light chain CDR1 containing an amino acid sequence set forth in SEQ ID NO: 34 or 52;
a light chain CDR2 containing an amino acid sequence set forth in SEQ ID NO: 35; and
a light chain CDR3 containing an amino acid sequence set forth in SEQ ID NO: 36.

| Sequence name (SEQ ID NO:) | Amino acid sequence |
|---|---|
| W27G2_LCDR1 (SEQ ID NO: 34) | RASQSIVHTNG |
| W27G2_LCDR2 (SEQ ID NO: 35) | KLLIYKV |
| W27G2_LCDR3 (SEQ ID NO: 36) | GVYYCFQGS |
| RS_LCDR1_A44S (SEQ ID NO: 52) | RSSQSIVHTNG |

In one aspect, the light chain of the monoclonal antibody that binds to enteric bacteria of the present disclosure includes:
a light chain CDR1 containing an amino acid sequence set forth in SEQ ID NO: 34;
a light chain CDR2 containing an amino acid sequence set forth in SEQ ID NO: 35; and
a light chain CDR3 containing an amino acid sequence set forth in SEQ ID NO: 36.

In one aspect, the light chain of the monoclonal antibody that binds to enteric bacteria of the present disclosure includes:
a light chain CDR1 containing an amino acid sequence set forth in SEQ ID NO: 52;
a light chain CDR2 containing an amino acid sequence set forth in SEQ ID NO: 35; and
a light chain CDR3 containing an amino acid sequence set forth in SEQ ID NO: 36.

In one aspect, the monoclonal antibody that binds to enteric bacteria of the present disclosure or the antigen-binding fragment thereof contains a sequence that binds to Protein L in the light chain variable region. The antibody or the antigen-binding fragment thereof containing the sequence that binds to Protein L can be purified using a Protein L column.

In one aspect, an antibody that binds to a C. *difficile* bacterial body and contains a sequence that binds to Protein L of the present disclosure or an antigen-binding fragment thereof includes a light chain variable region containing the SPASX₅SVSLGDRX₆ amino acid sequence, in which X₅ and X₆ are each independently a non-polar amino acid.

In one aspect, the antibody that binds to a C. *difficile* bacterial body and contains a sequence that binds to Protein L of the present disclosure or the antigen-binding fragment thereof includes a light chain variable region containing an amino acid sequence of SPASX₅SVSLGDRX₆, in which X₅ is leucine or methionine and X₆ is alanine or valine.

In one aspect, the antibody that binds to a C. *difficile* bacterial body and contains a sequence that binds to Protein L of the present disclosure or the antigen-binding fragment thereof includes a light chain variable region containing an amino acid sequence set forth in one selected from the group consisting of SEQ ID NOs: 53 to 55.

| Sequence name (SEQ ID NO:) | Amino acid sequence |
|---|---|
| RS_LFR1_L001 (SEQ ID NO: 53) | SPASLSVSLGDRA |
| RS_LFR1_L002 (SEQ ID NO: 54) | SPASLSVSLGDRV |
| RS_LFR1_L003 (SEQ ID NO: 55) | SPASMSVSLGDRA |
| RS_LFR1_L000 (SEQ ID NO: 56) | TPLSLPVSLGDQA |

In one aspect, the antibody that binds to a C. *difficile* bacterial body and contains a sequence that binds to Protein L of the present disclosure or the antigen-binding fragment thereof includes a light chain variable region containing an amino acid sequence set forth in SEQ ID NO: 54.

The sequence is obtained by introducing a mutation into the sequence TPLSLPVSLGDQA (SEQ ID NO: 56) of the light chain FR1 region of the W27G2 antibody so that Protein L binds to the antibody molecule.

In one aspect, the antibody that binds to a C. *difficile* bacterial body and contains a sequence that binds to Protein L of the present disclosure or the antigen-binding fragment thereof includes a light chain variable region containing an amino acid sequence set forth in SEQ ID NO: 55.

This sequence is obtained by further introducing a mutation into the light chain FR1 region of the light chain variable region containing an amino acid sequence set forth in SEQ ID NO: 54 so that Protein L binds to the antibody molecule more firmly.

In one aspect, the antibody that binds to a C. *difficile* bacterial body and contains a sequence that binds to Protein L of the present disclosure or the antigen-binding fragment thereof includes a light chain variable region containing an amino acid sequence set forth in SEQ ID NO: 58.

This sequence is obtained by further introducing a mutation into the light chain FR1 region of the light chain variable region containing an amino acid sequence set forth in SEQ ID NO: 54 so that Protein L binds to the antibody molecule more firmly.

The monoclonal antibody that binds to enteric bacteria of the present disclosure may include any combination of the heavy chain and the light chain described above, or may not contain a sequence that binds to Protein L.

In one aspect, the monoclonal antibody that binds to enteric bacteria of the present disclosure or the antigen-binding fragment thereof is an antibody including:
a heavy chain variable region containing:
a heavy chain CDR1 containing an amino acid sequence set forth in SEQ ID NO: 31 or 41;
a heavy chain CDR2 containing an amino acid sequence set forth in any one of SEQ ID NOs: 32 and 42 to 44; and
a heavy chain CDR3 containing an amino acid sequence set forth in SEQ ID NO: 33; and
a light chain variable region containing:
   a light chain CDR1 containing an amino acid sequence set forth in SEQ ID NO: 34 or 52;
   a light chain CDR2 containing an amino acid sequence set forth in SEQ ID NO: 35;
   a light chain CDR3 containing an amino acid sequence set forth in SEQ ID NO: 36; and
   a light chain FR1 containing an amino acid sequence set forth in any one of SEQ ID NOs: 53 to 56.

For example, the monoclonal antibody that binds to enteric bacteria of the present disclosure includes a combination of any of the following heavy chain variable regions and any of the following light chain variable regions.

| Heavy chain variable region | Amino acid sequence |
|---|---|
| W27G2_HV (SEQ ID NO: 37) | MKCSWIIFFLMAVVTGVNSEVQLQQSGSELVKSGASVKLS CTVSGFNFTDYYIHWVRQRTEQGLEWIGRIDPENDETTYA PKFQGKATMTADTSSNTAYLQLTSLTSEDTAVYYCARSTV LDYWGHGTTLTVSS |
| RS_HV001 (SEQ ID NO: 45) | MKCSWIIFFLMAVVTGVNSEVQLQQSGSELVKSGASVKLS CTVSGFNFTNYYIHWVRQRTEQGLEWIGRIDPENDETTYA PKFQGKATMTADTSSNTAYLQLTSLTSEDTAVYYCARSTV LDYWGHGTTLTVSS |
| RS_HV002 (SEQ ID NO: 46) | MKCSWIIFFLMAVVTGVNSEVQLQQSGSELVKSGASVKLS CTVSGFNFTDYYIHWVRQRTEQGLEWIGRIDPENNETTYA PKFQGKATMTADTSSNTAYLQLTSLTSEDTAVYYCARSTV LDYWGHGTTLTVSS |
| RS_HV003 (SEQ ID NO: 47) | MKCSWIIFFLMAVVTGVNSEVQLQQSGSELVKSGASVKLS CTVSGFNFTDYYIHWVRQRTEQGLEWIGRIDPENDQTTYA PKFQGKATMTADTSSNTAYLQLTSLTSEDTAVYYCARSTV LDYWGHGTTLTVSS |
| RS_HV004 (SEQ ID NO: 48) | MKCSWIIFFLMAVVTGVNSEVQLQQSGSELVKSGASVKLS CTVSGFNFTNYYIHWVRQRTEQGLEWIGRIDPENNETTYA PKFQGKATMTADTSSNTAYLQLTSLTSEDTAVYYCARSTV LDYWGHGTTLTVSS |
| RS_HV005 (SEQ ID NO: 49) | MKCSWIIFFLMAVVTGVNSEVQLQQSGSELVKSGASVKLS CTVSGFNFTNYYIHWVRQRTEQGLEWIGRIDPENDQTTYA PKFQGKATMTADTSSNTAYLQLTSLTSEDTAVYYCARSTV LDYWGHGTTLTVSS |
| RS_HV006 (SEQ ID NO: 50) | MKCSWIIFFLMAVVTGVNSEVQLQQSGSELVKSGASVKLS CTVSGFNFTDYYIHWVRQRTEQGLEWIGRIDPENNQTTYA PKFQGKATMTADTSSNTAYLQLTSLTSEDTAVYYCARSTV LDYWGHGTTLTVSS |
| RS_HV007 (SEQ ID NO: 51) | MKCSWIIFFLMAVVTGVNSEVQLQQSGSELVKSGASVKLS CTVSGFNFTNYYIHWVRQRTEQGLEWIGRIDPENNQTTYA PKFQGKATMTADTSSNTAYLQLTSLTSEDTAVYYCARSTV LDYWGHGTTLTVSS |

| Light chain variable region | Amino acid sequence |
|---|---|
| W27G2_LV (SEQ ID NO: 38) | MKLPVRLLVLMFWIPGFSSDVLMTQTPASLPVSLGDQASISC RASQSIVHTNGNTYLEWYLQKPGQSPKLLIYKVSNRFSGVPD RFSGSGSGTDFILKISRVEAEDLGVYYCFQGSHVPPTFGGGT KLEVK |
| RS_LV001 (SEQ ID NO: 57) | MKLPVRLLVLMFWIPGFSSDVLMTQSPASLSVSLGDRATISC RASQSIVHTNGNTYLEWYLQKPGQSPKLLIYKVSNRFSGVPD RFSGSGSGTDFILKISRVEAEDLGVYYCFQGSHVPPTFGGGT KLEVK |
| RS_LV002 (SEQ ID NO: 58) | MKLPVRLLVLMFWIPGFSSDVLMTQSPASLSVSLGDRVTISC RASQSIVHTNGNTYLEWYLQKPGQSPKLLIYKVSNRFSGVPD RFSGSGSGTDFILKISRVEAEDLGVYYCFQGSHVPPTFGGGT KLEVK |
| RS_LV003 (SEQ ID NO: 59) | MKLPVRLLVLMFWIPGFSSDVLMTQSPASLSVSLGDRVTISC RSSQSIVHTNGNTYLEWYLQKPGQSPKLLIYKVSNRFSGVPD RFSGSGSGTDFILKISRVEAEDLGVYYCFQGSHVPPTFGGGT KLEVK |
| RS_LV004 (SEQ ID NO: 60) | MKLPVRLLVLMFWIPGFSSDVLMTQSPASMSVSLGDRATISC RSSQSIVHTNGNTYLEWYLQKPGQSPKLLIYKVSNRFSGVPD RFSGSGSGTDFILKISRVEAEDLGVYYCFQGSHVPPTFGGGT KLEVK |
| RS_LV005 (SEQ ID NO: 61) | MKLPVRLLVLMFWIPGFSSDVLMTQSPASLSVSLGDRATISC RSSQSIVHTNGNTYLEWYLQKPGQSPKLLIYKVSNRFSGVPD RFSGSGSGTDFILKISRVEAEDLGVYYCFQGSHVPPTFGGGT KLEVK |

Preferably, the monoclonal antibody that binds to enteric bacteria of the present disclosure includes a combination of the following heavy chain variable region and light chain variable region.

| Heavy chain variable region | Light chain variable region |
|---|---|
| W27G2_HV (SEQ ID NO: 37) | RS_LV001 (SEQ ID NO: 57) |
| W27G2_HV (SEQ ID NO: 37) | RS_LV004 (SEQ ID NO: 60) |
| W27G2_HV (SEQ ID NO: 37) | RS_LV005 (SEQ ID NO: 61) |
| RS_HV001 (SEQ ID NO: 45) | RS_LV005 (SEQ ID NO: 61) |
| RS_HV002 (SEQ ID NO: 46) | RS_LV005 (SEQ ID NO: 61) |
| RS_HV003 (SEQ ID NO: 47) | RS_LV005 (SEQ ID NO: 61) |
| RS_HV004 (SEQ ID NO: 48) | RS_LV005 (SEQ ID NO: 61) |
| RS_HV005 (SEQ ID NO: 49) | RS_LV005 (SEQ ID NO: 61) |
| RS_HV006 (SEQ ID NO: 50) | RS_LV005 (SEQ ID NO: 61) |
| RS_HV007 (SEQ ID NO: 51) | RS_LV001 (SEQ ID NO: 57) |
| RS_HV007 (SEQ ID NO: 51) | RS_LV002 (SEQ ID NO: 58) |
| RS_HV007 (SEQ ID NO: 51) | RS_LV003 (SEQ ID NO: 59) |
| RS_HV007 (SEQ ID NO: 51) | RS_LV004 (SEQ ID NO: 60) |
| RS_HV007 (SEQ ID NO: 51) | RS_LV005 (SEQ ID NO: 61) |

Preferably, the monoclonal antibody that binds to enteric bacteria of the present disclosure includes the following.

| Antibody name | Heavy chain variable region | Light chain variable region | Explanation |
|---|---|---|---|
| SNK0001M | SNK0001_HV | SNK0001_LV | Hybridoma-producing IgM |
| SNK0001MR | SNK0001_HV | SNK0001_LV | CHO cell-producing recombinant IgM |
| SNK0001MA | SNK0001_HV | SNK0001_LV | CHO cell-producing recombinant IgA |
| SNK0002M | SNK0002_HV | SNK0002_LV | Hybridoma-producing IgM |
| SNK0002MR | SNK0002_HV | SNK0002_LV | CHO cell-producing recombinant IgM |
| SNK0002MA | SNK0002_HV | SNK0002_LV | CHO cell-producing recombinant IgA |
| SNK0003A | SNK0003_HV | SNK0003_LV | Hybridoma-producing IgA |
| SNK0003AR | SNK0003_HV | SNK0003_LV | CHO cell-producing recombinant IgA |
| W27G2 | W27G2_HV | W27G2_LV | Hybridoma-producing IgA |
| RS_H000_L001 | W27G2_HV | RS_LV001 | CHO cell-producing recombinant IgA Protein L binding type |
| RS H000 L005 | W27G2 HV | RS LV005 | CHO cell-producing recombinant IgA Protein L binding type |
| | | | Mutation to light chain CDR1 of W27G2 |
| RS_H001_L005 | RS_HV001 | RS_LV005 | CHO cell-producing recombinant IgA Protein L binding type |
| | | | Mutation to heavy chain CDR1 of W27G2 |
| | | | Mutation to light chain CDR1 of W27G2 |
| RS_H002_L005 | RS_HV002 | RS_LV005 | CHO cell-producing recombinant IgA Protein L binding type |
| | | | Mutation to heavy chain CDR2 of W27G2 |
| | | | Mutation to light chain CDR1 of W27G2 |
| RS_H003_L005 | RS_HV003 | RS_LV005 | CHO cell-producing recombinant IgA Protein L binding type |
| | | | Mutation to heavy chain CDR2 of W27G2 |
| | | | Mutation to light chain CDR1 of W27G2 |
| RS_H004_L005 | RS_HV004 | RS_LV005 | CHO cell-producing recombinant IgA Protein L binding type |
| | | | Mutation to heavy chains CDR1 and CDR2 of W27G2 |
| | | | Mutation to light chain CDR1 of W27G2 |
| RS_H005_L005 | RS_HV005 | RS_LV005 | CHO cell-producing recombinant IgA Protein L binding type |
| | | | Mutation to heavy chains CDR1 and CDR2 of W27G2 |
| | | | Mutation to light chain CDR1 of W27G2 |
| RS_H006_L005 | RS_HV006 | RS_LV005 | CHO cell-producing recombinant IgA Protein L binding type |
| | | | Mutation to heavy chain CDR2 of W27G2 Mutation to light chain CDR1 of W27G2 |
| RS_H007_L001 | RS_HV007 | RS_LV001 | CHO cell-producing recombinant IgA Protein L binding type |
| | | | Mutation to heavy chains CDR1 and CDR2 of W27G2 |
| RS_H007_L002 | RS_HV007 | RS_LV002 | CHO cell-producing recombinant IgA Protein L binding type |
| | | | Mutation to heavy chains CDR1 and CDR2 of W27G2 |
| RS_H007_L003 | RS_HV007 | RS LV003 | CHO cell-producing recombinant IgA Protein L binding type |
| | | | Mutation to heavy chains CDR1 and CDR2 of W27G2 Mutation to light chain CDR1 of W27G2 |
| RS_H007_L004 | RS_HV007 | RS_LV004 | CHO cell-producing recombinant IgA Protein L binding type |
| | | | Mutation to heavy chains CDR1 and CDR2 of W27G2 Mutation to light chain CDR1 of W27G2 |
| RS_H007_L005 | RS_HV007 | RS_LV005 | CHO cell-producing recombinant IgA Protein L binding type |
| | | | Mutation to heavy chains CDR1 and CDR2 of W27G2 |
| | | | Mutation to light chain CDR1 of W27G2 |

An antibody produced in CHO cells by introducing mutations into heavy chains or light chains of the W27G2 antibody so that antigen specificity is not substantially changed is referred to as an RS mutant recombinant purified antibody. These RS mutant recombinant purified antibodies further contain mutations for binding to Protein L.

The monoclonal antibody that binds to enteric bacteria of the present disclosure can have multispecificity that binds to a C. *difficile* bacterial body as well as to other disease-associated bacteria. For example, the IgA antibody that binds to enteric bacteria of the present disclosure further binds to *Fusobacterium nucleatum,* which is considered to be a causative bacterium of colorectal cancer, and preferably inhibits its growth.

The monoclonal antibody that binds to enteric bacteria of the present disclosure can contain a mutation in a constituent amino acid sequence, for example, a heavy chain variable region, light chain variable region, heavy chains CDR1 to 3, or light chains CDR1 to 3 thereof, as long as its antigen-binding characteristics are not lost. The mutation may be a substitution, a deletion, an insertion, or the like, and is not limited thereto. For example, in the case of the substitution, a conservative substitution technique can be adopted. Furthermore, the binding mode of the antibody and the antigen is analyzed in detail using three-dimensional structural analysis or the like, such that various mutations can be introduced into the monoclonal antibody that binds to enteric bacteria of the present disclosure as long as the antigen-binding characteristics are not lost.

A nucleic acid encoding the antibody of the present disclosure or the antigen-binding fragment thereof may be a ribonucleotide or a deoxynucleotide. In addition, the form of the nucleic acid is not particularly limited, and may be a single-stranded form or a double-stranded form. A codon used in the nucleic acid sequence is not particularly limited, and various codons can be appropriately selected and used according to the purpose. For example, it can be appropriately selected in consideration of codon frequency and the like according to the type of host cell to be adopted at the time of production. In one aspect, the nucleic acid encoding the antibody of the present disclosure or the antigen-binding fragment thereof is used for expressing and producing the antibody according to the present disclosure or the antigen-binding fragment thereof.

In a case where a base nucleic acid encoding the antibody of the present disclosure or the antigen-binding fragment thereof encodes an antigen-binding fragment, for example, two domains of Fv fragments and VL and VH may be encoded by separate nucleic acid molecules, or a single protein chain (single-stranded Fv (scFv)) in which a VL and VH region pair form a monovalent molecule may be encoded by a single nucleic acid using a recombinant technique.

Sequence information on the base nucleic acid encoding the antibody of the present disclosure or the antigen-binding fragment thereof is exemplified below.
>SEQ ID NO: 1:SNK0001_HCDR1
   SYGIS
>SEQ ID NO: 2: SNK0001_HCDR2
   EIYPRSGNTYYNEKFK
>SEQ ID NO: 3: SNK0001_HCDR3
   FCARLASS
>SEQ ID NO: 4: SNK0001_LCDR1
   KASQDINSYLS
>SEQ ID NO: 5: SNK0001_LCDR2
   RANRLVD
>SEQ ID NO: 6: SNK0001_LCDR3
   LQYDEFPLT
>SEQ ID NO: 7: SNK0001_HV 
MEWIWIFLFILSGTAGVQSQVQLQQSGAELARPGASVKLSCKASGYTFTSYGISWVKQR

TGQGLEWIGEIYPRSGNTYYNEKFKGKATLTADKSSSTAYMELRSLTSEDSAVYFCARL

ASSYYGSSYDWYFDVWGTGTTVTVSS
>SEQ ID NO: 8: SNK0001_LV 
MRTPAQFLGILLLWFPGIKCDIKMTQSPSSMYASLGERVTITCKASQDINSYLSWFQQK

PGKSPKTLIYRANRLVDGVPSRFSGSGSGQDYSLTISSLEYEDMGIYYCLQYDEFPLTF

GAGTKLELK
(SNK0001AR antibody heavy chain amino acid sequence)
>SEQ ID NO: 9: SNK0001AR_H 
MEWIWIFLFILSGTAGVQSQVQLQQSGAELARPGASVKLSCKASGYTFTSYGISWVKQR
TGQGLEWIGEIYPRSGNTYYNEKFKGKATLTADKSSSTAYMELRSLTSEDSAVYFCARL
ASSYYGSSYDWYFDVWGTGTTVTVSSESARNPTIYPLTLPRALSSDPVIIGCLIHDYFP
SGTMNVTWGKSGKDITTVNFPPALASGGGYTMSSQLTLPAVECPEGESVKCSVQHDSNA
VQELDVKCSGPPPPCPPCPPSCHPSLSLQRPALEDLLLGSDASLTCTLNGLRNPEGAVF
TWEPSTGKDAVQKKAVQNSCGCYSVSSVLPGCAERWNSGASFKCTVTHPESDTLTGTIA
KITVNTFPPQVHLLPPPSEELALNELVSLTCLVRAFNPKEVLVRWLHGNEELSPESYLV
FEPLKEPGEGATTYLVTSVLRVSAELWKQGDQYSCMVGHEALPMNFTQKTIDRLSGKPT
NVSVSVIMSEGDGICY
(SNK0001AR antibody light chain amino acid sequence)
>SEQ ID NO: 10: SNK0001AR_L 
MRTPAQFLGILLLWFPGIKCDIKMTQSPSSMYASLGERVTITCKASQDINSYLSWFQQK

PGKSPKTLIYRANRLVDGVPSRFSGSGSGQDYSLTISSLEYEDMGIYYCLQYDEFPLTF

GAGTKLELKRADAAPTVSIFPPSSEQLTSGGASVVCFLNNFYPKDINVKWKIDGSERQN

GVLNSWTDQDSKDSTYSMSSTLTLTKDEYERHNSYTCEATHKTSTSPIVKSFNRNEC
>SEQ ID NO: 11: SNK0002_HCDR1
   TFGMG
>SEQ ID NO: 12: SNK0002_HCDR2
   LAHIWWDDDKYYNPAL
>SEQ ID NO: 13: SNK0002_HCDR3
   YYCARIAG
>SEQ ID NO: 14: SNK0002_LCDR1
   SASSSVSYMHW
>SEQ ID NO: 15: SNK0002_LCDR2
   STSNLASG
>SEQ ID NO: 16: SNK0002_LCDR3
   QRSSYPYTF
>SEQ ID NO: 17: SNK0002_HV 
MGRLTSSFLLLIVPAYVLSQVTLKESGPGILQPSQTLSLTCSFSGFSLSTFGMGVGWIR
QPSGKGLEWLAHIWWDDDKYYNPALKSRLTISKDTSKNQVFLKIANVDTADTATYYCAR
IAGFDYWGQGTTLTVSS
>SEQ ID NO: 18: SNK0002_LV 
MHFQVQIFSFLLISASVIMSRGQIVLTQSPAIMSASPGEKVTITCSASSSVSYMHWFQQ

KPGTSPKLWIYSTSNLASGVPARFSGSGSGTSYSLTISRMEAEDAATYYCQQRSSYPYT

FGGGTKLEIK
(SNK0002AR antibody heavy chain amino acid sequence)
>SEQ ID NO: 19: SNK0002AR_H 
MGRLTSSFLLLIVPAYVLSQVTLKESGPGILQPSQTLSLTCSFSGFSLSTFGMGVGWIR
QPSGKGLEWLAHIWWDDDKYYNPALKSRLTISKDTSKNQVFLKIANVDTADTATYYCAR
IAGFDYWGQGTTLTVSSESARNPTIYPLTLPRALSSDPVIIGCLIHDYFPSGTMNVTWG
KSGKDITTVNFPPALASGGGYTMSSQLTLPAVECPEGESVKCSVQHDSNAVQELDVKCS
GPPPPCPPCPPSCHPSLSLQRPALEDLLLGSDASLTCTLNGLRNPEGAVFTWEPSTGKD
AVQKKAVQNSCGCYSVSSVLPGCAERWNSGASFKCTVTHPESDTLTGTIAKITVNTFPP
QVHLLPPPSEELALNELVSLTCLVRAFNPKEVLVRWLHGNEELSPESYLVFEPLKEPGE
GATTYLVTSVLRVSAELWKQGDQYSCMVGHEALPMNFTQKTIDRLSGKPTNVSVSVIMS
EGDGICY
(SNK0002AR antibody light chain amino acid sequence)
>SEQ ID NO: 20: SNK0002AR_L 
MHFQVQIFSFLLISASVIMSRGQIVLTQSPAIMSASPGEKVTITCSASSSVSYMHWFQQ

KPGTSPKLWIYSTSNLASGVPARFSGSGSGTSYSLTISRMEAEDAATYYCQQRSSYPYT
 
FGGGTKLEIKRADAAPTVSIFPPSSEQLTSGGASVVCFLNNFYPKDINVKWKIDGSERQ

NGVLNSWTDQDSKDSTYSMSSTLTLTKDEYERHNSYTCEATHKTSTSPIVKSFNRNEC
>SEQ ID NO: 21: SNK0003_HCDR1
   SYNIH
>SEQ ID NO: 22: SNK0003_HCDR2
   AIYSGNGATSHNQKFK
>SEQ ID NO: 23: SNK0003_HCDR3
   FCTRVGLR
>SEQ ID NO: 24: SNK0003_LCDR1
   KASQSVGTSVA
>SEQ ID NO: 25: SNK0003_LCDR2
   SASYRYS
>SEQ ID NO: 26: SNK0003_LCDR3
   QQYNNYPYT
>SEQ ID NO: 27: SNK0003_HV 
MGFSRIFLFLLSVTTGVHSQAYLQQSGAELVRPGASVRMSCKASDYTFTSYNIHWVKQT

PRQGLEWIGAIYSGNGATSHNQKFKGRATLTVDKSSSTAYMQLSSLTSEDSAVYFCTRV

GLRSPFDFWGQGTTLTVSS
>SEQ ID NO: 28: SNK0003_LV 
MESQTQVFVYMLLWLSGVDGDIVMTQSLKFMSTSGGDRVSVTCKASQSVGTSVAWYQQK

PGQSPKPLIYSASYRYSGVPDRFTGSGSGTDFSLTISNVQSEDLAEYFCQQYNNYPYTF

GGGTKLEIK
(SNK0003A antibody heavy chain amino acid sequence)
>SEQ ID NO: 29:SNK0003A_H 
MGFSRIFLFLLSVTTGVHSQAYLQQSGAELVRPGASVRMSCKASDYTFTSYNIHWVKQT

PRQGLEWIGAIYSGNGATSHNQKFKGRATLTVDKSSSTAYMQLSSLTSEDSAVYFCTRV

GLRSPFDFWGQGTTLTVSSESARNPTIYPLTLPRALSSDPVIIGCLIHDYFPSGTMNVT

WGKSGKDITTVNFPPALASGGGYTMSSQLTLPAVECPEGESVKCSVQHDSNAVQELDVK

CSGPPPPCPPCPPSCHPSLSLQRPALEDLLLGSDASLTCTLNGLRNPEGAVFTWEPSTG

KDAVQKKAVQNSCGCYSVSSVLPGCAERWNSGASFKCTVTHPESDTLTGTIAKITVNTF

PPQVHLLPPPSEELALNELVSLTCLVRAFNPKEVLVRWLHGNEELSPESYLVFEPLKEP

GEGATTYLVTSVLRVSAELWKQGDQYSCMVGHEALPMNFTQKTIDRLSGKPTNVSVSVI

MSEGDGICY
(SNK0003A antibody light chain amino acid sequence)
>SEQ ID NO: 30: SNK0003A_L 
MESQTQVFVYMLLWLSGVDGDIVMTQSLKFMSTSGGDRVSVTCKASQSVGTSVAWYQQK

PGQSPKPLIYSASYRYSGVPDRFTGSGSGTDFSLTISNVQSEDLAEYFCQQYNNYPYTF

GGGTKLEIKRADAAPTVSIFPPSSEQLTSGGASVVCFLNNFYPKDINVKWKIDGSERQN

GVLNSWTDQDSKDSTYSMSSTLTLTKDEYERHNSYTCEATHKTSTSPIVKSFNRNEC
>SEQ ID NO: 31: W27G2_HCDR1
   DYYIH
>SEQ ID NO: 32: W27G2_HCDR2
   RIDPENDETTYAPKFQ
>SEQ ID NO: 33: W27G2_HCDR3
   YCARSTVL
>SEQ ID NO: 34: W27G2_LCDR1
   RASQSIVHTNG
>SEQ ID NO: 35: W27G2_LCDR2
   KLLIYKV
>SEQ ID NO: 36: W27G2_LCDR3
   GVYYCFQGS
>SEQ ID NO: 37:W27G2_HV 
MKCSWIIFFLMAVVTGVNSEVQLQQSGSELVKSGASVKLSCTVSGFNFTDYYIHWVRQR

TEQGLEWIGRIDPENDETTYAPKFQGKATMTADTSSNTAYLQLTSLTSEDTAVYYCARS

TVLDYWGHGTTLTVSS
>SEQ ID NO: 38:W27G2_LV 
MKLPVRLLVLMFWIPGFSSDVLMTQTPLSLPVSLGDQASISCRASQSIVHTNGNTYLEW

YLQKPGQSPKLLIYKVSNRFSGVPDRFSGSGSGTDFILKISRVEAEDLGVYYCFQGSHV

PPTFGGGTKLEVK
(Mouse W27G2 hybridoma-producing antibody heavy chain amino
>SEQ ID NO: 39:W27G2_H 
MKCSWIIFFLMAVVTGVNSEVQLQQSGSELVKSGASVKLSCTVSGFNFTDYYIHWVRQR

TEQGLEWIGRIDPENDETTYAPKFQGKATMTADTSSNTAYLQLTSLTSEDTAVYYCARS

TVLDYWGHGTTLTVSSESARNPTIYPLTLPRALSSDPVIIGCLIHDYFPSGTMNVTWGK

SGKDITTVNFPPALASGGGYTMSSQLTLPAVECPEGESVKCSVQHDSNAVQELDVKCSG

PPPPCPPCPPSCHPSLSLQRPALEDLLLGSDASLTCTLNGLRNPEGAVFTWEPSTGKDA

VQKKAVQNSCGCYSVSSVLPGCAERWNSGASFKCTVTHPESDTLTGTIAKITVNTFPPQ

VHLLPPPSEELALNELVSLTCLVRAFNPKEVLVRWLHGNEELSPESYLVFEPLKEPGEG

ATTYLVTSVLRVSAELWKQGDQYSCMVGHEALPMNFTQKTIDRLSGKPTNVSVSVIMSE

GDGICY
(Mouse W27G2 hybridoma-producing antibody light chain amino
>SEQ ID NO: 40:W27G2_L 
MKLPVRLLVLMFWIPGFSSDVLMTQTPLSLPVSLGDQASISCRASQSIVHTNGNTYLEW

YLQKPGQSPKLLIYKVSNRFSGVPDRFSGSGSGTDFILKISRVEAEDLGVYYCFQGSHV

PPTFGGGTKLEVKRADAAPTVSIFPPSSEQLTSGGASVVCFLNNFYPKDINVKWKIDGS

ERQNGVLNSWTDQDSKDSTYSMSSTLTLTKDEYERHNSYTCEATHKTSTSPIVKSFNRN

EC
>SEQ ID NO: 41:RS_HCDR1_D50N
   NYYIH
>SEQ ID NO: 42:RS_HCDR2_D75N
   RIDPENNETTYAPKFQ
>SEQ ID NO: 43:RS_HCDR2_E76Q
   RIDPENDQTTYAPKFQ
>SEQ ID NO: 44:RS_HCDR2_D75N_E76Q
   RIDPENNQTTYAPKFQ
>SEQ ID NO: 45:RS_HV001 
MKCSWIIFFLMAVVTGVNSEVQLQQSGSELVKSGASVKLSCTVSGFNFTNYYIHWVRQR

TEQGLEWIGRIDPENDETTYAPKFQGKATMTADTSSNTAYLQLTSLTSEDTAVYYCARS

TVLDYWGHGTTLTVSS
>SEQ ID NO: 46:RS_HV002 
MKCSWIIFFLMAVVTGVNSEVQLQQSGSELVKSGASVKLSCTVSGFNFTDYYIHWVRQR

TEQGLEWIGRIDPENNETTYAPKFQGKATMTADTSSNTAYLQLTSLTSEDTAVYYCARS

TVLDYWGHGTTLTVSS
>SEQ ID NO: 47:RS_HV003 
MKCSWIIFFLMAVVTGVNSEVQLQQSGSELVKSGASVKLSCTVSGFNFTDYYIHWVRQR
TEQGLEWIGRIDPENDQTTYAPKFQGKATMTADTSSNTAYLQLTSLTSEDTAVYYCARS
TVLDYWGHGTTLTVSS
>SEQ ID NO: 48:RS_HV004 
MKCSWIIFFLMAVVTGVNSEVQLQQSGSELVKSGASVKLSCTVSGFNFTNYYIHWVRQR
TEQGLEWIGRIDPENNETTYAPKFQGKATMTADTSSNTAYLQLTSLTSEDTAVYYCARS
TVLDYWGHGTTLTVSS
>SEQ ID NO: 49:RS_HV005 
MKCSWIIFFLMAVVTGVNSEVQLQQSGSELVKSGASVKLSCTVSGFNFTNYYIHWVRQR

TEQGLEWIGRIDPENDQTTYAPKFQGKATMTADTSSNTAYLQLTSLTSEDTAVYYCARS

TVLDYWGHGTTLTVSS
>SEQ ID NO: 50:RS_HV006 
MKCSWIIFFLMAVVTGVNSEVQLQQSGSELVKSGASVKLSCTVSGFNFTDYYIHWVRQR

TEQGLEWIGRIDPENNQTTYAPKFQGKATMTADTSSNTAYLQLTSLTSEDTAVYYCARS

TVLDYWGHGTTLTVSS
>SEQ ID NO: 51:RS_HV007 
MKCSWIIFFLMAVVTGVNSEVQLQQSGSELVKSGASVKLSCTVSGFNFTNYYIHWVRQR

TEQGLEWIGRIDPENNQTTYAPKFQGKATMTADTSSNTAYLQLTSLTSEDTAVYYCARS

TVLDYWGHGTTLTVSS
>SEQ ID NO: 52: RS_LCDR1_A44S
   RSSQSIVHTNG
>SEQ ID NO: 53: RS_LFR1_L001
   SPASLSVSLGDRA
>SEQ ID NO: 54: RS_LFR1_L002
   SPASLSVSLGDRV
>SEQ ID NO: 55: RS_LFR1_L003
   SPASMSVSLGDRA
>SEQ ID NO: 56: RS_LFR1_L000
   TPLSLPVSLGDQA
>SEQ ID NO: 57:RS_LV001 
MKLPVRLLVLMFWIPGFSSDVLMTQSPASLSVSLGDRATISCRASQSIVHTNGNTYLEW
YLQKPGQSPKLLIYKVSNRFSGVPDRFSGSGSGTDFILKISRVEAEDLGVYYCFQGSHV
PPTFGGGTKLEVK
>SEQ ID NO: 58:RS_LV002 
MKLPVRLLVLMFWIPGFSSDVLMTQSPASLSVSLGDRVTISCRASQSIVHTNGNTYLEW
YLQKPGQSPKLLIYKVSNRFSGVPDRFSGSGSGTDFILKISRVEAEDLGVYYCFQGSHV
PPTFGGGTKLEVK
>SEQ ID NO: 59:RS_LV003 
MKLPVRLLVLMFWIPGFSSDVLMTQSPASLSVSLGDRVTISCRSSQSIVHTNGNTYLEW

YLQKPGQSPKLLIYKVSNRFSGVPDRFSGSGSGTDFILKISRVEAEDLGVYYCFQGSHV

PPTFGGGTKLEVK
>SEQ ID NO: 60:RS_LV004 
MKLPVRLLVLMFWIPGFSSDVLMTQSPASMSVSLGDRATISCRSSQSIVHTNGNTYLEW

YLQKPGQSPKLLIYKVSNRFSGVPDRFSGSGSGTDFILKISRVEAEDLGVYYCFQGSHV

PPTFGGGTKLEVK
>SEQ ID NO: 61:RS_LV005 
MKLPVRLLVLMFWIPGFSSDVLMTQSPASLSVSLGDRATISCRSSQSIVHTNGNTYLEW

YLQKPGQSPKLLIYKVSNRFSGVPDRFSGSGSGTDFILKISRVEAEDLGVYYCFQGSHV

PPTFGGGTKLEVK
>SEQ ID NO: 62: RS_HCDR1_X
   XYYIH
>SEQ ID NO: 63: RS_HCDR2_X
   RIDPENXXTTYAPKFQ
>SEQ ID NO: 64: RS_LCDR1_X
   RXSQSIVHTNG
(SNK0001AR antibody heavy chain cDNA sequence)
>SEQ ID NO: 65: SNK0001AR_H_CDNA 
ATGGAATGGATCTGGATCTTTCTCTTCATCCTGTCAGGAACTGCAGGTGTCCAATCCCA

GGTTCAGCTGCAGCAGTCTGGAGCTGAGCTGGCGAGGCCTGGGGCTTCAGTGAAGCTGT

CCTGCAAGGCTTCTGGCTACACCTTCACAAGCTATGGTATAAGCTGGGTGAAGCAGAGA

ACTGGACAGGGCCTTGAGTGGATTGGAGAGATTTATCCTAGAAGTGGTAATACTTACTA

CAATGAGAAGTTCAAGGGCAAGGCCACACTGACTGCAGACAAATCCTCCAGCACAGCGT
 
ACATGGAGCTCCGCAGCCTGACATCTGAGGACTCTGCGGTCTATTTCTGTGCAAGATTG
GCATCGAGCTACTACGGTAGTAGCTACGACTGGTACTTCGATGTCTGGGGCACAGGGAC
CACGGTCACCGTCTCCTCAGAGTCTGCGAGAAATCCCACCATCTACCCACTGACACTCC
CACGAGCTCTGTCAAGTGACCCAGTGATAATCGGCTGCCTGATTCACGATTACTTCCCT
TCCGGCACGATGAATGTGACCTGGGGAAAGAGTGGGAAGGATATAACCACCGTAAACTT
CCCACCTGCCCTGGCCTCTGGGGGAGGGTACACCATGAGCAGCCAGTTGACCCTGCCAG
CTGTCGAGTGCCCAGAAGGAGAATCCGTGAAATGTTCCGTGCAACATGACTCTAACGCC
GTCCAAGAATTGGATGTGAAGTGCTCTGGTCCTCCTCCTCCTTGTCCTCCTTGTCCTCC
TTCCTGCCATCCCAGCCTGTCACTGCAGCGGCCAGCTCTTGAGGACCTGCTCCTGGGTT
CAGATGCCAGCCTCACATGTACTCTGAATGGCCTGAGAAATCCTGAGGGAGCTGTCTTC
ACCTGGGAGCCCTCCACTGGGAAGGATGCAGTGCAGAAGAAAGCTGTGCAGAATTCCTG
CGGCTGCTACAGTGTGTCCAGCGTCCTGCCTGGCTGTGCTGAGCGCTGGAACAGTGGCG
CATCATTCAAGTGCACAGTTACCCATCCTGAGTCTGACACCTTAACTGGCACAATTGCC
AAAATCACAGTGAACACCTTCCCACCCCAGGTCCACCTGCTACCGCCGCCGTCGGAGGA
GCTGGCCCTGAATGAGCTCGTGTCCCTGACATGCCTGGTGCGAGCTTTCAACCCTAAAG
AAGTGCTGGTGCGATGGCTGCATGGAAATGAGGAGCTGTCCCCAGAAAGCTACCTAGTG
TTTGAGCCCCTAAAGGAGCCAGGCGAGGGAGCCACCACCTACCTGGTGACAAGCGTGTT
GCGTGTATCAGCTGAACTCTGGAAACAGGGTGACCAGTACTCCTGCATGGTGGGCCACG
AGGCCTTGCCCATGAACTTCACCCAGAAGACCATCGACCGTCTGTCGGGTAAACCCACC
AATGTCAGCGTGTCTGTGATCATGTCAGAGGGAGATGGCATCTGCTACTGA
(SNK0001AR antibody light chain cDNA sequence)
>SEQ ID NO: 66:SNK0001AR_L_CDNA 
ATGAGGACCCCTGCTCAGTTTCTTGGAATCTTGTTGCTCTGGTTTCCAGGTATCAAATG

TGACATCAAGATGACCCAGTCTCCATCTTCCATGTATGCATCTCTAGGAGAGAGAGTCA
 
CTATCACTTGCAAGGCGAGTCAGGACATTAATAGCTATTTAAGCTGGTTCCAGCAGAAA

CCAGGGAAATCTCCTAAGACCCTGATCTATCGTGCAAACAGATTGGTAGATGGGGTCCC

ATCAAGGTTCAGTGGCAGTGGATCTGGGCAAGATTATTCTCTCACCATCAGCAGCCTGG

AGTATGAAGATATGGGAATTTATTATTGTCTACAGTATGATGAGTTTCCGCTCACGTTC

GGTGCTGGGACCAAGCTGGAGCTGAAACGGGCTGATGCTGCACCAACTGTATCCATCTT

CCCACCATCCAGTGAGCAGTTAACATCTGGAGGTGCCTCAGTCGTGTGCTTCTTGAACA

ACTTCTACCCCAAAGACATCAATGTCAAGTGGAAGATTGATGGCAGTGAACGACAAAAT

GGCGTCCTGAACAGTTGGACTGATCAGGACAGCAAAGACAGCACCTACAGCATGAGCAG

CACCCTCACGTTGACCAAGGACGAGTATGAACGACATAACAGCTATACCTGTGAGGCCA

CTCACAAGACATCAACTTCACCCATTGTCAAGAGCTTCAACAGGAATGAGTGTTGA
(SNK0002AR antibody heavy chain cDNA sequence)
>SEQ ID NO: 67: SNK0002AR_H_CDNA 
ATGGGCAGGCTTACTTCTTCATTCCTGTTACTGATTGTCCCTGCATATGTCCTGTCCCA

GGTTACTCTGAAAGAGTCTGGCCCTGGGATATTGCAGCCCTCCCAGACCCTCAGTCTGA

CTTGTTCTTTCTCTGGGTTTTCACTGAGCACTTTTGGTATGGGTGTAGGCTGGATTCGT

CAGCCTTCAGGGAAGGGTCTGGAGTGGCTGGCACACATTTGGTGGGATGATGATAAGTA

CTATAACCCAGCCCTGAAGAGTCGGCTCACAATCTCCAAGGATACCTCCAAAAACCAGG

TATTCCTCAAGATCGCCAATGTGGACACTGCAGATACTGCCACATACTACTGTGCTCGA

ATAGCTGGGTTTGACTACTGGGGCCAAGGCACCACTCTCACAGTCTCCTCAGAGTCTGC

GAGAAATCCCACCATCTACCCACTGACACTCCCACGAGCTCTGTCAAGTGACCCAGTGA

TAATCGGCTGCCTGATTCACGATTACTTCCCTTCCGGCACGATGAATGTGACCTGGGGA

AAGAGTGGGAAGGATATAACCACCGTAAACTTCCCACCTGCCCTGGCCTCTGGGGGAGG

GTACACCATGAGCAGCCAGTTGACCCTGCCAGCTGTCGAGTGCCCAGAAGGAGAATCCG

TGAAATGTTCCGTGCAACATGACTCTAACGCCGTCCAAGAATTGGATGTGAAGTGCTCT
 
GGTCCTCCTCCTCCTTGTCCTCCTTGTCCTCCTTCCTGCCATCCCAGCCTGTCACTGCA
GCGGCCAGCTCTTGAGGACCTGCTCCTGGGTTCAGATGCCAGCCTCACATGTACTCTGA
ATGGCCTGAGAAATCCTGAGGGAGCTGTCTTCACCTGGGAGCCCTCCACTGGGAAGGAT
GCAGTGCAGAAGAAAGCTGTGCAGAATTCCTGCGGCTGCTACAGTGTGTCCAGCGTCCT
GCCTGGCTGTGCTGAGCGCTGGAACAGTGGCGCATCATTCAAGTGCACAGTTACCCATC
CTGAGTCTGACACCTTAACTGGCACAATTGCCAAAATCACAGTGAACACCTTCCCACCC
CAGGTCCACCTGCTACCGCCGCCGTCGGAGGAGCTGGCCCTGAATGAGCTCGTGTCCCT
GACATGCCTGGTGCGAGCTTTCAACCCTAAAGAAGTGCTGGTGCGATGGCTGCATGGAA
ATGAGGAGCTGTCCCCAGAAAGCTACCTAGTGTTTGAGCCCCTAAAGGAGCCAGGCGAG
GGAGCCACCACCTACCTGGTGACAAGCGTGTTGCGTGTATCAGCTGAACTCTGGAAACA
GGGTGACCAGTACTCCTGCATGGTGGGCCACGAGGCCTTGCCCATGAACTTCACCCAGA
AGACCATCGACCGTCTGTCGGGTAAACCCACCAATGTCAGCGTGTCTGTGATCATGTCA
GAGGGAGATGGCATCTGCTACTGA
(SNK0002AR antibody light chain cDNA sequence)
>SEQ ID NO: 68: SNK0002AR_L_CDNA 
ATGCATTTTCAAGTGCAGATTTTCAGCTTCCTGCTAATCAGTGCCTCAGTCATAATGTC

CAGAGGACAAATTGTTCTCACCCAGTCTCCAGCAATCATGTCTGCATCTCCAGGGGAGA

AGGTCACCATAACCTGCAGTGCCAGCTCAAGTGTAAGTTACATGCACTGGTTCCAGCAG

AAGCCAGGCACTTCTCCCAAACTCTGGATTTATAGCACATCCAACCTGGCTTCTGGAGT

CCCTGCTCGCTTCAGTGGCAGTGGATCTGGGACCTCTTACTCTCTCACAATCAGCCGAA

TGGAGGCTGAAGATGCTGCCACTTATTACTGCCAGCAAAGGAGTAGTTACCCGTACACG

TTCGGAGGGGGGACCAAGCTGGAAATAAAACGGGCTGATGCTGCACCAACTGTATCCAT

CTTCCCACCATCCAGTGAGCAGTTAACATCTGGAGGTGCCTCAGTCGTGTGCTTCTTGA

ACAACTTCTACCCCAAAGACATCAATGTCAAGTGGAAGATTGATGGCAGTGAACGACAA
 
AATGGCGTCCTGAACAGTTGGACTGATCAGGACAGCAAAGACAGCACCTACAGCATGAG

CAGCACCCTCACGTTGACCAAGGACGAGTATGAACGACATAACAGCTATACCTGTGAGG

CCACTCACAAGACATCAACTTCACCCATTGTCAAGAGCTTCAACAGGAATGAGTGTTGA
(SNK0003A antibody heavy chain cDNA sequence)
>SEQ ID NO: 69: SNK0003A_H_CDNA 
ATGGGATTCAGCAGGATCTTTCTCTTCCTCCTGTCAGTAACTACAGGTGTCCACTCCCA

GGCTTATCTACAGCAGTCTGGGGCTGAGCTGGTGAGGCCTGGGGCCTCAGTGAGGATGT

CCTGCAAGGCTTCTGACTACACATTTACCAGTTACAATATCCACTGGGTAAAGCAGACA

CCTAGACAGGGCCTGGAATGGATTGGAGCTATTTATTCAGGAAATGGTGCTACTTCCCA

CAATCAGAAGTTCAAGGGCAGGGCCACACTGACTGTAGACAAATCCTCCAGCACAGCCT

ACATGCAACTCAGCAGCCTGACATCTGAAGACTCTGCGGTCTATTTCTGTACAAGAGTG

GGACTCCGGTCCCCTTTTGACTTCTGGGGCCAAGGCACCACTCTCACAGTCTCCTCAGA

GTCTGCGAGAAATCCCACCATCTACCCACTGACACTCCCACGAGCTCTGTCAAGTGACC

CAGTGATAATCGGCTGCCTGATTCACGATTACTTCCCTTCCGGCACGATGAATGTGACC

TGGGGAAAGAGTGGGAAGGATATAACCACCGTAAACTTCCCACCTGCCCTGGCCTCTGG

GGGAGGGTACACCATGAGCAGCCAGTTGACCCTGCCAGCTGTCGAGTGCCCAGAAGGAG

AATCCGTGAAATGTTCCGTGCAACATGACTCTAACGCCGTCCAAGAATTGGATGTGAAG

TGCTCTGGTCCTCCTCCTCCTTGTCCTCCTTGTCCTCCTTCCTGCCATCCCAGCCTGTC

ACTGCAGCGGCCAGCTCTTGAGGACCTGCTCCTGGGTTCAGATGCCAGCCTCACATGTA

CTCTGAATGGCCTGAGAAATCCTGAGGGAGCTGTCTTCACCTGGGAGCCCTCCACTGGG

AAGGATGCAGTGCAGAAGAAAGCTGTGCAGAATTCCTGCGGCTGCTACAGTGTGTCCAG

CGTCCTGCCTGGCTGTGCTGAGCGCTGGAACAGTGGCGCATCATTCAAGTGCACAGTTA

CCCATCCTGAGTCTGACACCTTAACTGGCACAATTGCCAAAATCACAGTGAACACCTTC

CCACCCCAGGTCCACCTGCTACCGCCGCCGTCGGAGGAGCTGGCCCTGAATGAGCTCGT
 
GTCCCTGACATGCCTGGTGCGAGCTTTCAACCCTAAAGAAGTGCTGGTGCGATGGCTGC

ATGGAAATGAGGAGCTGTCCCCAGAAAGCTACCTAGTGTTTGAGCCCCTAAAGGAGCCA

GGCGAGGGAGCCACCACCTACCTGGTGACAAGCGTGTTGCGTGTATCAGCTGAACTCTG

GAAACAGGGTGACCAGTACTCCTGCATGGTGGGCCACGAGGCCTTGCCCATGAACTTCA

CCCAGAAGACCATCGACCGTCTGTCGGGTAAACCCACCAATGTCAGCGTGTCTGTGATC

ATGTCAGAGGGAGATGGCATCTGCTACTGA
(SNK0003A antibody light chain cDNA sequence)
>SEQ ID NO: 70: SNK0003A_L_CDNA 
ATGGAGTCACAGACTCAGGTCTTTGTATACATGTTGCTGTGGTTGTCTGGTGTTGATGG
AGACATTGTGATGACCCAGTCTCTAAAATTCATGTCCACATCGGGAGGAGACAGGGTCA
GCGTCACCTGCAAGGCCAGTCAGAGTGTGGGTACAAGTGTAGCCTGGTATCAGCAGAAA
CCAGGACAATCTCCTAAACCACTGATTTATTCGGCATCTTATCGGTACAGTGGAGTCCC
TGATCGCTTCACAGGCAGTGGATCTGGGACAGATTTCAGTCTCACCATCAGCAATGTGC
AGTCTGAAGACTTGGCAGAATATTTCTGTCAGCAATATAACAACTATCCGTACACGTTC
GGAGGGGGGACCAAGCTGGAGATAAAACGGGCTGATGCTGCACCAACTGTATCCATCTT
CCCACCATCCAGTGAGCAGTTAACATCTGGAGGTGCCTCAGTCGTGTGCTTCTTGAACA
ACTTCTACCCCAAAGACATCAATGTCAAGTGGAAGATTGATGGCAGTGAACGACAAAAT
GGCGTCCTGAACAGTTGGACTGATCAGGACAGCAAAGACAGCACCTACAGCATGAGCAG
CACCCTCACGTTGACCAAGGACGAGTATGAACGACATAACAGCTATACCTGTGAGGCCA
CTCACAAGACATCAACTTCACCCATTGTCAAGAGCTTCAACAGGAATGAGTGTTGA
(Mouse W27G2 hybridoma-producing antibody heavy chain cDNA sequence)
>SEQ ID NO: 71:W27G_MOUSE_IGA_H_CDNA 
ATGAAATGCAGCTGGATCATCTTCTTCCTGATGGCAGTGGTTACAGGGGTCAATTCAGA
GGTTCAGCTGCAGCAGTCTGGGTCAGAGCTTGTGAAGTCTGGGGCCTCAGTCAAGTTGT
CCTGCACAGTTTCTGGGTTCAACTTTACAGACTACTATATACACTGGGTGAGGCAGAGG
ACTGAACAGGGCCTGGAATGGATTGGAAGGATTGATCCTGAGAATGATGAAACTACATA
TGCCCCGAAATTCCAGGGCAAGGCCACTATGACAGCAGACACATCTTCCAACACAGCCT
ACCTGCAGCTCACCAGCCTGACATCTGAAGACACTGCCGTCTATTACTGTGCTAGATCT
ACGGTCCTTGACTACTGGGGCCACGGCACCACTCTCACAGTCTCCTCAGAGTCTGCGAG
AAATCCCACCATCTACCCACTGACACTCCCACGAGCTCTGTCAAGTGACCCAGTGATAA
TCGGCTGCCTGATTCACGATTACTTCCCTTCCGGCACGATGAATGTGACCTGGGGAAAG
AGTGGGAAGGATATAACCACCGTAAACTTCCCACCTGCCCTGGCCTCTGGGGGAGGGTA
CACCATGAGCAGCCAGTTGACCCTGCCAGCTGTCGAGTGCCCAGAAGGAGAATCCGTGA
AATGTTCCGTGCAACATGACTCTAACGCCGTCCAAGAATTGGATGTGAAGTGCTCTGGT
CCTCCTCCTCCTTGTCCTCCTTGTCCTCCTTCCTGCCATCCCAGCCTGTCACTGCAGCG
GCCAGCTCTTGAGGACCTGCTCCTGGGTTCAGATGCCAGCCTCACATGTACTCTGAATG
GCCTGAGAAATCCTGAGGGAGCTGTCTTCACCTGGGAGCCCTCCACTGGGAAGGATGCA
GTGCAGAAGAAAGCTGTGCAGAATTCCTGCGGCTGCTACAGTGTGTCCAGCGTCCTGCC
TGGCTGTGCTGAGCGCTGGAACAGTGGCGCATCATTCAAGTGCACAGTTACCCATCCTG
AGTCTGACACCTTAACTGGCACAATTGCCAAAATCACAGTGAACACCTTCCCACCCCAG
GTCCACCTGCTACCGCCGCCGTCGGAGGAGCTGGCCCTGAATGAGCTCGTGTCCCTGAC
ATGCCTGGTGCGAGCTTTCAACCCTAAAGAAGTGCTGGTGCGATGGCTGCATGGAAATG
AGGAGCTGTCCCCAGAAAGCTACCTAGTGTTTGAGCCCCTAAAGGAGCCAGGCGAGGGA
GCCACCACCTACCTGGTGACAAGCGTGTTGCGTGTATCAGCTGAACTCTGGAAACAGGG
TGACCAGTACTCCTGCATGGTGGGCCACGAGGCCTTGCCCATGAACTTCACCCAGAAGA
CCATCGACCGTCTGTCGGGTAAACCCACCAATGTCAGCGTGTCTGTGATCATGTCAGAG
GGAGATGGCATCTGCTACTGA
(Mouse W27G2 hybridoma-producing antibody light chain cDNA sequence)
>SEQ ID NO: 72:W27G_MOUSE_IGA_L_CDNA 
ATGAAGTTGCCTGTTAGGCTGTTGGTGCTGATGTTCTGGATTCCTGGATTCAGCAGTGA
TGTTTTGATGACCCAAACTCCACTCTCCCTGCCTGTCAGTCTTGGAGATCAAGCCTCCA
TCTCTTGTAGAGCTAGTCAGAGCATTGTACACACTAATGGGAACACCTATTTAGAATGG
TACCTGCAGAAACCAGGCCAGTCTCCAAAGCTCCTGATCTACAAAGTTTCCAACCGATT
TTCTGGGGTCCCAGACAGGTTCAGTGGCAGTGGATCTGGGACAGATTTCATACTCAAGA
TCAGCAGAGTGGAGGCTGAGGATCTGGGAGTTTATTACTGCTTTCAAGGTTCACATGTT
CCTCCGACGTTCGGTGGAGGCACCAAGCTGGAAGTCAAACGGGCTGATGCTGCACCAAC
TGTATCCATCTTCCCACCATCCAGTGAGCAGTTAACATCTGGAGGTGCCTCAGTCGTGT
GCTTCTTGAACAACTTCTACCCCAAAGACATCAATGTCAAGTGGAAGATTGATGGCAGT
GAACGACAAAATGGCGTCCTGAACAGTTGGACTGATCAGGACAGCAAAGACAGCACCTA
CAGCATGAGCAGCACCCTCACGTTGACCAAGGACGAGTATGAACGACATAACAGCTATA
CCTGTGAGGCCACTCACAAGACATCAACTTCACCCATTGTCAAGAGCTTCAACAGGAAT
GAGTGTTAG
(Mouse W27G2 IGG antibody heavy chain cDNA sequence)
>SEQ ID NO: 73:W27G2 _MOUSE_IGG_H_CDNA 
ATGAAATGCAGCTGGATCATCTTCTTCCTGATGGCAGTGGTTACAGGGGTCAATTCAGA

GGTTCAGCTGCAGCAGTCTGGGTCAGAGCTTGTGAAGTCTGGGGCCTCAGTCAAGTTGT

CCTGCACAGTTTCTGGGTTCAACTTTACAGACTACTATATACACTGGGTGAGGCAGAGG

ACTGAACAGGGCCTGGAATGGATTGGAAGGATTGATCCTGAGAATGATGAAACTACATA

TGCCCCGAAATTCCAGGGCAAGGCCACTATGACAGCAGACACATCTTCCAACACAGCCT
 
ACCTGCAGCTCACCAGCCTGACATCTGAAGACACTGCCGTCTATTACTGTGCTAGATCT
ACGGTCCTTGACTACTGGGGCCACGGCACCACTCTCACAGTCTCCTCAAAAACGACACC
CCCATCTGTCTATCCACTGGCCCCTGGATCTGCTGCCCAAACTAACTCCATGGTGACCC
TGGGATGCCTGGTCAAGGGCTATTTCCCTGAGCCAGTGACAGTGACCTGGAACTCTGGA
TCCCTGTCCAGCGGTGTGCACACCTTCCCAGCTGTCCTGCAGTCTGACCTCTACACTCT
GAGCAGCTCAGTGACTGTCCCCTCCAGCACCTGGCCCAGCGAGACCGTCACCTGCAACG
TTGCCCACCCGGCCAGCAGCACCAAGGTGGACAAGAAAATTGTGCCCAGGGATTGTGGT
TGTAAGCCTTGCATATGTACAGTCCCAGAAGTATCATCTGTCTTCATCTTCCCCCCAAA
GCCCAAGGATGTGCTCACCATTACTCTGACTCCTAAGGTCACGTGTGTTGTGGTAGACA
TCAGCAAGGATGATCCCGAGGTCCAGTTCAGCTGGTTTGTAGATGATGTGGAGGTGCAC
ACAGCTCAGACGCAACCCCGGGAGGAGCAGTTCAACAGCACTTTCCGCTCAGTCAGTGA
ACTTCCCATCATGCACCAGGACTGGCTCAATGGCAAGGAGTTCAAATGCAGGGTCAACA
GTGCAGCTTTCCCTGCCCCCATCGAGAAAACCATCTCCAAAACCAAAGGCAGACCGAAG
GCTCCACAGGTGTACACCATTCCACCTCCCAAGGAGCAGATGGCCAAGGATAAAGTCAG
TCTGACCTGCATGATAACAGACTTCTTCCCTGAAGACATTACTGTGGAGTGGCAGTGGA
ATGGGCAGCCAGCGGAGAACTACAAGAACACTCAGCCCATCATGGACACAGATGGCTCT
TACTTCGTCTACAGCAAGCTCAATGTGCAGAAGAGCAACTGGGAGGCAGGAAATACTTT
CACCTGCTCTGTGTTACATGAGGGCCTGCACAACCACCATACTGAGAAGAGCCTCTCCC
ACTCTCCTGGTAAAtaa
(Human chimeric W27G2 IgG antibody heavy chain cDNA sequence)
>SEQ ID NO: 74: W27G2_HUMAN_CHIMERA_IGG_H_CDNA 
ATGAAATGCAGCTGGATCATCTTCTTCCTGATGGCAGTGGTTACAGGGGTCAATTCAGA

GGTTCAGCTGCAGCAGTCTGGGTCAGAGCTTGTGAAGTCTGGGGCCTCAGTCAAGTTGT

CCTGCACAGTTTCTGGGTTCAACTTTACAGACTACTATATACACTGGGTGAGGCAGAGG
 
ACTGAACAGGGCCTGGAATGGATTGGAAGGATTGATCCTGAGAATGATGAAACTACATA
TGCCCCGAAATTCCAGGGCAAGGCCACTATGACAGCAGACACATCTTCCAACACAGCCT
ACCTGCAGCTCACCAGCCTGACATCTGAAGACACTGCCGTCTATTACTGTGCTAGATCT
ACGGTCCTTGACTACTGGGGCCACGGCACCACTCTCACAGTCTCCTCAGCATCCCCGAC
TAGTTCCACCAAGGGCCCATCGGTCTTCCCCCTGGCACCCTCCTCCAAGAGCACCTCTG
GGGGCACAGCGGCCCTGGGCTGCCTGGTCAAGGACTACTTCCCCGAACCGGTGACGGTG
TCGTGGAACTCAGGCGCCCTGACCAGCGGCGTGCACACCTTCCCGGCTGTCCTACAGTC
CTCAGGACTCTACTCCCTCAGCAGCGTGGTGACCGTGCCCTCCAGCAGCTTGGGCACCC
AGACCTACATCTGCAACGTGAATCACAAGCCCAGCAACACCAAGGTGGACAAGAAAGTT
GAGCCCAAATCTTGTGACAAAACTCACACATGCCCACCGTGCCCAGCACCTGAACTCCT
GGGGGGACCGTCAGTCTTCCTCTTCCCCCCAAAACCCAAGGACACCCTCATGATCTCCC
GGACCCCTGAGGTCACATGCGTGGTGGTGGACGTGAGCCACGAAGACCCTGAGGTCAAG
TTCAACTGGTACGTGGACGGCGTGGAGGTGCATAATGCCAAGACAAAGCCGCGGGAGGA
GCAGTACAACAGCACGTACCGTGTGGTCAGCGTCCTCACCGTCCTGCACCAGGACTGGC
TGAATGGCAAGGAGTACAAGTGCAAGGTCTCCAACAAAGCCCTCCCAGCCCCTATCGAG
AAAACCATCTCCAAAGCCAAAGGGCAGCCCCGAGAACCACAGGTGTACACCCTGCCCCC
ATCCCGGGATGAGCTGACCAAGAACCAGGTCAGCCTGACCTGCCTGGTCAAAGGCTTCT
ATCCCAGCGACATCGCCGTGGAGTGGGAGAGCAATGGGCAGCCGGAGAACAACTACAAG
ACCACGCCTCCCGTGCTGGACTCCGACGGCTCCTTCTTCCTCTACAGCAAGCTCACCGT
GGACAAGAGCAGGTGGCAGCAGGGGAACGTCTTCTCATGCTCCGTGATGCATGAGGGTC
TGCACAACCACTACACGCAGAAGAGCCTCTCCCTGTCTCCGGGTAAATGA
(Human chimeric W27G2 IgG antibody light chain cDNA sequence)
>SEQ ID NO: 75: W27G2_HUMAN_CHIMERA_IGG_L_CDNA ATGGACATGAAGTTGCCTGTTAGGCTGTTGGTGCTGATGTTCTGGATTCCTGGATTCAG 
CAGTGATGTTTTGATGACCCAAACTCCACTCTCCCTGCCTGTCAGTCTTGGAGATCAAG
CCTCCATCTCTTGTAGAGCTAGTCAGAGCATTGTACACACTAATGGGAACACCTATTTA
GAATGGTACCTGCAGAAACCAGGCCAGTCTCCAAAGCTCCTGATCTACAAAGTTTCCAA
CCGATTTTCTGGGGTCCCAGACAGGTTCAGTGGCAGTGGATCTGGGACAGATTTCATAC
TCAAGATCAGCAGAGTGGAGGCTGAGGATCTGGGAGTTTATTACTGCTTTCAAGGTTCA
CATGTTCCTCCGACGTTCGGTGGAGGCACCAAGCTGGAAGTCAAACGGGCTGATGCTGC
ACCAACTGTATCCATCTTCCCACCATCCAGTGAGCAGTTAACATCTGGAGGTGCCTCTG
TTGTGTGCCTGCTGAATAACTTCTATCCTCGAGAGGCCAAAGTACAGTGGAAGGTGGAT
AACGCCCTCCAATCGGGTAACTCCCAGGAGAGTGTCACAGAGCAGGACAGCAAGGACAG
CACCTACAGCCTCAGCAGCACCCTGACGCTGAGCAAAGCAGACTACGAGAAACACAAAG
TCTACGCCTGCGAAGTCACCCATCAGGGCCTGAGCTCGCCCGTCACAAAGAGCTTCAAC
AGGGGAGAGTGTTAG
(Mouse W27G2 IGG antibody heavy chain amino acid sequence)
>SEQ ID NO: 76: W27G2_MOUSE_IGG_H 
MKCSWIIFFLMAVVTGVNSEVQLQQSGSELVKSGASVKLSCTVSGFNFTDYYIHWVRQR
TEQGLEWIGRIDPENDETTYAPKFQGKATMTADTSSNTAYLQLTSLTSEDTAVYYCARS
TVLDYWGHGTTLTVSSKTTPPSVYPLAPGSAAQTNSMVTLGCLVKGYFPEPVTVTWNSG
SLSSGVHTFPAVLQSDLYTLSSSVTVPSSTWPSETVTCNVAHPASSTKVDKKIVPRDCG
CKPCICTVPEVSSVFIFPPKPKDVLTITLTPKVTCVVVDISKDDPEVQFSWFVDDVEVH
TAQTQPREEQFNSTFRSVSELPIMHQDWLNGKEFKCRVNSAAFPAPIEKTISKTKGRPK
APQVYTIPPPKEQMAKDKVSLTCMITDFFPEDITVEWQWNGQPAENYKNTQPIMDTDGS
YFVYSKLNVQKSNWEAGNTFTCSVLHEGLHNHHTEKSLSHSPGK
(Human chimeric W27G2 IgG antibody heavy chain amino acid sequence)
>SEQ ID NO: 77:W27G2_HUMAN_CHIMERA_IGG_H 
MKCSWIIFFLMAVVTGVNSEVQLQQSGSELVKSGASVKLSCTVSGFNFTDYYIHWVRQR

TEQGLEWIGRIDPENDETTYAPKFQGKATMTADTSSNTAYLQLTSLTSEDTAVYYCARS

TVLDYWGHGTTLTVSSASPTSSTKGPSVFPLAPSSKSTSGGTAALGCLVKDYFPEPVTV

SWNSGALTSGVHTFPAVLQSSGLYSLSSVVTVPSSSLGTQTYICNVNHKPSNTKVDKKV

EPKSCDKTHTCPPCPAPELLGGPSVFLFPPKPKDTLMISRTPEVTCVVVDVSHEDPEVK

FNWYVDGVEVHNAKTKPREEQYNSTYRVVSVLTVLHQDWLNGKEYKCKVSNKALPAPIE

KTISKAKGQPREPQVYTLPPSRDELTKNQVSLTCLVKGFYPSDIAVEWESNGQPENNYK

TTPPVLDSDGSFFLYSKLTVDKSRWQQGNVFSCSVMHEGLHNHYTQKSLSLSPGK
(Human chimeric W27G2 IgG antibody light chain amino acid sequence)
>SEQ ID NO: 78:W27G2_HUMAN_CHIMERA_IGG_L

MDMKLPVRLLVLMFWIPGFSSDVLMTQTPLSLPVSLGDQASISCRASQSIVHTNGNTYL

EWYLQKPGQSPKLLIYKVSNRFSGVPDRFSGSGSGTDFILKISRVEAEDLGVYYCFQGS

HVPPTFGGGTKLEVKRADAAPTVSIFPPSSEQLTSGGASVVCLLNNFYPREAKVQWKVD

NALQSGNSQESVTEQDSKDSTYSLSSTLTLSKADYEKHKVYACEVTHQGLSSPVTKSFN

RGEC

### 3. Pharmaceutical Composition

In one aspect, the composition of the present disclosure is provided as a pharmaceutical composition. The pharmaceutical composition of the present disclosure is typically used to treat various impairments and/or diseases attributed to inflammation in the liver.

In the present disclosure, the term "treatment" has a general meaning in the art, and means that the composition of the present disclosure is administered to a subject for the purpose of ameliorating, eradicating, or curing any impairment or disease state, for the purpose of preventing or delaying (preventing) the occurrence of these impairments or disease states, or for the purpose of preventing or reducing the possibility of recurrence of these impairments or disease states (recurrence inhibition).

In one aspect, the pharmaceutical composition of the antibody drug complex of the present disclosure is a pharmaceutical composition for preventing or treating inflammation in liver tissue. In one aspect, the pharmaceutical composition of the present disclosure is a pharmaceutical composition for preventing or treating inflammation in liver tissue in a cholestasis liver impairment. In one aspect, the pharmaceutical composition of the present disclosure is used to prevent or treat various impairments or diseases attributed to inflammation of liver tissue due to causes including the cholestasis liver impairment. Non-limiting examples of impairments or diseases to be treated by the pharmaceutical composition of the present disclosure include chronic hepatitis, liver fibrosis, liver cirrhosis, liver inflammation following liver transplantation, autoimmune hepatitis, and intrahepatic lithiasis.

The pharmaceutical composition of the present disclosure may contain an effective amount of the enteric bacteria-binding monoclonal antibody or the antigen-binding fragment thereof according to the present disclosure. The pharmaceutical composition can be appropriately set, for example, so that a content ratio of the antibody according to the present invention in 100 wt% of the pharmaceutical composition is in a range of 0.001 to 99.99 wt%, in consideration of the type of a target disease, a dosage form, an administration method, a target to be administered, a degree of symptoms of an administration subject, a degree of effect exerted by administration, and the like.

In the present disclosure, the term "effective amount" refers to an amount in which the enteric bacteria-binding monoclonal antibody or the antigen-binding fragment thereof according to the present disclosure can inhibit inflammation of liver tissue in a living body, or an amount that exerts a therapeutic effect on an impairment or disease associated with liver inflammation.

The pharmaceutical composition according to the present disclosure may contain a pharmaceutically acceptable carrier or additive together with the enteric bacteria-binding monoclonal antibody or the antigen-binding fragment thereof. The pharmaceutically acceptable carrier or additive means any carrier, diluent, excipient, suspending agent, lubricant, adjuvant, medium, delivery system, emulsifier, disintegrant, absorbent, preservative, surfactant, colorant, flavoring, or sweetener, and a known pharmaceutically acceptable carrier or additive may be employed.

Examples of the subject to be administered include, but are not limited to, mammals such as a human, a mouse, a rat, a guinea pig, a rabbit, a hamster, a dog, a cat, a weasel, a cow, and a pig.

A dosage and administration method of the pharmaceutical composition of the present disclosure are appropriately selected depending on the type, gender, species, age, general condition, severity of the disease, degree of desired effect, and the like of the disease that affects the individual to be administered. Usually, the dosage may be appropriately set in a range of 0.001 to 100 mg/kg/day.

In the administration of the pharmaceutical composition according to the present disclosure, the above amount may be administered once a day, or may be administered in several divided doses. In addition, an administration interval may be every day, every other day, every week, every other week, every 2 or 3 weeks, every month, every other month, or every 2 or 3 months as long as the therapeutic effect on the disease is obtained.

The administration method of the pharmaceutical composition of the present disclosure is not particularly limited, but it is preferable to directly administer to the digestive tract, and as such an administration method, oral administration, nasal administration, transmucosal administration, enteral administration, and the like can be used.

Here, the enteral administration is not limited to administration via the anus, and includes, for example, administration via a tube or the like inserted into the digestive tract from the outside of the individual like a gastrostomy or the like. A position where the digestive tract is inserted is not limited to the intestine, and examples thereof include esophagus, stomach, small intestine (including duodenum, jejunum, ileum, and the like), and large intestine (including cecum, colon, rectum, and the like). In addition, for example, the composition of the present disclosure can be blended with a known component and used as an intestinal washing liquid.

In one aspect, the pharmaceutical composition of the present disclosure can contain a plurality of enteric bacteria-binding monoclonal antibodies of the present disclosure in combination as long as their functions are not impaired. In addition, the pharmaceutical composition of the present disclosure can be used in combination with other therapeutic agents, for example, other antiinflammatory agents or therapeutic agents for liver fibrosis as long as its function is not impaired.

When the pharmaceutical compositions of the present disclosure are used in combination or in combination with other therapeutic agents, these agents may be contained in a single preparation or may be contained in another preparation. In a case where the pharmaceutical composition is provided by another preparation, the pharmaceutical composition may be provided in the form of a kit containing a combination of the preparations, and each pharmaceutical composition may be provided as a single agent for combination with another preparation. These preparations may be administered simultaneously or sequentially.

### 4. Food Composition

In one aspect, the composition of the present disclosure is provided as a food composition. The food composition is a composition in which the oral or enteral composition of the present disclosure is preferably used exclusively in the field of food.

The form of the food composition of the present disclosure is not particularly limited, and for example, the food composition can be provided as a food or drink such as a supplement, a solid food, a fluid food, or a beverage. The food composition of the present disclosure can be provided as a food for special use such as a food for specified health uses, a food with functionality, or a nutrient functional food. The food composition of the present disclosure can be provided as a health functional food, a food for a patient, a dairy product, fermented milk, a prepared milk powder, a lactic beverage, an acidic beverage, a yogurt, cheese, bread, a biscuit, a cracker, a pizza crust, a prepared milk powder, a liquid food, a food for a patient, a nutritional food, a frozen food, a food composition, a processed food, other commercially available foods, and the like.

The food composition of the present disclosure includes, in addition to a general food, a food for specified health uses including a food for conditional specified health uses, a nutritional supplementary food, a functional food, and a food for a patient. In one aspect, the food composition of the present disclosure can be provided as a food composition labeled, for example, for improving liver function, preventing deterioration of liver function, or the like.

Specific examples of the form of the food composition of the present disclosure include, but are not limited to, drinks such as soft drinks, carbonated drinks, nutritional drinks, fruit drinks, lactic acid drinks, and milk drinks; frozen desserts such as ice cream, ice sorbet, and shaved ice; confectioneries such as candies, candies, gum, chocolate, tablet candies, snacks, biscuits, jelly, jam, cream, and baked confectioneries; noodles such as buckwheat noodles, thick noodles made from wheat flour, starch noodles, Chinese-style noodles, and instant noodles; fish or livestock processed foods such as fish cakes, ham, and sausage; dairy products such as processed milk products and fermented milk products; oil and fat and oil and fat processed foods such as salad oil, tempura oil, margarine, mayonnaise, shortening, whipped cream, and dressing; seasonings such as sauces and tare sauce; and soups, stews, salads, side dishes, furikake, pickles, bread, and cereal. In addition, the food for specified health uses, the nutritional supplementary food, the functional food, and the like can be provided as the form of powders, granules, capsules, troches, tablets, syrups, and the like.

In one aspect, the composition of the present disclosure is provided as a food material. The shape of the food material of the present disclosure is not particularly limited, and is appropriately set according to the type of food to be used, such as a liquid, paste, powder, or solid. The food material of the present disclosure may be further formed into a desired shape by a treatment such as crushing, pulverization, and particle sizing as necessary.

The food composition of the present disclosure may contain an effective amount of the enteric bacteria-binding monoclonal antibody or the antigen-binding fragment thereof according to the present disclosure. For example, a content ratio of the antibody according to the present invention in 100 wt% of the composition can be appropriately set so as to be in a range of 0.001 to 99.99 wt% in consideration of a degree of the effect to be exhibited and the like.

### 5. Feed Composition

In one aspect, the composition of the present disclosure is provided as a feed composition. The feed composition is a composition for which the feed composition of the present disclosure is exclusively used in the field of feed.

The specific form of the feed composition described above is not particularly limited, and for example, as long as the effect exhibited by the feed composition of the present disclosure described above is not impaired, a feed composition may be prepared by mixing a food feed with a normal feed, and as necessary, by mixing a component that can be blended with a normal feed, or a food feed composition itself may be used as feed.

The sample composition of the present disclosure may contain an effective amount of the enteric bacteria-binding monoclonal antibody or the antigen-binding fragment thereof according to the present disclosure. For example, a content ratio of the antibody according to the present invention in 100 wt% of the composition can be appropriately set so as to be in a range of 0.001 to 99.99 wt% in consideration of a degree of the effect to be exhibited and the like.

### 6. Therapeutic Method

In one aspect, the present disclosure includes a therapeutic method using the antibody, the pharmaceutical composition, or the food composition of the present disclosure described above. The therapeutic method in the present disclosure includes administering the antibody or composition described in "Monoclonal Antibody That Binds to Enteric Bacteria", "Pharmaceutical Composition", "Food Composition", or "Feed Composition" to a subject. Specific preventive or therapeutic target impairments or diseases, subjects to be administered, administration methods, dosages, and the like may also be those described in "Pharmaceutical Composition", "Food Composition", or "Feed Composition".

### Examples

Hereinafter, the present disclosure will be described in more detail with reference to Example, but these Examples are merely illustrative and do not limit the present disclosure.

### Example 1-1: Inhibition of Liver Inflammation by Enteric Bacteria-Binding Monoclonal Antibody

### (Materials and Methods)

### 1. Mouse

C57BL/6 were bred in SPF conditions, and 13-week-old male C57BL/6 mice were used.

### 2. Common Bile Duct Ligation

Common bile duct ligation was performed as a cholestasis liver impairment model. A body weight of a mouse was measured before surgery, the mouse was placed in the supine position under general anesthesia by isoflurane inhalation, the abdomen was opened by median incision, and the common bile duct was doubly ligated with 8-0 silk. It was confirmed that there was no bile leakage, and the abdomen was sutured and closed in two layers of peritoneum and abdominal wall. The surgery was performed on a heat pad (set at 37°C) in order to prevent hypothermia during the surgery, and 500 µL of a 5% glucose solution was administered intraperitoneally immediately before closing the abdomen in order to prevent postoperative dehydration.

### 3. Administration of Test Antibodies

As a test antibody, a W27IgA antibody was adjusted to 10 pg/100 µl with physiological saline, and administered once using an oral sonde (22 G) before anesthesia for common bile duct ligation. 100 µl of physiological saline was administered to a control group. After the common bile duct ligation, 10 pg/100 µl of the W27IgA antibody and 100 µl of physiological saline were administered to the control group every three days. After the common bile duct ligation, the W27IgA administration group and the control group were kept in different cages. For the purpose of evaluating liver impairments and liver fibrosis due to the common bile duct ligation, mice subjected to only laparotomy under general anesthesia were defined as a normal liver group.

### 4. Sample Collection

Mice were placed in sterile plastic cases prior to anesthesia for the common bile duct ligation, and feces immediately after defecation were collected and immediately frozen with liquid nitrogen. The general condition was observed 14 days after the common bile duct ligation, the body weight was measured, the mice were sacrificed under general anesthesia, venous blood was collected from the inferior vena cava, the liver was extracted, and feces in the rectum were collected. The jejunum (about 3 cm from the Treitz) and large intestine were collected. For blood collection, plasma was centrifuged using EDTA-2K as an anticoagulant. The liver, jejunum, and rectal tissues were immersed and fixed in 10% neutral buffered formalin for 48 hours, formed into paraffin blocks, and then subjected to histopathological analysis. A part of the tissue of each organ was cut into 3 mm square pieces for gene expression analysis, and immediately frozen with liquid nitrogen. The feces collected from the rectum were also immediately frozen with liquid nitrogen.

### 5. Biochemical Analysis

ALT and T-Bil of plasma were measured using a dry clinical chemical analyzer Spot Chem EZ SP-4430 (manufactured by ARKRAY, Inc.).

### 6. Histopathologic Analysis

Tissue section slides having a thickness of 4 um were prepared from the paraffin blocks of the liver tissue, and HE staining and Sirius Red staining were performed.

### 6-1. H&E Staining

### Hematoxylin staining solution: Mayer's hematoxylin (MUTO PURE CHEMICALS CO., LTD. #30002)

Eosin staining solution: 1% eosin alcohol (MUTO PURE CHEMICALS CO., LTD. #32022) was diluted 4 times with 95% ethanol and used. H&E staining was performed in the following procedure.

### Order

| | | |
|---|---|---|
| 1 | Deparaffinization | Xylene 3 tanks, 5 minutes each |
| 2 | Dexylenization | 100% ethanol 2 tanks, 5 minutes each |
| 3 | Immersion | 95% ethanol 2 tanks 70% ethanol 1 tank, 5 minutes each |
| 4 | Flowing water washing | Tap water, 5 minutes |
| 5 | Hematoxylin staining | Hematoxylin staining solution, 6 minutes |
| 6 | Flowing water washing (coloring) | Tap water, 1 minute |
| 7 | Weak alkalization | 0.1% aqueous sodium bicarbonate solution (bluing agent), 1 minute |
| 8 | Flowing water washing | Tap water, 1 minute |
| 9 | Dehydration | 80% ethanol, 1 minute |
| 10 | Eosin staining | Eosin staining solution, 4 minutes |
| 11 | Fractionation | 95% ethanol 2 tanks, quickly |
| 12 | Dehydration | 100% ethanol 2 tanks, 5 minutes each |
| 13 | Clearing | Xylene 2 tanks, 5 minutes each |
| 14 | Sealing | |

### 6-2. Sirius Red Staining

Sirius Red staining solution: 0.1% (wt/vol) Direct Red 80/saturated picric acid
0.5% Glacial acetic acid water: 0.5% (vol/vol) glacial acetic acid
Sirius Red staining was performed in the following procedure.

### Order

| | | |
|---|---|---|
| 1 | Deparaffinization | Xylene 3 tanks, 5 minutes each |
| 2 | Dexylenization | 100% ethanol 2 tanks, 5 minutes each |
| 3 | Immersion | |
| 4 | Flowing water washing | Tap water, 5 minutes |
| 5 | Sirius Red staining | Sirius Red staining solution, 20 minutes |
| 6 | Washing | 0.5% glacial acetic acid water, 4 tanks, quickly |
| 7 | Washing | 100% ethanol, quickly |
| 8 | Dehydration | 100% ethanol |
| 9 | Clearing | Xylene |
| 10 | Sealing | |

### 6-3. Quantitative Evaluation of Fibrosis

Five images were randomly captured at 40-fold per slide and ImageJ (National Institutes of Health) was used to calculate the area% of red areas (fibers) per tissue area.

### 7. Gene Expression Analysis

### 7-1. Extraction of total RNA from Liver Tissue

The liver tissue of 3 mm square (25 to 50 mg) was homogenized using 500 µL of TRIZOL reagent. In order to separate proteins, incubation was performed at room temperature for 5 minutes, 100 µL of chloroform was added, the solution was vigorously shaken and mixed in a tube for 15 seconds, and then the mixed solution was allowed to stand at room temperature for 3 minutes and centrifuged at 4°C and 12,000 g for 15 minutes. It was confirmed that the solution was separated into a dark red phenol-chloroform phase, an intermediate phase, and a colorless upper aqueous phase, and the aqueous phase in which RNA was dissolved was transferred to a new tube. (The volume of the aqueous phase was about 60% (= 300 µL) of the TRIZOLR reagent used) 250 µL of isopropyl alcohol was added, the solution was vigorously shaken and mixed in the tube for 15 seconds, and then the mixed solution was allowed to stand at room temperature for 10 minutes and centrifuged at 4°C and 12,000 g for 10 minutes. The RNA of the white pellet was confirmed at the bottom of the tube, the supernatant was removed, 1,000 µL of 75% ethanol was added, and the RNA pellet was vortexed and centrifuged at 4°C and 7,600 g, for 5 minutes. The supernatant was removed, the RNA pellet was dried (air-dried for 5 to 10 minutes), 100 µL of RNase-free water was added, and then mixing and dissolution were performed by pipetting.

### 7-2. Cleanup of total RNA (RNeasy mini Kit, manufactured QIAGEN)

350 µl of buffer RLT was added to and mixed with 100 µL of RNA solution (< 1 µg/uL), and 250 µL of 100% ethanol was added thereto and mixed well. 700 µL of the solution was applied to an RNA spin column set in a 2 ml collection tube. RNA was cleaned up according to the procedure.

### 7-3. RNA-seq Analysis

The RNA sample was confirmed to have an absorbance ratio OD260/OD280 ≥ 1.8 and was further measured for RNA Integrity Number (RIN) by Bioanalyzer (manufactured by Agilent Technologies, Inc.) to confirm that the absorbance ratio was 7 or more. Poly(A) RNA was extracted, fragmented, and converted into cDNA from 100 ng of total RNA using NEBNext Poly(A) mRNA Magnetic Isolation Module (manufactured by BioLabs) and NEBNext Ultra II RNA Library Prep Kit for Illumina (manufactured by BioLabs), and an adaptor was added using NEBNext Adaptor (manufactured by BioLabs). A barcode sequence for identifying a sample was added to the prepared cDNA using NEBNext Multiplex Oligos for Illumina (manufactured by BioLabs). A concentration and purity of the prepared library were confirmed by Qubit (manufactured by Thermo Fisher Scientific Inc.) and Bioanalyzer (manufactured by Agilent Technologies, Inc.), respectively. The cDNA region 75 bp and the barcode sequence were subjected to fragment analysis using Illumina NextSeq. CLC Genomics Workbench 12.0.3 (hereinafter, referred to as GWB) was used for data analysis, the output fastq file was imported into GWB, and then the reads were trimmed with GWB Trim reads 2.3 tool. The parameters were set by default except that the 3' end was deleted by 1 bp.

### 8. Intestinal Flora Analysis

Feces in the rectum obtained from mice sacrificed before the common bile duct ligation and 14 days after the common bile duct ligation were subjected to bacterial flora analysis. The analysis was performed by Bioengineering Lab. Co., Ltd. (Japan).

### 8-1. DNA Extraction

DNA was extracted from fecal samples using Fast DNA spin kit (MP Bio).

### 8-2 Quantitative Measurement of DNA Solution

The concentration of the extracted DNADNA solution was measured using Qubit and dsDNA HS Assay Kit (Thermo Fisher Scientific)Fisher Scientific)Fisher Scientific).

### 8-3 Preparation of Library

A library was prepared using a 2-step tailed PCR method.

### 8-4. Quantification of Library

A concentration measurement of the prepared library was performed using Qubit and dsDNA HS Assay Kit.

### 8-5. Quality Confirmation of Library

Quality confirmation of the prepared library was performed using Fragment Analyzer and dsDNA 915 Reagent Kit (Advanced Analytical Technologies).

### 8-6. Sequencing Analysis

Sequencing was performed using MiSeq under a condition of 2 × 300 bp.

### 8-7. Quality Filtering of Reads

Using fastq_barcode_spliltter of Fastx toolkit, only a sequence whose sequence reading start completely matched the used primer was extracted. The primer sequence of the extracted sequence was deleted. Thereafter, a sequence having a quality value of less than 20 was removed using sickle tools, and a sequence having a length of 100 bases or less and a pair sequence thereof were discarded.

### 8-8. Merging of Reads

Paired end merge script FLASH was used to merge the sequences through quality filtering. The merging conditions were a merged fragment length of 260 bases, a read fragment length of 230 bases, and a minimum overlap length of 10 bases.

### 8-9. Chimera Check

All filtered sequences were checked for chimeric sequences using the uchime algorithm of usearch. The database was 97% OTU of Greengene attached to the bacterial flora analysis pipeline Qiime, and all sequences that were not determined to be chimeras were extracted and used for subsequent analysis.

### 8-10. OTU Creation and System Estimation

The OTU creation and the system estimation were performed using the workflow script of Qiime under the default conditions of no reference and parameters.

### 8-11. ANCOM

Analysis of composition of microbiomes (ANCOM) was performed according to the reference (Mandal et al., Microb Ecol Health Dis. 2015 May 29; 26:27663. doi:10.3402/mehd.v26.27663).

### (Results)

### 1 Serum Biochemical Analysis

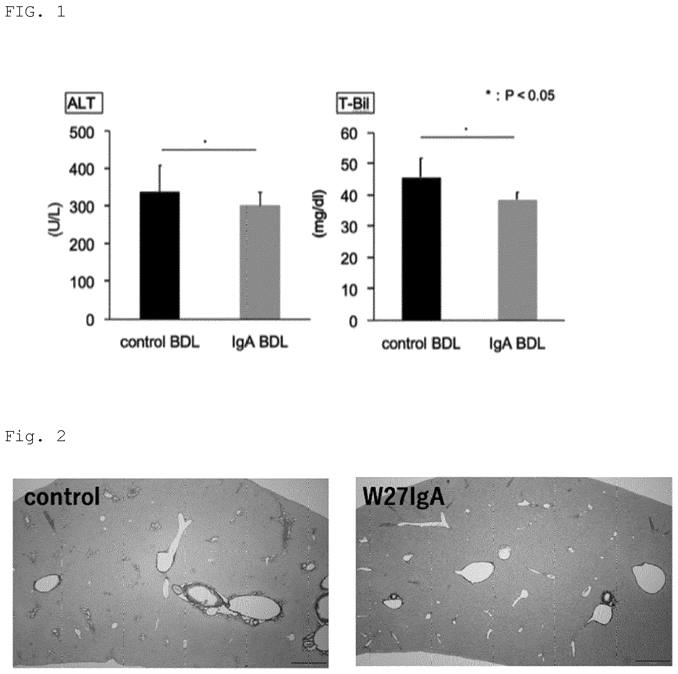

ALT as an indicator of a liver cell impairment and T-Bil as an indicator of jaundice were measured in venous plasma obtained from mice sacrificed 14 days after the common bile duct ligation (Fig. 1). In the W27IgA administration group, both ALT and T-Bil were significantly reduced as compared with the control group.

### 2 Pathological Analysis

### 2-1 Sirius Red Staining

Collagen of a liver tissue section obtained from a mouse sacrificed 14 days after common bile duct ligation was stained with Sirius Red. After W27IgA administration, a decrease in fibrosis in the portal vein area as compared with that in the BDL group was observed (Fig. 2).

### 2-2 Area Ratio of Sirius Red Staining Area

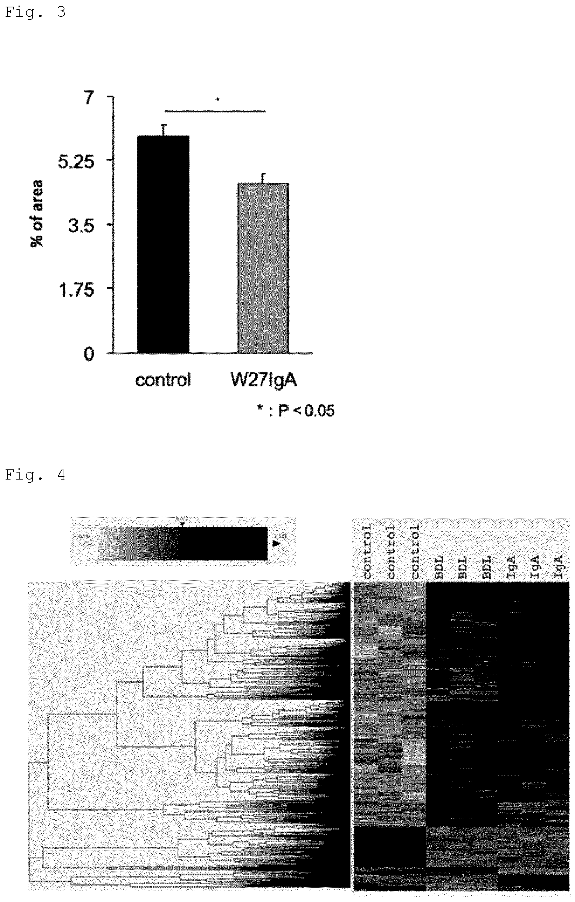

An area ratio of the stained portions on the image of the Sirius Red staining of 2-1 was calculated by ImageJ. As a result, it was found that the area after IgA administration was significantly reduced as compared with BDL (Fig. 3).

### 3 Gene Expression Analysis

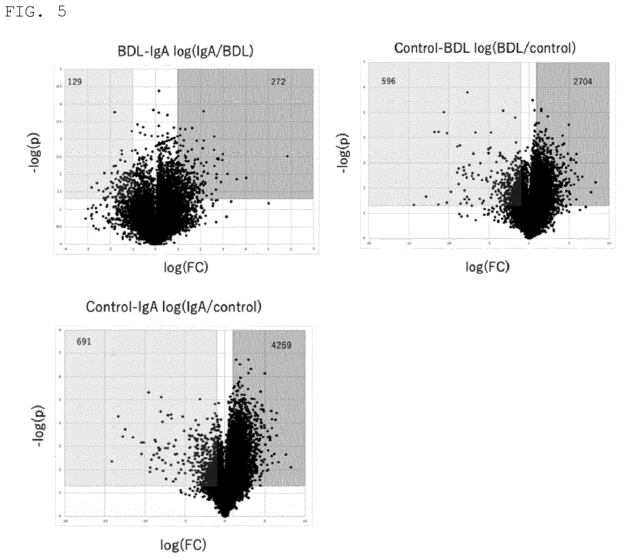

A heat map was created by extracting differentially expressed genes in three groups of the control group, the BDL group, and the IgA group (Fig. 4). Differentially expressed genes between each two groups of the control group-BDL group, the BDL group-IgA group, and the control group-IgA group were extracted (Fig. 5). The gene group in which the expression was significantly increased was represented in dark gray, and the gene group in which the expression was significantly decreased was represented in light gray. The number shown on the graph is the number of differentially expressed genes identified.

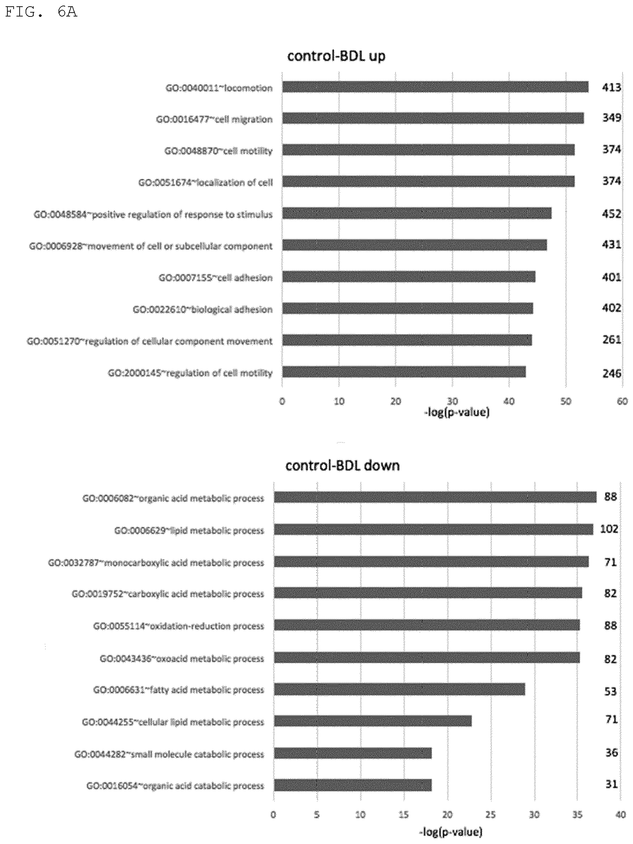

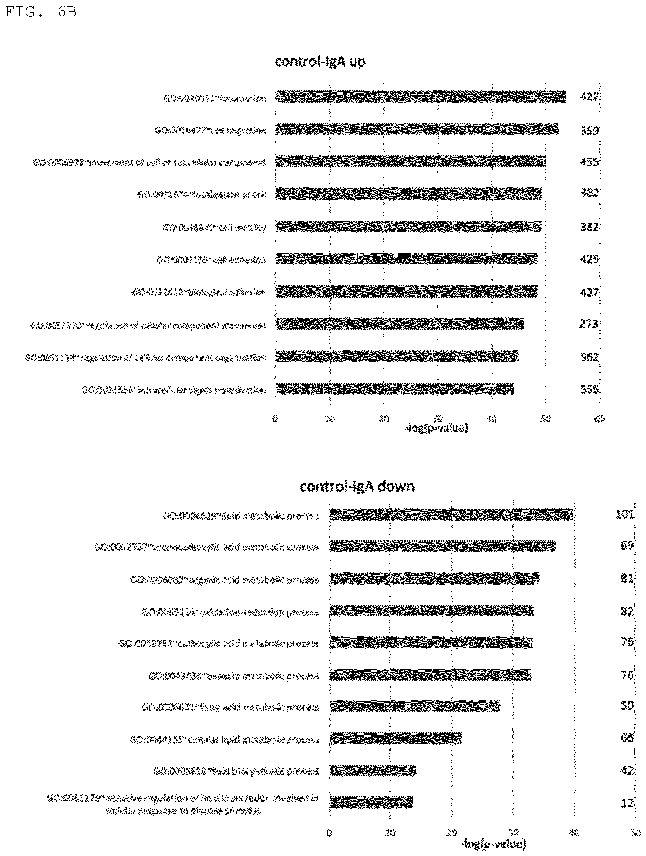

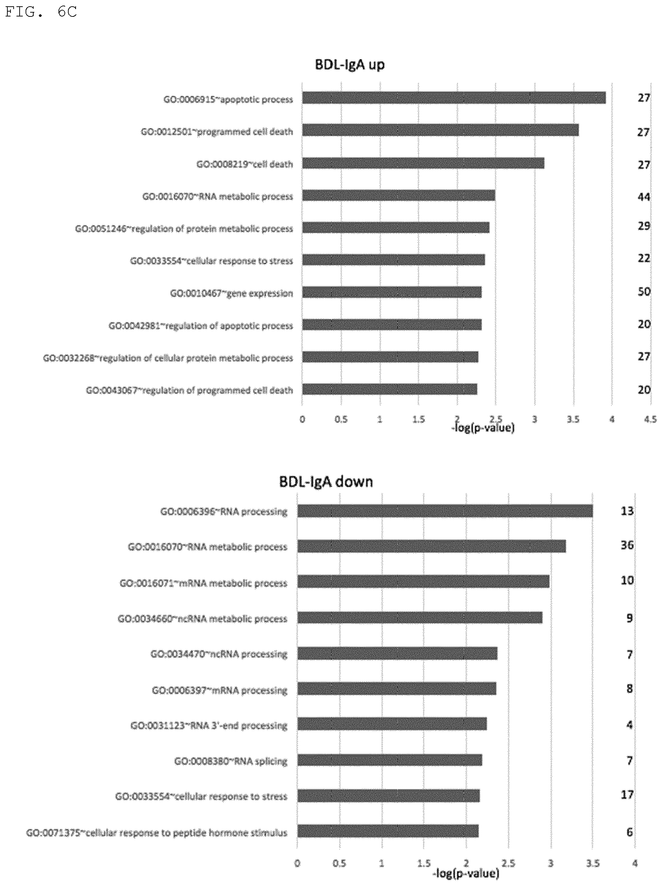

As a result of the pathway analysis, a pathway of cell death was observed in the gene group in which the expression was increased in the BDL-IgA group (Figs. 6A to 6C). Each number next to the bar graph is the number of genes indicating the pathway.

### 4 Intestinal Flora Analysis

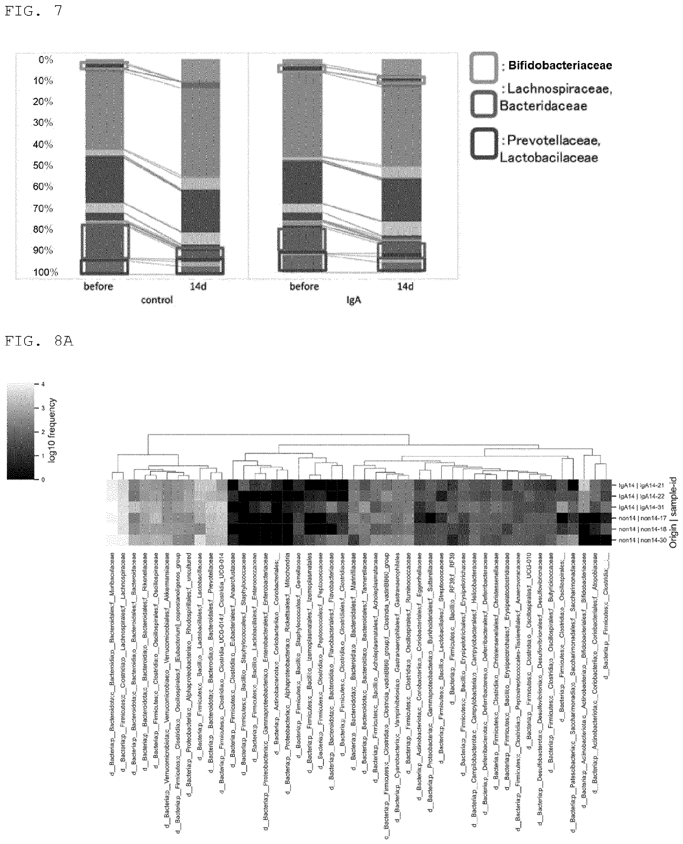

In the control group, the number of bacteria belonging to Bifidobacteriaceae significantly decreased 14 days after BDL, whereas in the W27IgA administration group, the number of bacteria belonging to Bifidobacteriaceae was maintained (Fig. 7). Group comparison (multivariate analysis) by ANCOM was performed to find specific bacteria that were significantly different in abundance between the two groups. Also, in the heat map of enteric bacteria (Fig. 8A) obtained by visualizing the ANCOM results, it was confirmed that the occupancy of bifidobacteria was large.

### 5 Considerations

In the W27IgA administration group, the values of ALT and T-Bil were lower than those in the control group, and the area of the number of cells undergoing fibrosis was smaller, such that liver impairments and liver fibrosis were reduced. Further refluxing cholestasis together with W27IgA was considered to be effective for complete recovery of liver impairments.

As a result of extracting the differentially expressed genes, not a few genes whose expression varied from the BDL group to the IgA administration group were observed. From this, it is found that changes at the gene level contribute to the reduction of cholestatic liver impairments by IgA administration. In addition, it was revealed from the results of the pathway analysis that genes whose expression significantly increased from the BDL group to the IgA group were associated with cell death.

In addition, since the expression level of TNF-α receptor is decreased, it is considered that liver impairments are reduced due to inhibition of cell death of hepatocytes or inhibition of activation of kupffer cells. Hepatocytes are usually resistant to cell death by TNF-α due to inhibition of JNK pathway by NF-κB. In addition, it has been reported that cell death in TNF-α is induced in hepatocytes with knockdown of NF-κB (Reference 8). Although it is not intended to limit the present disclosure, since the data of this study shows a tendency that the expression level of NF-κB is increased, it is considered that when hepatocytes in the BDL state do not have resistance to cell death by TNF-α, the hepatocytes acquired resistance to TNF-α by IgA administration, and the hepatocytes did not induce cell death and contributed to the reduction of liver impairments.

In addition, it has been reported that LPS binds to a Toll-like receptor (Toll-like Receptor 4: TLR4) and TNF-α is produced via MyD88 downstream thereof in kupffer cells (Reference 9). In the data of this study, since the gene expression level of TLR4 is decreased and the expression level of MyD88 downstream thereof is also decreased, it is considered that the production amount of TNF-α via TLR4 is decreased. Although it is not intended to limit the present disclosure, it is conceivable that liver impairments may be reduced because activation of kupffer cells is inhibited.

Example 1-2: Effect of Improving Intestinal Flora of Enteric Bacteria-Binding Monoclonal Antibody in Common Bile Duct Ligation (BDL) Untreated Subject

According to the materials and methods of Example 1-1, the effect of an enteric bacteria-binding monoclonal antibody on wild-type C57BL/6 mice without common bile duct ligation (BDL) was verified. Specifically, to four C57BL/6 mice, 10 pg/100 µl of the W27IgA antibody was orally administered every three days in the same manner as in Example 1-1.

Feces before (pre 1 to pre 4) and after (post 1 to post 4) administration of the W27IgA antibody were collected and immediately frozen with liquid nitrogen. When comparison was performed between before and after administration by the ANCOM, it was confirmed that the occupancy of bifidobacteria was increased by administration of W27IgA.

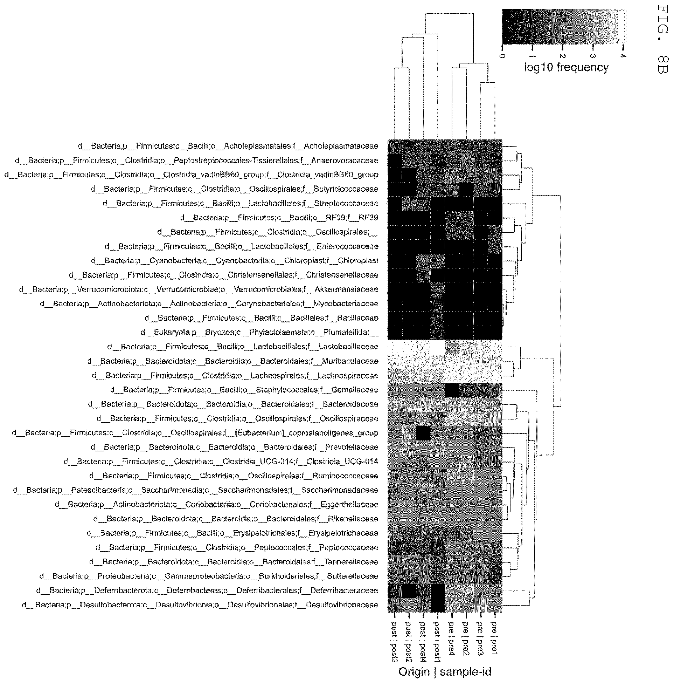

From the present results, it was found that even in mice in which BDL was not performed, in the W27IgA administration group, there was a tendency that the number of deferribacteraceae of aerobic gram-negative bacilli was significantly decreased and the number of lactobacillaceae was increased (Fig. 8B).

### References

[1] Aldin MM et al. Liver fibrosis. Truk J Gastroenterol 2018; 29: 14-21.2018.
[2] Derrick E. Fouts et al. Bacterial translocation and changes in the intestinal microbiome in mouse models of liver disease. Journal of Hepatology 2012; 56: 1283-1292.
[3] Inamine T et al. Immunoglobulin A and liver diseases. J Gastroenterol. 53(6); 691-700, 2018.
[4] Isabel Gomez-Hurtado, Arlette Santacruz, Gloria Peiro, Pedro Zapater, Ana Gutierrez, Miguel Perez-Mateo, Yolanda Sanz, Ruben Frances. Gut Microbiota Dysbiosis Is Associated with Inflammation and Bacterial Translocation in Mice with CCl4-Induced Fibrosis. Plos One. 2011;6(7):e23037.
[5] Mark J. Pallen, Mohammed N. Quraishi. The Gut Microbiota and the Hepatologist: Will Our Bugs Prove to be the Missing Link? Dig dis 2017;35(4):377-383.
[6] Okai S et al. High-affinity monoclonal IgA regulates gut microbiota and prevents colitis in mice. Nat Microbiol. 1(9); 16103, 2016.
[7] Wataru Fujibuchi, Katsuhisa Horimoto, Microarray data statistical analysis protocols-Everything from data standardization to significant difference analysis, clustering, and network analysis methods, mainly using Excel-Yodosha Co., Ltd.
[8] Yang X, Bialik S, Jones BE, Iimuro Y, Kitsis RN, Srinivasan A, et al. NF-κB inactivation converts a hepatocyte cell line TNF-α response from proliferation to apoptosis. Am J Physiol 1998; 275: C1058-66.
[9] Misako Matsumoto, Tsukasa Seya Functions of Toll-like receptors Biochemistry Vol. 81, No. 3, pp. 156-164, 2009

### Example 2: Identification of C. Difficile Antigen Molecules of W27 Antibodies

It was confirmed that the W27 antibody bound to serine hydroxymethyl transferase (SHMT) of *E. coli* (WO 2014/142084 A and the like), and it was confirmed that the W27 antibody bound to *E. coli* SHMT and a C. *difficile* antigen molecule of the W27 antibody was identified by the following method. The W27G2 antibody has the same heavy chain variable region and light chain variable region as the W27 antibody.

### (Materials and Methods)

*E. coli* was statically cultured in 25 ml of LB medium for 16 hours. Bacteria were collected by centrifugation at 2,150 g for 5 minutes. After removing the supernatant, the medium was suspended in 1 ml of a lysis buffer (sterile 1 × PBS, 10% NP-40, ×100 Protease Inhibitor Cocktail (Nacalai)), ultrasonically crushed, and then allowed to stand on ice for 30 minutes. After dispensing 200 µl of the bacterial lysate into a new tube, 50 µl of 2-ME and 200 µl of 10% SDS were added and denatured (95°C, 10 min). To dilute 2-ME and SDS, 800 µl of a diluent (sterile 1 × PBS, 0.1% NP-40, ×100 Protease Inhibitor Cocktail) was added. In order to separate the protein solution into soluble and insoluble fractions, centrifugation was performed at 15,000 rpm for 5 minutes. The soluble fractions were used to perform 2D-PAGE to identify target protein spots.

For C. *difficile,* a bacterial lysate was prepared in the same manner, and 2D-PAGE was performed using the soluble fractions.

The method of 2D-PAGE is described below. First, in order to prepare a sample of 2D-PAGE, cooled acetone was added in an amount of 2 times the amount of the immunoprecipitation product, the mixture was allowed to stand at -20°C for 20 minutes, the supernatant was removed by centrifugation (16,630 g, 10 min), and then the mixture was dried. The sample was dissolved in a sample buffer for the one-dimensional electrophoresis (60 mM Tris-HCl (pH 8.8), 5 M Urea, 1 M Thiourea, 5 mM EDTA, 1% CHAPS, 1% NP-40), 1/10 of 1 M iodoacetamide was added thereto, and the mixture was allowed to stand at room temperature for 10 minutes, thereby preparing a one-dimensional sample for 2D-PAGE. For the two-dimensional electrophoresis, two SDS-PAGE gels were used for performing Western blot and silver stain. After the one-dimensional electrophoresis using Immobiline^{™} DryStrip (GE Healthcare) with a pH of 3 to 8, the two-dimensional SDS-PAGE was performed, and then Western blot and silver stain were performed on each gel. In order to easily identify the spot recognized by the W27 antibody, the membrane after blotting was stained with Pierce^{™} Reversible Protein Stain, and the positions of all proteins on the membrane were confirmed. This stain was erased with a stain eraser, and then the W27 antibody was reacted to confirm a spot by Western blot. Silver stain was performed using Sil-Best Stain One (Nacalai) and following the protocol. By comparing the results of these three types (Western blot, Pierce^{™} Reversible Protein Stain, and silver stain by W27), a spot specifically recognized by the W27 antibody was cut out and subjected to in-gel enzyme digestion.

A method of in-gel enzyme digestion is described below. 400 µl of MilliQ water was added to the cut gel piece, the mixture was shaken for 10 minutes, and then the supernatant was removed. This operation was repeated twice, 200 µl of 100% acetonitrile was then added, and the mixture was shaken for 10 minutes. After removing the supernatant, drying under reduced pressure was performed for 20 minutes, 20 µl of a protease solution (50 mM ammonium hydrogen carbonate, 10 µl/ml Trypsin) was added, and the gel piece was swollen on ice for 30 minutes to absorb trypsin. The surplus protease solution was removed, 100 µl of a reaction solution (50 mM ammonium hydrogen carbonate) was added, and enzyme digestion was performed at 37°C overnight. 50 µl of the extract (5% formic acid, 50% acetonitrile) was further added to the reaction solution, and the mixture was shaken for 30 minutes. 200 µl of 0.1% formic acid was added to the recovered solution, and drying under reduced pressure was performed until the sample amount reached about half. The sample was transferred to a spin filter (UltraFree 0.1 µm PVDF) and centrifuged (2,460 g, 5 min, 4°C). The solution dropped on the lower layer was transferred to a vial and analyzed with LCMS-IT-TOF (Shimadzu). Mascot search was used to identify the amino acid sequence.

### (Results)

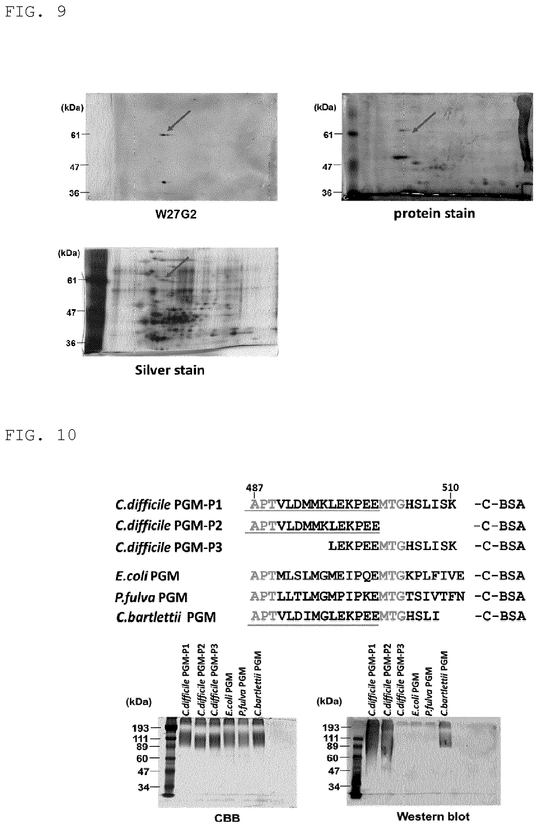

*E. coli* SHMT and 2,3-bisphosphoglycerate-independent phosphoglycerate mutase (iPGM) of *C*. *difficile* were identified as antigen molecules of the W27 antibody by the above method. As an example, two-dimensional electrophoresis data when an antigen molecule of C. *difficile* is identified is illustrated in Fig. 9.

### Example 3: Determination of Epitope of Antigen Molecule of W27 Antibody

It was identified that the W27 antibody was an epitope at the N-terminal 25 to 30 amino acids of *E. coli* SHMT (published paper). An attempt was made to prepare a truncation protein also for *C*. *difficile* iPGM, but the efficiency of protein expression was significantly reduced, and thus, the reaction of the W27 antibody was not confirmed by Western blot analysis.

Therefore, the fact that the W27 antibody did not recognize *E. coli* iPGM was confirmed by Western blot, and a plasmid expressing a chimeric protein of C. *difficile* iPGM and *E. coli* iPGM recognized by the W27 antibody was prepared using In-FusionR HD Cloning Kit (TaKaRa). Primers were created from the iPGM gene sequence information of each of bacteria on the database (table). The chimeric protein was overexpressed with DH5α, and the recognition of the W27 antibody was confirmed by Western blot to narrow down the site of the epitope sequence.

**Table 2: Primer base sequences used for In-Fusion cloning**

| ***C. difficile* PGM** | |
|---|---|
| | Base sequence (5'-3') |
| *C.dif* F primer | GTG GTG GAA TTC ACC ATG ATG AAA AAA CCA GTT GCT |
| *C.dif* R primer | GAA GGG CCC TCT AGA TTT TGA AAT AAG TGA ATG ACC |
| *C.dif* 50F primer | GAT GTT GGT CTA CCT GAT GGT CAA ATG GGA AAT TCA |
| *C.dif* 50R primer | AAG ACC ACT AGC ATT TAT CAA TGT GTT TGG ATA CTC |
| *C.dif* 320F primer | AAA ACT CAA CTT AGA GCT GCT GAA ACT GAA AAG TAT |
| *C.dif* 320R primer | TCC ATT TTT AGC TAG ATA CTC ACC TAG AGT GTT GGC |
| *C.dif* 390F primer | GTA TTA AAC TTT GCA AAT CCT GAT ATG GTA GGT CAT |
| *C.dif* 390R primer | TAT AAA GTC AAA CTT ATC TTC TCC TAG TTT ATC TAA |
| *C.dif* 411F primer | ATG GAA GCG GCG GTT AAG GCT GTT GAA ACT GTA GAT |
| *C.dif* 443F primer | GCT GAC CAC GGT AAC GCT GAA TAT ATG TTA GAC CCA |
| *C.dif* 464F primer | ACC AAC CTG CCA GTT CCA TTT ATT GTA GTT GGT CAA |
| *C.dif* 487F primer | AAA CTT TCT GAC ATC GCA CCG ACT GTT TTA GAT ATG |
| *E.coli* F primer | GTG GTG GAA TTC ACC ATG TTG GTT TCT AAA AAA CCT |
| *E.coli* R primer | GAA GGG CCC TCT AGA TTC CAC GAT GAA CAG CGG CTT |
| *E.coli* 52F primer | AAT GCT AGT GGT CTT GAA GTC GGT CTG CCT GAC CGT |
| *E.coli* 52R primer | CAG ACC GGA AGC GTC GAT CAG AGG TAG ACC AAC ATC |
| *E.coli* 325F primer | CTA GCT AAA AAT GGA AAA ACT CAG TTG CGT ATT TCC |
| *E.coli* 325R primer | GTC GTT TTT CGC CAT CCA CTC TCT AAG TTG AGT TTT |
| *E.coli* 395F primer | AAG TTT GAC TTT ATA ATC TGT AAC TAT CCG AAC GGC |
| *E.coli* 395R primer | AAT GGT GTC GTA TTT GCC GCT TGC AAA GTT TAA TAC |

As a result of the analysis, in C. *difficile* iPGM, the amino acids at positions 490 to 510 at the C-terminus were important as epitopes. Peptides containing each epitope candidate (BSA-conjugate) were outsourced to SIGMA to further confirm epitopes. Since the molecular weight of the peptide alone was small, BSA was conjugated to each peptide as confirmed by Western blot.

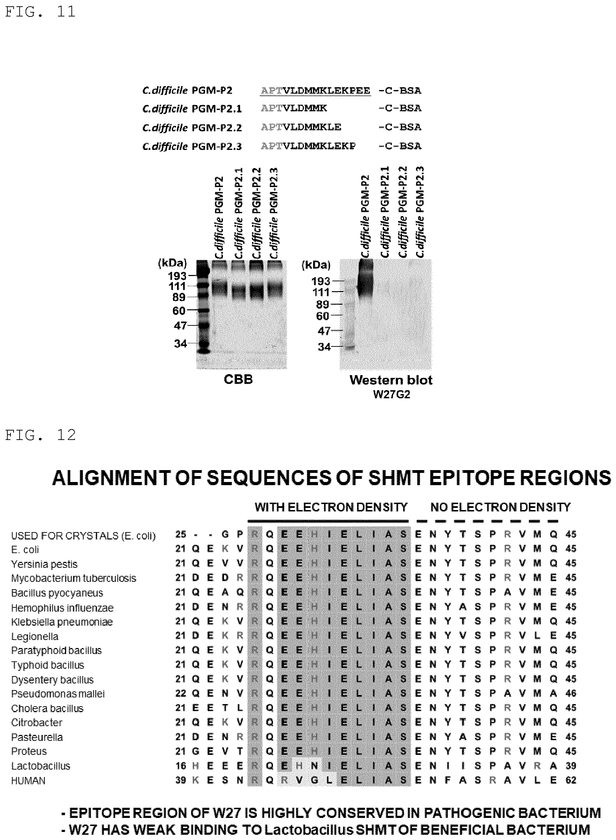

4 × SDS buffer was added to the peptide, and Western blot with the W27 antibody was performed to confirm which synthetic peptide was reacted. The results are illustrated in Figs. 10 and 11.

As a result of the above test, the epitope sequence of *E. coli* SHMT was found to be RQEEHIELIASEN, and the epitope sequence of C. *difficile* iPGM was found to be APTVLDMMKLEKPEEMTGHSLISK.

As a result of database analysis of bacteria sharing an epitope of *E. coli* SHMT recognized by the W27 antibody, bacteria having an EEHI sequence were mostly bacteria belonging to Proteabacteria, and contained many pathogenic bacteria (Fig. 12). The amino acid sequences at the same portion of *lactobacillus* and human SHMT were different, and it was considered that the W27 antibody recognized this difference. (Okai et al., Nature microbiology 1,16103, 2016)

### Example 4: Crystal Structure Analysis of W27 Antibody and Antigen Molecule

### (4-1) Preparation and Purification of IgG W27 Fab

To examine the antigen recognition of the W27 antibody, a W27 recombinant IgG antibody expressed by CHO (Chinese hamster ovary) cells was prepared. This is because it is necessary to secure a large amount of antigen binding sites (Fab) for crystal structure analysis. The crude purified IgG W27 was adjusted to 1 mg/ml with 10 mM Tris-HCl (pH 8.0), and then digested with 0.05 mg/ml papain (Nacalai Tesque) at 20°C for 24 hours to digest the Fc region and the Fab region. Thereafter, purification was performed by cation exchange column chromatography using HiTrap SP HP column (Cytiva). As the buffer, 10 mM Bis-Tris buffer (pH 6.0) (buffer A), 10 mM Bis-Tris buffer (pH 6.0), and 300 mM NaCl (buffer B) were used, and Fab and Fc were separated and purified by linear gradient elution in which a concentration of the buffer B was gradually increased. Thereafter, the Fab rich fraction was further purified by gel filtration chromatography using a HiLoad 26/600 Superdex 2000 pg gel filtration column (Cytiva). As a buffer for gel filtration, 5 mM BisTris (pH 6.5) and 100 mM NaCl (gel filtration buffer) were used. After purification by gel filtration chromatography, impurities of a trace amount of Fc were adsorbed and removed by a column filled with rProtein A Sepharose Fast Flow resin (Cytiva), and Fab present in the flow-through fraction was recovered. The purified Fab was concentrated to 48.2 mg/ml with Amicon Ultra 10,000 MWCO (Millipore) under the conditions of a gel filtration buffer, dispensed into an Eppendorf tube by 20 ul, instantaneously frozen with liquid nitrogen, and then stored at -80°C until use. The yield of Fab was 17.4 mg from 50 mg of IgG W27.

### (4-2) Expression and Purification of E. Coli SHMT (25-45) (For Crystal Structure Analysis)

The cDNA region encoding *E*. *coli*-derived SHMT (25-45) was amplified using PrimeSTAR Max DNA Polymerase (TaKaRa), and then the plasmid pGEX6P-3 (Cytiva) was incorporated into the Sma I and NotI multiple cloning sites of the modified plasmid pGEXM (Kim SY, et al. (2021) Sci Rep 11 (1):2120) using In-Fusion HD Cloning Kit (Clontech) to construct an expression plasmid. The prepared expression plasmid was transformed into *E. coli* Rosetta2 strain (Merck). Note that SHMT (25-45) was expressed as a Gultathione-S-transferase (GST) fusion protein (hereinafter, referred to as GST-SHMT (25-45)). In addition, since a cleavage sequence LEVLFQGP by human rhinovirus 3C protease (hereinafter, HRV3C protease) (the cleavage site is between Q and G) is inserted between GST and the target protein, the expressed GST fusion protein can separate GST and the target protein from each other by HRV3C protease. The amino acid sequence of the final purified *E. coli* SHMT (25-45) is GPRQEEHIELIASENYTSPRVMQ.

Culture of *E. coli* was started at 37°C using LB medium containing 50 ug/ml of ampicillin, 25 ug/ml of chloramphenicol, 0.5% (W/V) of glycerin, 0.05% (W/V) of glucose, 0.2% (W/V) of lactose, and 1 mM magnesium sulfate. When the turbidity of the medium reached 0.6 (measured at a wavelength of 600 nm), the medium was cooled, and then 1 M isopropyl-β-D-thio-galactopyranoside (hereinafter, IPTG) was added so that the final concentration was 0.1 mM to induce expression of a target protein. After addition of IPTG, culture was further performed at 18°C for 24 hours. The bacterial bodies in the culture solution were collected by centrifugation at 4,000 rpm (Beckman J2-M1 JA10 rotor) at 4°C for 20 minutes. The collected bacterial bodies were stored at -30°C until used for purification.

Purification of SHMT was performed as follows. The bacterial bodies were suspended in 10 mM Tris-HCl (pH 8.0) and 300 mM NaCl, ultrasonically disrupted at 4°C, and then centrifuged at 4°C and 20,000 rpm for 40 minutes. Then, the insoluble fraction was removed, and the soluble fraction of the supernatant was used for the next purification. Purification of GST-SHMT (25-45) from the soluble fraction was performed using an affinity column using Glutathione Sepharose 4B resin (hereinafter, GS4B) (Cytiva). The soluble fraction after disruption was applied to an affinity column filled with GS4B to adsorb GST-SHMT (25-45), and then GS4B was sufficiently washed with 10 mM Tris-HCl (pH 8.0), 300 mM NaCl (hereinafter, washing buffer). Thereafter, GST-SHMT (25-45) was eluted from the resin with 10 mM Tris-HCl (pH 8.0), 300 mM NaCl, and 20 mM Glutathione. The eluate was digested using HRV 3C protease (Merck) at 4°C for 18 hours to cleave into GST and SHMT (25-45), and GST was removed. In SHMT (25-45), an extra GP sequence including a part of the recognition sequence of HRV3C protease was added at the N-terminal. SHMT (25-45) after GST cleavage was subjected to gel filtration purification using HiLoad 26/600 Superdex 75 pg gel filtration column (Cytiva) under the conditions of 5 mM BisTris (pH 6.5) and 100 mM NaCl. The purified SHMT (25-45) was concentrated to 4.72 mg/ml with a centrifugal device with 1 K available from PALL Corporation under the conditions of 5 mM BisTris (pH 6.5) and 100 mM NaCl (gel filtration buffer), dispensed into an Eppendorf tube by 20 ul, instantaneously frozen with liquid nitrogen, and then stored at -80°C until use. As for the yield, 5.9 mg of SHMT (25-45) used for crystallization was obtained from 2 L of the cultured bacterial bodies.

### (4-3) Expression and Purification of C. Difficile iPGM (486-509) (For Crystal Structure Analysis)

Cloning of cDNA of *Clostridium difficile-derived* iPGM (486-509) (*C*. *difficile* iPGM (486-509)) into a pGEX vector was performed by almost the same method as SHMT. At this time, since tyrosine or tryptophan was not present in *C. difficile* iPGM (486-509) and absorption at a wavelength of 280 nm was not observed in the purification process, a tyrosine 1 residue was added at the N-terminal and cloning was performed. In iPGM (486-509), an extra GPY sequence including a part of the recognition sequence of HRV3C protease was added at the N-terminal. The amino acid sequence of the final purified *C*. *difficile* iPGM (486-509) is GPYAPTVLDMMKLEKPEEMTG HSLISK. The procedures related to transformation, culture, and purification were performed in the same manner as in SHMT (25-45), and 48.52 mg of iPGM (486-509) used for crystallization was obtained from 4 L of the cultured bacterial bodies.

### (4-4) Crystallization Screening of RS_H000_L000GR Fab-E. Coli SHMT (25-45) Complex

A mixed solution was prepared using the purified RS_H000_L000GR Fab and *E. coli* SHMT (25-45) at a molar ratio of 1:5 (0.2 mM:1.0 mM), and crystallization screening was performed.

Screening of crystallization conditions was performed using JCSG core suite I-IV, PACT suite (Qiagen), which is a commercially available crystallization screening kit, and a crystallization robot mosquito (TTP Labtech). A crystallization plate (VIOLAMO) was filled with a precipitating agent for equilibration, droplets (drops) obtained by mixing the precipitating agent and the protein solution at a volume ratio of 1:1 (0.2 µl:0.2 ul) were prepared, and the crystallization conditions were searched by a sitting drop vapor diffusion method. As the protein solution used in the crystallization screening, a solution obtained by removing an insoluble fraction by centrifugation in advance was used. The incubation was performed under the temperature conditions were 4°C and 20°C.

### (4-5) Optimization of Crystallization Conditions of RS_H000_L000GR Fab-E. Coli SHMT (25-45) Complex

A precipitating agent whose pH and PEG concentration were changed based on the conditions of the crystals obtained by the crystallization screening was prepared, and the precipitation was performed by a sitting drop vapor diffusion method.

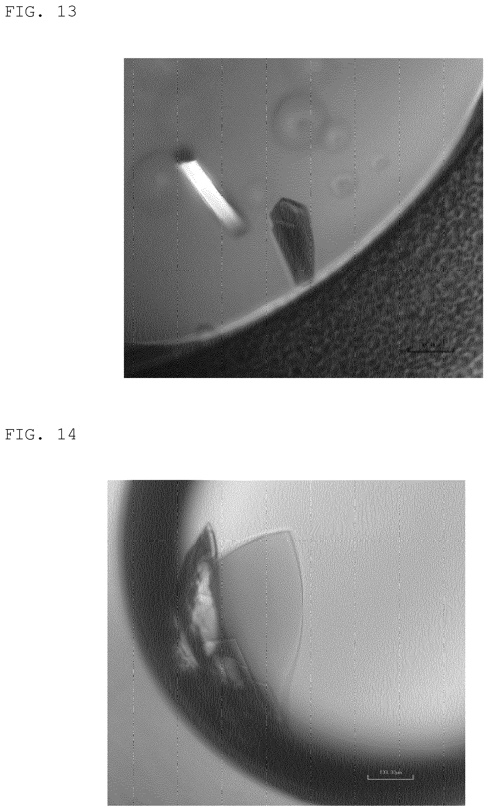

Crystallization conditions were 0.2 M Magnesium Acetate, 0.1 M Sodium Cacodylate, pH 6.3, and 22% PEG 10000 as a reservoir solution. The protein solution obtained by mixing 0.2 mM RS_H000_L000GR Fab and 0.6 mM *E. coli* SHMT (25-45) and the reservoir were mixed at 0.2 µl:0.2 ul, the temperature condition was 20°C, and crystallization by the sitting drop vapor diffusion method was observed after 7 days. The scale bar indicates 100 um (Fig. 13).

### (4-6) X-Ray Diffraction Experiment and Three-Dimensional Structure Determination of RS_H000_L000GR Fab-E. Coli SHMT (25-45) Complex

The RS_H000_L000GR Fab-E. *coli* SHMT (25-45) complex crystals obtained in the optimization of the crystallization conditions described above were frozen in liquid nitrogen using 0.2 M Magnesium Acetate, 0.1 M Sodium Cacodylate pH 6.3, 22.0% PEG 10000, 20.0% Glycerol as an anti-freezing agent (cryoprotectant). Thereafter, measurement was performed at BL44XU of a large synchrotron radiation facility SPring-8 in Hyogo-ken. The obtained diffraction data was processed by an XDS program, and then a phase was determined by a molecular substitution method using structural information of RS_H000_L000GR Fab. The space group was P2₁2₁2₁, the lattice constants were a = 60.4 A, b = 140.7 A, c = 180.7 A, α = 90°, β = 90°, and γ = 90°, and three molecules of RS_H000_L000GR Fab-E. *coli* SHMT (25-45) were present in the asymmetric unit. The structure refinement was performed using the programs refmac 5, phenix refine, and coot in combination, and R_{work}/R_{free} finished the refinement at 22.8% and 25.7%, respectively. The structural diagram was created using the program PyMOL.

### (4-7) Optimization of Crystallization Conditions of RS_H000_L000GR Fab-C. difficile iPGM (486-509) Complex

Crystallization of IgG W27 Fab C. *difficile* iPGM (486-509) was performed by a sitting drop vapor diffusion method using the precipitating agent prepared by changing the pH and the concentration of PEG 10,000 based on the structurally analyzed IgG W27 Fab-E. *coli* SHMT (25-45) crystallization conditions (0.2 M Magnesium Acetate, 0.1 M Sodium Cacodylate pH 6.3, 22% PEG 10000).

Crystallization conditions were 0.2 M Magnesium Acetate, 0.1 M Sodium Cacodylate, pH 6.5, and 23% PEG 10000 as a reservoir solution. The protein solution obtained by mixing 0.25 mM RS_H000_L000GR Fab and 1.0 mM C. *difficile* iPGM (486-509) and the reservoir were mixed at a mixing ratio of 0.2 µl:0.2 ul, the temperature condition was 20°C, the scale bar was 100 µm, and crystallization by the sitting drop vapor diffusion method was observed after 10 days (Fig. 12).

### (4-8) X-Ray Diffraction Experiment and Three-Dimensional Structure Determination of RS_H000_L000GR Fab-C. difficile iPGM (486-509) Complex

The RS_H000_L000GR Fab-C. *difficile* iPGM (486-509) complex crystals obtained in the optimization of the crystallization conditions described above were frozen in liquid nitrogen using 0.2 M Magnesium Acetate, 0.1 M Sodium Cacodylate pH 6.5, 23.0% PEG 10000, 20.0% Glycerol as an anti-freezing agent (cryoprotectant). Thereafter, measurement was performed at BL41XU of a large synchrotron radiation facility SPring-8 in Hyogo-ken. The obtained diffraction data was processed by an XDS program, and then a phase was determined by a molecular substitution method using structural information of IgG W27 Fab. The space group was P2₁2₁2₁, the lattice constants were a = 60.423 A, b = 140.185 A, c = 185.956 A, α = 90°, β = 90°, and γ = 90°, and three molecules of RS_H000_L000GR Fab-C. *difficile* iPGM (486-509) were present in the asymmetric unit. The structure refinement was performed using the programs refmac 5, phenix refine, and coot in combination, and R_{work}/R_{free} finished the refinement at 22.2% and 25.2%, respectively. The structural diagram was created using the program PyMOL.

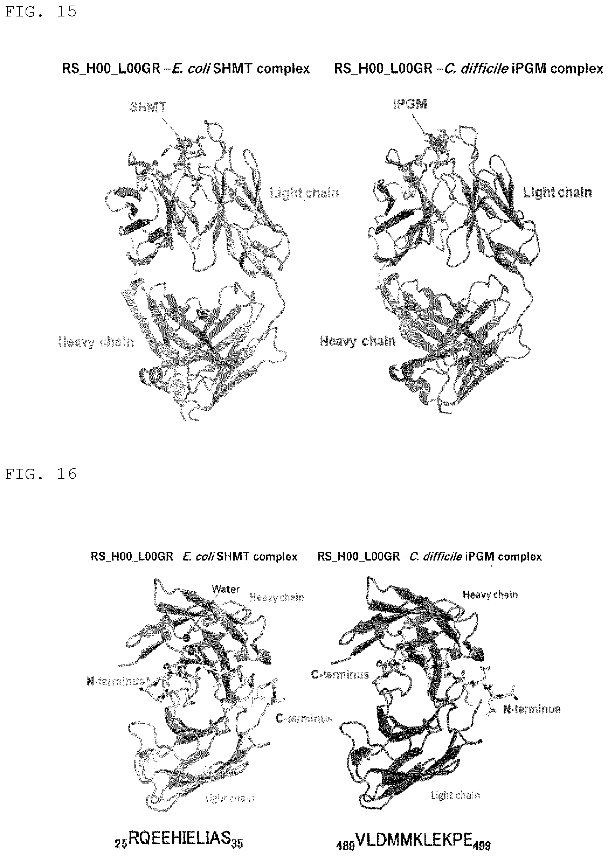

As a result of a series of experiments described above, the details of the interaction between the epitope peptide and the RS_H000_L000GR antibody were clarified (Figs. 15 and 16).

Specifically, epitope peptides of SHMT and iPGM bound in opposite directions to each other, and the amino acid sequence of the epitope showed the following similarity.

### E. coli SHMT(N→C): ROEEH-IELIAS-

### C. difficile iPGM(C→N): --EPKELKMMDLV

Based on these pieces of information, not only the type of the amino acid residue but also the effect of the amino acid side chain on the interaction can be determined. Based on these pieces of information obtained from the structural analysis, amino acid mutations that did not affect antigen binding were selected to prepare a series of RS mutant antibodies, and as described in detail below, it was revealed that all of them retained the same activity as that of the W27G2 antibody. Therefore, using the structural analysis result of the present disclosure, it has become possible to produce various RS mutant recombinant antibodies retaining the same activity as that of the W27G2 antibody.

### Example 5: Preparation of Genetically Modified Recombinant Antibody

For preparation of RS mutations, PCR was performed using an H-chain expression vector and an L-chain vector as templates and primers for mutagenesis containing base sequences corresponding to respective mutations, thereby amplifying the full length of the vector. Thereafter, the template vector DNA was treated with DpnI, and then the DNA was purified by isopropanol precipitation to transform into DH5α competent cells. The plasmid was extracted from the obtained clone, the clone into which the target mutation was introduced was selected, and the gene was introduced into ExpiCHO to obtain a mutant recombinant antibody.

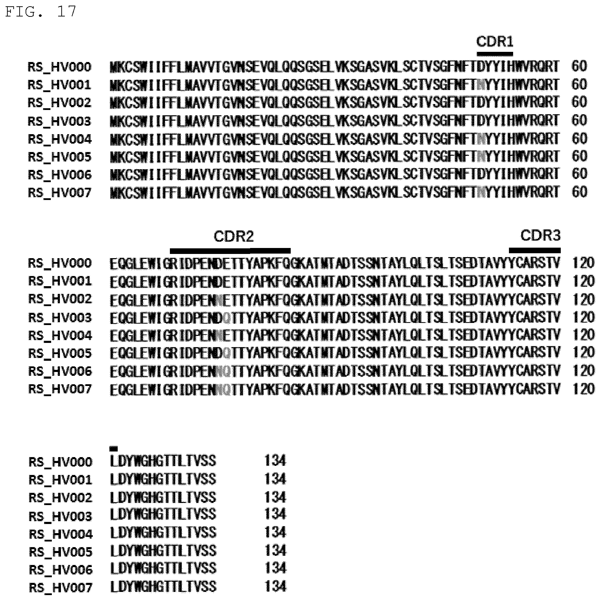

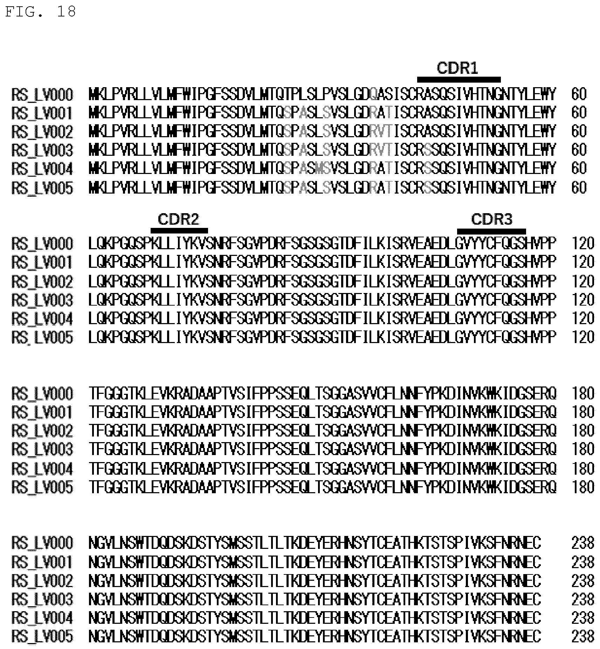

The amino acid sequences of the heavy chain variable region and the light chain variable region of a part of the produced mutant recombinant antibody are shown in Figs. 17 and 18.

By non-reduced SDS-PAGE and Coomassie blue stain of each RS mutation recombinant purified antibody, it was confirmed that dimers were mainly produced (Fig. 19).

### Example 6: Antigen Recognition Activity of Recombinant RS Modified Antibody

The binding force of the recombinant RS modified antibody to *E. coli* serine hydroxymethyltransferase (SHMT), which is an antigen molecule of the W27G2 antibody, was measured by ELISA.

### (Materials and Methods)

Wild-type SHMT and SHMT mutant of *E. coli* were overexpressed in *E. coli* as GST fusion proteins and purified. These bacteria were suspended in 0.05 M Na₂CO₃ buffer so as to be 2 µg/ml and immobilized to an ELISA plate, a dilution series of each variant purified antibody was prepared and added, and the binding force was detected by the same method as ELISA for measuring the binding force to the bacteria.

### (Results)

As a representative example, 6 types of mutant purified antibodies (RS_H00_L001, RS_H007_L001, RS_H007_L002, RS_H00_L005, RS_H007_L005, and RS_H007_L004) were tested, and it was confirmed that these antibodies bound to *E. coli* wild-type SHMT to almost the same extent as the hybridoma-derived W27G2 antibody (gel filtration purification), and did not bind to SHMT mutant (Fig. 20).

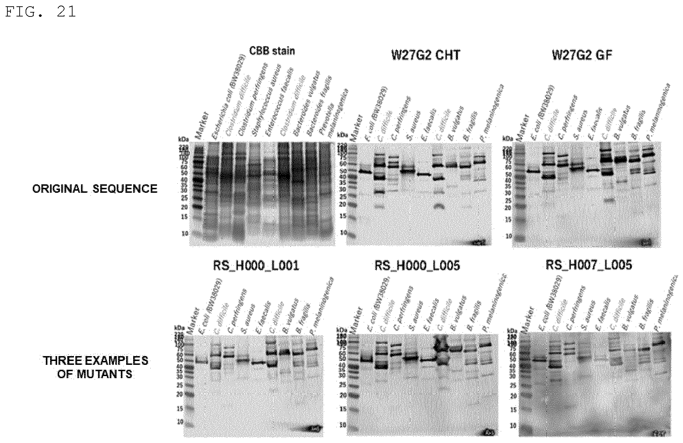

It was found that the W27G2 antibody was an antibody that recognizes different antigen molecules of a plurality of bacteria. Total proteins were extracted from the plurality of bacteria (including pathogenic bacteria) and proteins were transferred to nitrocellulose membranes after reduced SDS-PAGE, as shown by the Western analysis illustrated in Fig. 21. W27G2CHT (obtained by crudely purifying a W27G2 hybridoma culture supernatant with a hydroxyapatite column) and W27G2GF (obtained by further purifying W27G2CHT into a multimer fraction with a gel filtration column) were used as primary antibodies, and each of 3 types of recombinant purified antibodies (RS_H000_L001, RS_H000_L005, and RS_H007_L005) was reacted at a concentration of 2 ug/ml. Goat anti-mouse IgA (Southern Biotech) as a secondary antibody and IR800-conjugated anti-goat IgG (LI-COR) as a tertiary antibody were reacted, and a signal was detected with an Odyssey scanner. The gel after SDS-PAGE electrophoresis was stained using Coomassie brilliant blue (Nacalai) for the measurement of the protein amount of the sample loaded into each well. Since the hybridoma-derived W27G2 antibody and the recombinant purified antibody recognized antigen molecules of the same pattern, it was confirmed that there was no change in antigen recognition after introduction of mutation.

### Example 7: Confirmation of E. coli Growth Inhibitory Effect of Recombinant Gene-Modified Antibody

An *E. coli* BW38029 strain was subjected to stationary anaerobic culture at 37°C for 16 hours in LB medium. Bacteria were collected by centrifugation at 8,000 g for 5 minutes. After washing with sterile anaerobic LB, the viable cell count was measured with a flow cytometer.

Based on the above measurement results, bacteria were diluted with LB medium so as to be 300 cells/5 µl, 25 µl of each antibody or PBS was added, 30 µl of M9 minimum medium was further added, and after 20 hours, a bacterial count was measured by the above method using a flow cytometer. The final concentration of antibodies was 0.42 mg/ml.

### (Results)

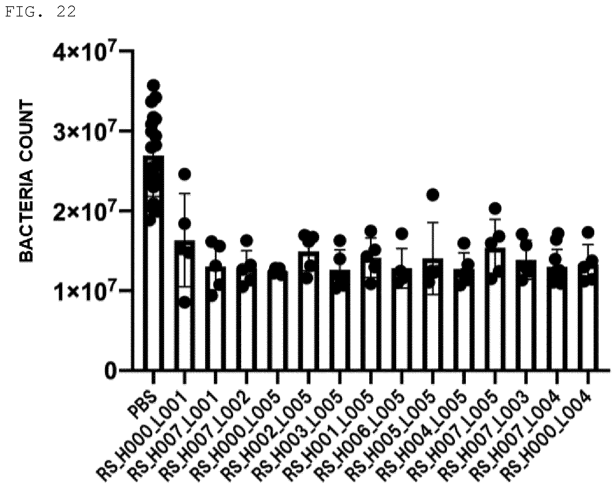

All of the various mutant recombinant antibodies inhibited the growth of *E. coli* as compared with the negative control (PBS added) sample (Fig. 22).

From all the experiments described above, it was revealed that the RS variant recombinant antibody prepared based on the three-dimensional structure analysis result retained the same properties as the original hybridoma W27G2 antibody. Therefore, it is understood that the RS variant recombinant antibody exhibits an effect of inhibiting liver inflammation similarly to the W27G2 antibody.

### Example 8: Screening of Antibodies Binding to Bacteria

### (8-1) Separation of Small Intestinal and Large Intestinal Mucous Lamina Propria Cells

The mice were euthanized and then subjected to laparotomy to remove the entire small intestine. After the connective tissue and Peyer's patches were removed from the removed small intestine, the small intestine was longitudinally incised, and contents of the small intestine were washed with PBS. Next, the washed small intestine was cut into a length of about 1 cm and added to a 100 ml beaker filled with 50 ml of PBS containing 1 mM EDTA. After shaking at 37°C for 20 minutes, small intestine fragments were collected in a strainer and PBS containing EDTA was discarded. The small intestine fragments were added to a 50 ml tube, 20 ml of PBS was added thereto, the mixture was vigorously shaken for 10 seconds, the small intestine fragments were collected again in a strainer, and the PBS was discarded. This operation was repeated twice, and only small intestine epithelial cells were removed from the small intestine tissue. Next, 50 ml of a digestive enzyme solution (adjusted with 100 ml RPMI 1640, 5 ml of FCS (final concentration 5%), 50 ul of 2-mercaptoethanol (final concentration 55 µM), 0.15 g of collagenase, and 1 ml of dispase (50 U/ml) (final concentration 0.5 U/ml)) warmed to 37°C was added to a 100 ml beaker, and the small intestine tissue was further finely cut and shaken at 37°C for 30 minutes. Thereafter, the 100 ml beaker was allowed to stand, the small intestine tissue was immersed, and then the supernatant was transferred to a new 50 ml tube. The digestion step using the digestive enzyme solution was repeated once for 20 minutes. The digestive fluid was centrifuged at 1,500 rpm and room temperature for 5 minutes, and the supernatant was discarded. Mucosal lamina propria cells obtained as precipitates were washed once with RPMI 1640 containing 2% FCS, suspended in 2 ml of RPMI 1640 containing 2% FCS, and then stored on ice. The same operation was performed on the supernatant subjected to the reaction in the second digestion step, the first cell suspension and the second cell suspension were combined, and then the combined suspension was passed through a filter to remove tissue pieces, thereby obtaining small intestine (large intestine) mucosa lamina propria cells.

### (8-2) Preparation and Cloning of Antibody-Producing Hybridomas

Hybridomas were prepared by fusing NS1 cells, which are mouse myeloma cells, with small intestine (large intestine) mucosal lamina propria cells or spleen cells. Cell fusion was performed according to ClonaCell-HY Hybridoma Cloning Kit (STEMCELL Technologies) according to the reagent and procedure of the kit. The resulting hybridomas were grown in a methylcellulose-containing medium according to the ClonaCell-HY Hybridoma Cloning Kit (STEMCELL Technologies), clones were picked up with the naked eye, and each clone was further grown in a 96-well plate. Thereafter, a frozen cell stock was prepared from the cloned hybridomas, and at the same time, a culture supernatant was obtained. For the antibody contained in the supernatant, the isotype of the antibody was confirmed by a normal sandwich ELISA method, and then the antibody titer of the antibody contained in the supernatant was measured and separated as each isotype antibody-producing hybridoma. For the antibodies used in the ELISA, 2 µg/ml of Anti-goat-mouse IgA (Southern Biotech), Anti-goat-mouse IgG (Southern Biotech), or Anti-goat-mouse IgM (Southern Biotech) was used for coating the plate, and 0.5 µg/ml of Alkaline Phosphatase (ALP)-conjugated anti-goat mouse IgA (Southern Biotech), Alkaline Phosphatase (ALP)-conjugated anti-goat mouse IgG (Southern Biotech), or Alkaline Phosphatase (ALP)-conjugated anti-goat mouse IgM (Southern Biotech) was used for detection. Mouse IgAκ (Immunology Consultants Laboratory), Purified mouse IgG1κ Isotype Control (BD Pharmingen), and PE-CF594 mouse IgMκ Isotype Control (BD Horizon) were used as control antibodies. OD₄₀₅ nm was measured using TriStar² LB942 (BERTHOLD TECHNOLOGIES). 500 or more hybridoma clones were obtained in total by the above operation.

### (8-3) Analysis of Binding Force of Hybridoma IgA Antibody to Each Enteric Bacterium

To screen for antibodies binding to each enteric bacterium, ELISA for each bacterium was performed. In particular, a screening method performed on *Clostridium difficile* (*C. difficile*) is described. *C. difficile* was subjected to anaerobic culture (Brain Heart Infusion media, 80% N₂, 10% H₂, 10% CO₂) at 37°C, and collected by centrifugation. Bacteria were suspended in a 0.05 M Na₂CO₃ buffer and coated onto an ELISA plate. After blocking with PBS containing 1% BSA, each antibody culture supernatant in a 96-well plate was added, and a reaction was allowed to proceed at room temperature for about 1 hour. Thereafter, the plate was washed with PBS added with 0.05% Tween 20, the secondary detection antibody (described above) corresponding to each antibody isotype was added and reacted, and then a color development reaction was performed using Alkali Phosphatase tablet (Sigma). For sufficient reaction, the ELISA plate after color development was reacted at 4 degrees overnight, and OD₄₀₅ nm was measured using TriStar² LB942 (BERTHOLD TECHNOLOGIES). A clone having OD₄₀₅ nm of 2.0 or more was defined as an antibody binding to C. *difficile.*

The selected clones were expanded and cultured (about 100 mL culture), and antibodies were purified from the culture solution using a Protein L column (Cytiva) in the case of IgM and IgA antibodies, and using a Protein A column (Cytiva) in the case of IgG antibodies. Elution of the antibodies was performed using a 10 mM citrate buffer (pH 2.5), the eluate was immediately neutralized with a 1 M citrate buffer (pH 9.0), and then, the neutralized eluate was concentrated with Amicon (100 kDa), dialyzed against a dialysis membrane (100 kDa pore size), and replaced with PBS. After dialysis, the antibody solution was recovered in a clean bench, and then the antibodies were sterilized using a syringe filter (0.22 um) and stored at 4°C. A concentration of the antibodies was measured by a sandwich ELISA method in the same manner as described above. RNA of the selected clone was extracted by ELISA, and the full length of the antibody gene was cloned and used to prepare a recombinant antibody.

### (8-4) Cloning of Full Length of Antibody Gene of Hybridoma-Derived Antibody

RNA was extracted from the cells of each clone using IsogenII (Nippon Gene Co., Ltd.). cDNA was synthesized using the extracted RNA as a template, and RT-PCR was performed using 7 types of primers (MH1-7) for a V_{H} region of the antibody and Cα, C?, or Cγ region-specific primers. For the light chain, PCR was performed with a Vκ primer and a Cκ primer. The base sequences of the primers are shown in Table 1. The amplified V_{H} region PCR product or Vκ region PCR product was directly sequenced, and the variable region gene sequence of each clone was obtained. This was searched with Ig blast, further upstream sequences (including signal sequences) of specific V_{H} or Vκ genes were obtained, PCR primers were prepared based on the most upstream sequences, and PCR was performed together with the reverse primer at the 3' end of each C region secretory sequence, thereby obtaining full-length gene base sequences of an H chain and an L chain. Based on this, pcDNA 3.1 (+) vector (Invitrogen) was cloned into the H chain, L chain, and J chain (search a base sequence from database and obtain full-length gene sequences by PCR using cDNA of hybridoma).

**Table 1. Base sequence of primer for amplification of antibody gene variable region of hybridoma-derived antibody**

| Primer name | Base sequence (5'-3') |
|---|---|
| MH1 | SARGTNMAGCTGSAGTC |
| MH2 | SARGTNMAGCTGSAGSAGTCWGG |
| MH3 | CAGGTTACTCTGAAAGWTSTG |
| MH4 | GAGGTCCARCTGCAACARTC |
| MH5 | CAGGTCCAACTVCAGCARCC |
| MH6 | GAGGTGAASSTGGTGGAATC |
| MH7 | GATGTGAACTTGGAAGTGTC |
| IgAR | GATGGTGGGATTTCTCGCAGAC |
| degVκ | GGCTGCAGSTTCAGTGGCAGTGGRTCWGGRAC |
| CκR | AACGTGAGGGTGCTGCTCATG |

In the base sequences in the table, S represents G or C, R represents A or G, N represents A, C, G, or T, W represents A or T, and V represents G, C, or A.

The above expression vectors were transfected into Expi CHOS cells (Thermo Fischer Scientific) according to the kit protocol so that the amount ratio was H:L:J = 2:2:1. On days 10 to 12 after transfection (Thermo Fischer Scientific, ExpiCHO Expression System), the culture supernatant was recovered, and the antibody was purified and concentrated by a Protein L column in the same manner as described above, replaced with PBS by dialysis, and sterilized with a filter.

Recombinant multimeric IgA antibodies SNK0001AR and SNK0002AR were obtained by preparing an expression vector in which a gene sequence of an antibody gene variable region derived from SNK0001M and SNK0002M hybridomas obtained by the screening and an IgA antibody constant region gene sequence were bonded, further constructing a J-chain expression vector, and transfecting CHO cells with 3 types of expression vectors (H chain, L chain, and J chain). In addition, SNK0003A was obtained as an IgA antibody derived from an IgA-producing hybridoma.

### Example 9: Effect of Antibody Obtained in Screening on Inhibition of Liver Fibrosis

The inhibitory effect of the antibody obtained in Example 8 on liver inflammation is confirmed using the same BDL test system as in Example 1. It can be confirmed from the same experiment that the monoclonal antibody obtained in Example 8 can also be used for inhibition of liver inflammation, inhibition of liver fibrosis, and prevention or treatment of various impairments or diseases associated with liver inflammation.

### Industrial Applicability

The present disclosure provides a pharmaceutical composition, a food composition, or a feed composition containing a monoclonal antibody that binds to enteric bacteria, and can be used to inhibit liver inflammation, inhibit liver fibrosis, and prevent or treat various impairments or diseases associated with liver inflammation. The present disclosure can be further used as a food composition or feed for improving liver function and preventing deterioration of liver function.

## Claims

1. A pharmaceutical composition, a food composition, or a feed composition comprising a monoclonal antibody that binds to enteric bacteria or an antigen-binding fragment thereof,
wherein the pharmaceutical composition, the food composition, or the feed composition is used for preventing or treating an impairment or disease attributed to liver inflammation.

2. The composition according to claim 1, wherein the monoclonal antibody that binds to enteric bacteria or the antigen-binding fragment thereof is a monoclonal antibody that binds to a *Clostridium difficile* bacterial body or an antigen-binding fragment thereof.

3. The composition according to claim 1, wherein the monoclonal antibody that binds to enteric bacteria or the antigen-binding fragment thereof is one or more antibodies or antigen-binding fragments thereof, the antibodies being selected from the group consisting of:
a) an antibody including:
a heavy chain variable region containing an amino acid sequence of a heavy chain CDR1, an amino acid sequence of a heavy chain CDR2, and an amino acid sequence of a heavy chain CDR3, the heavy chain variable region containing an amino acid sequence set forth in SEQ ID NO: 7; and
a light chain variable region containing an amino acid sequence of a light chain CDR1, an amino acid sequence of a light chain CDR2, and an amino acid sequence of a light chain CDR3, the light chain variable region containing an amino acid sequence set forth in SEQ ID NO: 8;
b) an antibody including:
a heavy chain variable region containing an amino acid sequence of a heavy chain CDR1, an amino acid sequence of a heavy chain CDR2, and an amino acid sequence of a heavy chain CDR3, the heavy chain variable region containing an amino acid sequence set forth in SEQ ID NO: 17; and
a light chain variable region containing an amino acid sequence of a light chain CDR1, an amino acid sequence of a light chain CDR2, and an amino acid sequence of a light chain CDR3, the light chain variable region containing an amino acid sequence set forth in SEQ ID NO: 18;
c) an antibody including:
a heavy chain variable region containing an amino acid sequence of a heavy chain CDR1, an amino acid sequence of a heavy chain CDR2, and an amino acid sequence of a heavy chain CDR3, the heavy chain variable region containing an amino acid sequence set forth in SEQ ID NO: 27; and
a light chain variable region containing an amino acid sequence of a light chain CDR1, an amino acid sequence of a light chain CDR2, and an amino acid sequence of a light chain CDR3, the light chain variable region containing an amino acid sequence set forth in SEQ ID NO: 28;
d) an antibody including:
a heavy chain variable region containing an amino acid sequence of a heavy chain CDR1, an amino acid sequence of a heavy chain CDR2, and an amino acid sequence of a heavy chain CDR3, the heavy chain variable region containing an amino acid sequence set forth in SEQ ID NO: 37; and
a light chain variable region containing an amino acid sequence of a light chain CDR1, an amino acid sequence of a light chain CDR2, and an amino acid sequence of a light chain CDR3, the light chain variable region containing an amino acid sequence set forth in SEQ ID NO: 38; and
e) an antibody containing,
with respect to a reference antibody including a heavy chain variable region containing an amino acid sequence set forth in SEQ ID NO: 37 and a light chain variable region containing an amino acid sequence set forth in SEQ ID NO: 38,
at least one amino acid mutation in at least one region selected from heavy chains CDR1 to 3, light chains CDR1 to 3, and a light chain FR1, and
binding to the amino acid sequence RQEEHIELIAS in *E*. *coli* SHMT protein and the amino acid sequence VLDMMKLEKPE in C. *difficile* iPGM protein.

4. The composition according to claim 1, wherein the monoclonal antibody that binds to enteric bacteria is one or more antibodies or antigen-binding fragments thereof, the antibodies being selected from the group consisting of:
a) an antibody including:
a heavy chain variable region containing:
a heavy chain CDR1 containing an amino acid sequence set forth in SEQ ID NO: 1;
a heavy chain CDR2 containing an amino acid sequence set forth in SEQ ID NO: 2; and
a heavy chain CDR3 containing an amino acid sequence set forth in SEQ ID NO: 3; and
a light chain variable region containing:
a light chain CDR1 containing an amino acid sequence set forth in SEQ ID NO: 4;
a light chain CDR2 containing an amino acid sequence set forth in SEQ ID NO: 5; and
a light chain CDR3 containing an amino acid sequence set forth in SEQ ID NO: 6;
b) an antibody including:
a heavy chain variable region containing:
a heavy chain CDR1 containing an amino acid sequence set forth in SEQ ID NO: 11;
a heavy chain CDR2 containing an amino acid sequence set forth in SEQ ID NO: 12; and
a heavy chain CDR3 containing an amino acid sequence set forth in SEQ ID NO: 13; and
a light chain variable region containing:
a light chain CDR1 containing an amino acid sequence set forth in SEQ ID NO: 14;
a light chain CDR2 containing an amino acid sequence set forth in SEQ ID NO: 15; and
a light chain CDR3 containing an amino acid sequence set forth in SEQ ID NO: 16;
c) an antibody including:
a heavy chain variable region containing:
a heavy chain CDR1 containing an amino acid sequence set forth in SEQ ID NO: 21;
a heavy chain CDR2 containing an amino acid sequence set forth in SEQ ID NO: 22; and
a heavy chain CDR3 containing an amino acid sequence set forth in SEQ ID NO: 23; and
a light chain variable region containing:
a light chain CDR1 containing an amino acid sequence set forth in SEQ ID NO: 24;
a light chain CDR2 containing an amino acid sequence set forth in SEQ ID NO: 25; and
a light chain CDR3 containing an amino acid sequence set forth in SEQ ID NO: 26;
d) an antibody including:
a heavy chain variable region containing:
a heavy chain CDR1 containing an amino acid sequence set forth in SEQ ID NO: 31;
a heavy chain CDR2 containing an amino acid sequence set forth in SEQ ID NO: 32; and
a heavy chain CDR3 containing an amino acid sequence set forth in SEQ ID NO: 33; and
a light chain variable region containing:
a light chain CDR1 containing an amino acid sequence set forth in SEQ ID NO: 34;
a light chain CDR2 containing an amino acid sequence set forth in SEQ ID NO: 35; and
a light chain CDR3 containing an amino acid sequence set forth in SEQ ID NO: 36; and
e) an antibody containing,
with respect to a reference antibody including a heavy chain variable region containing an amino acid sequence set forth in SEQ ID NO: 37 and a light chain variable region containing an amino acid sequence set forth in SEQ ID NO: 38,
at least one amino acid mutation in at least one region selected from heavy chains CDR1 to 3, light chains CDR1 to 3, and a light chain FR1, and
binding to the amino acid sequence RQEEHIELIAS in *E*. *coli* SHMT protein and the amino acid sequence VLDMMKLEKPE in C. *difficile* iPGM protein.

5. The composition according to claim 1, wherein the monoclonal antibody that binds to enteric bacteria is an antibody including:
a heavy chain variable region containing:
a heavy chain CDR1 containing an amino acid sequence of X₁YYIH;
a heavy chain CDR2 containing an amino acid sequence of RIDPENX₂X₃TTYAPKFQ; and
a heavy chain CDR3 containing an amino acid sequence of YCARSTVL; and
a light chain variable region containing:
a light chain CDR1 containing an amino acid sequence of RX₄SQSIVHTNG;
a light chain CDR2 containing an amino acid sequence of KLLIYKV; and
a light chain CDR3 containing an amino acid sequence of GVYYFQGS,
in which a light chain FR1 contains an amino acid sequence of TPLSLPVSLGDQA or an amino acid sequence of SPASX₅SVSLGDRX₆,
X₁, X₂, and X₃ are each independently a neutral polar amino acid or an acidic polar amino acid,
X₄ is a non-polar amino acid or a neutral polar amino acid, and
X₅ and X₆ are each independently a non-polar amino acid.

6. The composition according to any one of claims 1 to 5, wherein the monoclonal antibody that binds to enteric bacteria is an IgA antibody in a multimeric form or a monomeric form, an IgM antibody in a multimeric form or a monomeric form, an IgG antibody in a monomeric form or a combination thereof.

7. The composition according to any one of claims 1 to 6, wherein the disease or impairment attributed to liver inflammation is chronic hepatitis, liver fibrosis, liver cirrhosis, liver inflammation following liver transplantation, autoimmune hepatitis, or intrahepatic lithiasis.

8. A method for preventing or treating an impairment or disease attributed to liver inflammation, the method comprising administering a monoclonal antibody that binds to enteric bacteria to a subject in need of prevention or treatment of an impairment or disease attributed to liver inflammation.
